# EUROPÄISCHE PATENTANMELDUNG

(11) **EP 0 548 793 A2**
(43) Veröffentlichungstag der Anmeldung: **30.06.1993**
(21) Anmeldenummer: 92121458.1
(22) Anmeldetag: 17.12.1992
(51) Int. Cl.: C07J 9/00, A61K 31/575, C07J 41/00, C07J 31/00, C07J 51/00, C07J 43/00

(54) **Ethylenisch ungesättigte Gallensäurederivate, Verfahren zu ihrer Herstellung und Vorstufen**

(30) Priorität: 20.12.1991 DE 4142323; 01.09.1992 DE 4229033
(71) Anmelder: HOECHST AKTIENGESELLSCHAFT, D-65926 Frankfurt am Main (DE)
(72) Erfinder: Enhsen, Alfons, Dr., W-6087 Büttelborn (DE); Glombik, Heiner, Dr., W-6238 Hofheim am Taunus (DE); Müllner, Stefan, Dr., W-6203 Hochheim (DE); Wess, Günther, Dr., W-6455 Erlensee (DE)

(57) **Zusammenfassung**

Es werden ethylenisch ungesättigte Gallensäurederivate der Formel I

G - X - A I

worin G, X und A die angegebenen Bedeutungen haben, beschrieben. Sie eignen sich zur Herstellung von polymeren Gallensäurederivaten. Es werden ferner Gallensäurederivate der Formel IVa

G - X' IVa

worin G und X' die angegebenen Bedeutungen haben, beschrieben. Sie sind wertvolle Synthesebausteine für die Herstellung von Pharmaka.

## Beschreibung

Die Erfindung betrifft ethylenisch ungesättigte Gallensäurederivate. Sie eignen sich zur Synthese von Gallensäurepolymeren. Die Erfindung betrifft ferner Vorstufen, die als Bausteine zur Synthese von Gallensäurederivaten verwendet werden können.

Gallensäuren haben wichtige physiologische Funktionen bei der Fettverdauung, z. B. als Cofaktoren der pankreatischen Lipasen und als natürliche Detergentien zur Solubilisierung von Fetten und fettlöslichen Vitaminen. Als Endprodukte des Cholesterinstoffwechsels werden sie in der Leber synthetisiert, in der Gallenblase gespeichert und aus dieser durch Kontraktion in den Dünndarm abgegeben, wo sie ihre physiologische Wirkung entfalten. Der größte Teil der sezernierten Gallensäuren wird über den enterohepatischen Kreislauf wieder zurückgewonnen.

Nicht resorbierbare, unlösliche, basische und vernetzte Polymere werden seit geraumer Zeit zur Bindung von Gallensäuren verwendet und aufgrund dieser Eigenschatten therapeutisch genutzt.

Die erfindungsgemäßen Gallensäurederivate besitzen eine hohe Affinität zu den spezifischen physiologischen Gallensäuretransportsystemen in Mensch und Tier. Diese Verbindungen sind somit in der Lage, den spezifischen Gallensäuretransport konzentrationsabhängig zu hemmen, wobei sie selbst schlecht bis gar nicht transportiert werden. Überraschenderweise wurde nun gefunden, daß aus den erfindungsgemäßen Verbindungen hergestellte polymere Gallensäurederivate unter Erhalt bzw. sogar unter Steigerung der Affinität mit den Transportsystemen wechselwirken, selbst aber nicht resorbiert werden. Dies macht sie für den Einsatz als nicht systemisch wirkende Arzneimittel tauglich.

Die Erfindung betrifft ethylenisch ungesättigte Gallensäurederivate der Formel I

G-X-A (I)

in der
- G: ein Gallensäurederivat,
- X: eine Brückengruppe und
- A: eine polymerisierbare, ethylenisch ungesättigte Gruppe
bedeutet.

Die Verbindungen der Formel I dienen als Ausgangsverbindungen zur Herstellung von polymeren Gallensäurederivaten.

Bevorzugt wird unter den einzelnen Gruppen verstanden:
- G:: eine freie Gallensäure bzw. ihr Alkali- oder Eralkalisalz oder eine am Ring D veresterte Gallensäure, die über ihren Ring A oder B, vorzugsweise über Ring A, verbunden ist mit der Gruppe
- X,: für die die Formel II gilt

(Y)ₒ - (Z)ₚ, (II)

worin
- Y: benachbart zu G steht und -O-,-NR'-, 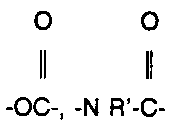 bedeutet,
- Z: (C₁-C₁₂)-Alkylen oder (C₇-C₁₃)-Aralkylen ist, wobei einzelne, bevorzugt 1 bis 4, Methylengruppen in der Alkylenkette des Alkylen- oder
Aralkylenrestes durch Gruppen wie -O-,-NR'-, 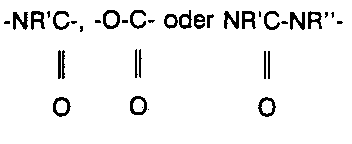 bevorzugt durch Gruppen eines Typs, ersetzt sein können, und
- o und p: unabhängig voneinander null oder 1, wobei o und p nicht gleichzeitig null bedeuten, sind,
- A:: 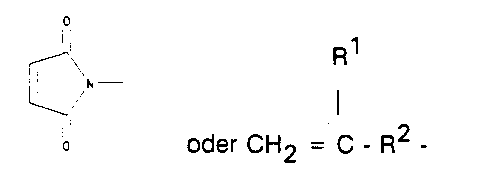 wobei
- R¹: Wasserstoff oder CH₃ und
- R²: 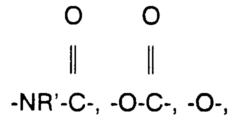 -NR'- oder eine Einfachbindung bedeuten, wobei die Carbonylgruppen benachbart zur C-C-Doppelbindung stehen, und
- R' und R'': unabhängig voneinander Wasserstoff oder (C₁-C₆)-Alkyl, bevorzugt (C₁-C₃)-Alkyl, bedeuten.

Bevorzugt sind Verbindungen der Formel I, in denen
G der Formel III 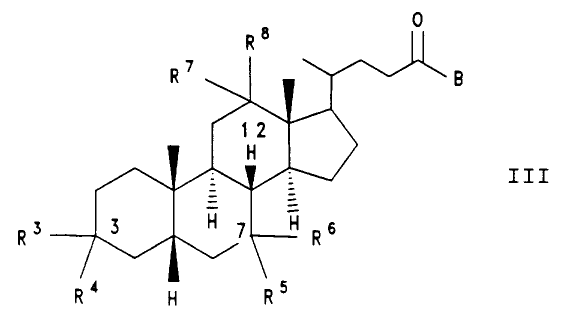
entspricht, worin
- R³ bis R⁸: unabhängig voneinander Wasserstoff, OH, NH₂ oder eine mit einer OH- Schutzgruppe geschützte OH Gruppe und einer der Reste R³, R⁴, R⁵, R⁶ eine Bindung zur Gruppe X bedeuten, wobei diese Bindung von den Positionen 3 (R³ oder R⁴), bevorzugt 3β, oder 7 (R⁵ oder R⁶) ausgeht und die jeweils andere Position 7 oder 3 eine OH Gruppe oder eine geschützte OH Gruppe trägt,
- B: -OH, -O-Alkali, -O-Erdalkali, -O-(C₁-C₁₂)-Alkyl, -O-Allyl oder -O-Benzyl bedeutet, bevorzugt -OH, -O-Alkali oder -O(C₁-C₆-Alkyl, -O-Allyl oder -O-Benzyl, bedeutet,
wobei eine gebildete Estergruppe 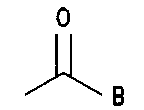 sowohl sauer wie auch basisch verseifbare Ester darstellt,
- Y: -O-,-NR'-, 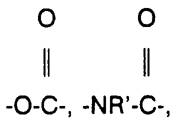
- Z: (C₁-C₁₂)-Alkylen, (C₇-C₁₃)-Aralkylen, wobei 1 bis 3 Methylengruppen in der Alkylenkette durch die Gruppen -O-,-NR'-, 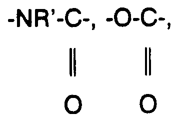 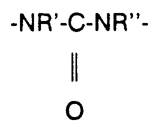 ersetzt sind und
- o und p: unabhängig voneinander null oder 1 bedeuten, aber nicht gleichzeitig null sind,
- A: 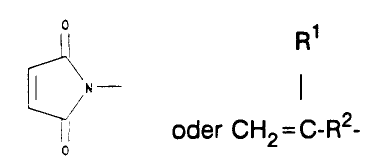 bedeuten, wobei
- R¹: Wasserstoff oder CH₃ und
- R²: 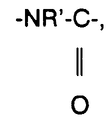 -NR'- oder eine Einfachbindung bedeutet, worin
- R' und R'': unabhängig voneinander Wasserstoff oder (C₁-C₆)-Alkyl bedeuten.

Sofern p = null und o = 1 gilt, ist Y bevorzugt 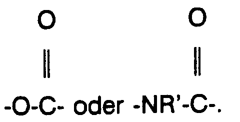
Sofern p = 1 und o = null gilt, ist Z bevorzugt (C₁-C₁₂)-Alkylen, wobei 1 - 3 Methylengruppen durch 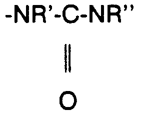
ersetzt sind.

Sofern p = 1 und o = 1 gilt, ist Y bevorzugt -O-. Hierunter ist bevorzugt, daß Z (C₁-C₁₂)-Alkylen oder (C₇-C₁₃)-Aralkylen bedeutet, wobei 1 oder 2 Methylengruppen,
bevorzugt eine Methylengruppe, durch 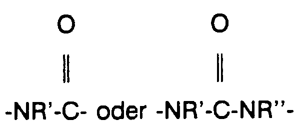
ersetzt sind.

Weiterhin ist hierunter bevorzugt, daß eine Methylengruppe von Z dann 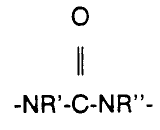
bedeutet, wenn Z selbst ein Aralkylenrest bedeutet, worin der Arylrest meta-verknüpft ist, Z einerseits als Rest A eine Gruppe 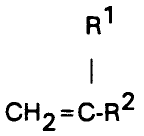
trägt, worin R² eine Einfachbindung bedeutet und andererseits eine 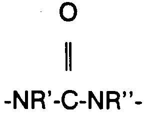
Gruppe trägt, die über eine Methylengruppe mit dem Aralkylenrest meta-verknüpft ist.

Ebenso ist hierunter bevorzugt, daß, sofern Z eine (C₁-C₁₂)-Alkylengruppe bedeutet,
maximal eine Methylengruppe durch 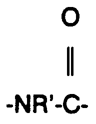
ersetzt ist und als Rest A 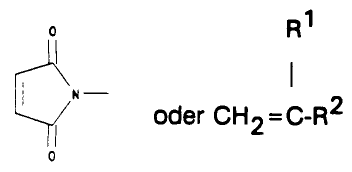
gilt, wobei R² 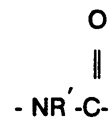
bedeutet.

Sofern R² eine Einfachbindung bedeutet und o = 0 und p = 1 ist, gilt für Z bevorzugt (C₁-C₃)-Alkylen.

Besonders bevorzugt ist weiterhin, daß Y nicht direkt mit der eine Methylengruppe von Z ersetzenden Gruppe direkt benachbart ist, und auch nicht mit 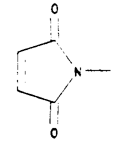
oder 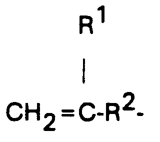
benachbart ist, sofern R² eine Einfachbindung bedeutet.

In den vorstehenden und folgenden Ausführungen wird unter "Alkyl" ein geradkettiger oder verzweigter Alkylrest verstanden.

Unter den OH-Schutzgruppen werden verstanden:
Ein Alkylrest mit 1 - 10 C-Atomen oder Alkylenrest mit 2 bis 10 C-Atomen, der verzweigt oder unverzweigt ist,
ein Cycloalkylrest mit 3 - 8 C-Atomen,
ein Phenylrest, der unsubstituiert oder 1 - 3fach substituiert ist mit F, Cl, Br, (C₁-C₄)-Alkyl oder (C₁-C₄)-Alkoxy,
ein Benzylrest, der unsubstituiert oder 1 - 3fach substituiert ist mit F, Cl, Br, (C₁-C₄)-Alkoxy oder
ein 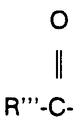
Rest, wobei R''' Wasserstoff oder (C₁-C₄)-Alkyl bedeutet.

Die genannten erfindungsgemäßen Gallensäurederivate werden zur Herstellung von polymeren Gallensäurederivaten verwendet. Polymere Gallensäurederivate, deren Herstellung und Verwendung werden in der gleichzeitig eingereichten Patentanmeldung P 4142379.8 beschrieben. Die polymeren Gallensäurederivate besitzen eine hohe Affinität zum Gallensäuretransportsystem verschiedener Organe, werden aber aufgrund ihrer Größe und ihres Molekulargewichts selbst nicht transportiert. Daher kann mit Hilfe der erfindungsgemäßen Gallensäurederivate bzw. polymeren Gallensäurederivate der physiologische Gallensäuretransport spezifisch gehemmt werden. Dies ist insbesondere von Bedeutung für die Inhibition der Gallensäurerückresorption im Dünndarm, da diese in Mensch und Tier erwiesener Maßen zu einer Senkung des Serumcholesterinspiegels führt. Die aus den erfindungsgemäßen Verbindungen der Formel I herstellbaren polymeren Gallensäurederivate stellen daher wertvolle Arzneimittel, insbesondere Hypolipidämika, dar.

Die erfindungsgemäßen Verbindungen werden in Abhängigkeit von den jeweiligen funktionellen Gruppen, vorzugsweise gemäß den Syntheseprinzipien 1) bis 9) hergestellt. Die letzte Stufe beinhaltet dabei meist die Einfügung der Gruppe A in Verbindungen der Formel IV

G - X' (IV)

worin G die zu Formel I angegebene Bedeutung hat und X' eine wie zu Formel I angegebene Brückengruppe darstellt, die zusätzlich einen reaktiven Rest aufweist, der die Einführung von A erlaubt.

Verbindungen der Formel IVa

G - X' (IVa)

in der
- G: einen Gallensäurerest der Formel V 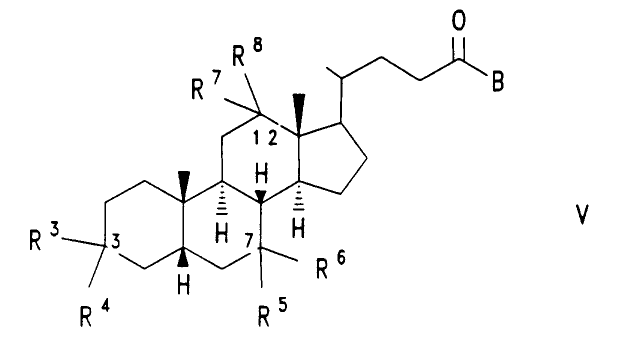
bedeutet, worin
einer der Reste R³ bis R⁶ die Bedeutung von X' hat und die übrigen Reste R³ bis R⁸ unabhängig voneinander Wasserstoff, OH oder eine geschützte OH-Gruppe darstellen
- B: -OH, -O-Alkali, O-Erdalkali, -O-(C₁-C₁₂)-Alkyl, -O-Allyl oder O-Benzyl bedeutet, bevorzugt -OH, -O-Alkali oder -O-(C₁-C₆)-Alkyl, -O-Allyl oder -O-Benzyl,
und
- X': einen Rest der Formel VI

Y' - Z' (VI)

bedeutet, worin
- Y': benachbart zu G steht und eine einfache Bindung,
-O-(CH₂)₂₋₁₂-,
-(CH₂)₁₋₆- oder 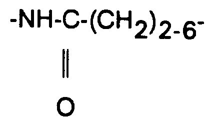 bedeutet
- Z': -OH, -OSO₂-CH₃, 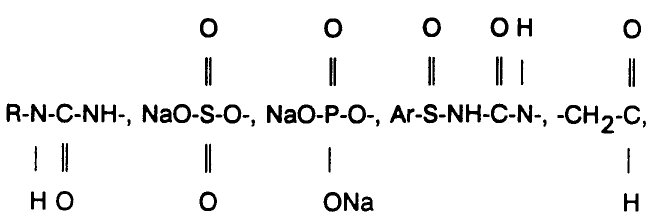 -O-Benzyl, -O-Allyl, -O-t-Butyldimethylsilyl, 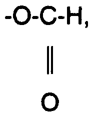 -N₃, -NH₂, -CN oder Brom darstellt
sind neu. Die Erfindung betrifft daher auch die Verbindungen der Formel IVa.

Ar steht für einen Phenylrest, der unsubstituiert oder 1 bis 2-fach substituiert ist mit F, Cl, Br, (C₁-C₄)-Alkyl oder (C₁-C₄)-Alkoxy,
R steht für (C₁-C₄)-Alkyl oder Ar.

Die erfindungsgemäßen Verbindungen IVa sind wertvolle Synthesebausteine zur Herstellung von Pharmaka (vergl. z. B. EP-A-0 489 423) und der Verbindungen der Formel I. Dabei können sie als solche oder nach Überführung von Z in eine andere reaktive Gruppe wie z. B. von N₃ nach NH₂ bei der Synthese von Pharmaka eingesetzt werden.

### Syntheseprinzipien (erläutert an Beispielen, in denen G einen Rest der Formel III bedeutet) :

1) Verbindungen der Formel I, in denen
   Y = -O-, Z = Alkylen oder Aralkylen A = 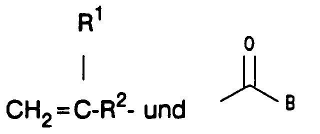 eine freie Säure, ihr Salz oder einen basisch verseifbaren Ester bedeuten, erhält man z. B. durch folgende Reaktionsfolge aus Verbindungen (1), die z. T. in EP-A-0 489 423 beschrieben sind. Dabei werden Verbindungen (1) durch Reaktionen mit *α*,ω-Alkandiolen (Alkan = 2 - 12 C-Atome) unter Basenzusatz (z. B. Pyridin, Triethylamin oder KOH) bei Temperaturen von 60 bis 140 °C zu kettenverlängerten Gallensäurederivaten der Formel (2) umgesetzt, z. B. arbeitet man bei Umsetzung mit 1,6-Hexandiol (6 C-Atome) bei einem Überschuß von Pyridin ohne weitere Lösungsmittel und bei Temperaturen von
   100°C. Schutzgruppen für die übrigen OH-Gruppen im Gallensäuremolekül sind dabei nicht erforderlich.
   Verbindungen der Formel (3) erhält man durch säurekatalysierte Veresterung der -COOH-Funktion aus Verbindung (2) unter Verwendung eines Überschusses an Methanol unter Wasserausschluß. Basisch verseifbare Ester höherer Alkohole sind ebenso darstellbar. Insbesondere werden folgende Alkohole genannt: Ethanol, i-Propanol, n-Propanol, n-Butanol. Zahlreiche Säuren können dabei als Katalysatoren eingesetzt werden, z. B. BF₃, Molekularsieb zum Wasserentzug und Halogenwasserstoffsäuren. Bevorzugt verwendet man HCl, die auch in situ aus organischen Säurechloriden, R^{.}COCl (R^{.} = CH₃, C₂H₅, C₃H₇), erzeugt werden kann. Die Reaktionstemperatur liegt hierbei zwischen 0 und 30 °C.
   Verbindungen der Formel (4) erhält man durch Umsetzung von Verbindung (3) mit Methansulfonsäurechlorid unter einem Überschuß einer organischen Base, bevorzugt Pyridin, bei Temperaturen von -10 ° bis 0 °C (unter Kühlung). Als Lösungsmittel können hierbei inerte Lösungsmittel, insbesondere Dichlormethan, verwendet werden.
   Durch Umsetzung von Verbindung (4) mit Alkalimetallaziden, vorzugsweise Na-Azid in einem aprotischen Lösemittel, z. B. DMF, bei Temperaturen von 20 ° bis 100 °C, werden die Azide (5) hergestellt. Diese können mit Wasserstoff über einen Katalysator, vorzugsweise bei Raumtemperatur, zu den Aminen (6) reduziert werden. Als Katalysatoren können z. B. verwendet werden: Pd auf Kohle, Rh auf Aluminiumoxid oder Raney-Nickel. Als Lösemittel werden Essigester, Methanol oder THF bevorzugt. Wählt man einen mäßig aktiven Katalysator (z. B. Pd/C mit Wasser) und einen geringen Wasserstoffdruck (z. B. 1 - 5 bar), so kann zunächst das Nitril IVa (6a) isoliert werden, das seinerseits unter drastischeren Bedingungen (bevorzugt Rh auf Al₂O₃; Druck (H₂) = 30 - 100 bar) weiter zu Verbindung (6) reduziert werden kann.
   Amine der Formel (6) können zu polymerisierbaren Acrylaten der Formel (I) (7) umgesetzt werden, in dem man sie mit aktivierten Derivaten der Acrylsäure kuppelt. Beispielsweise kann das ungesättigte Säurechlorid zwischen 0 ° und -20 °C in inerten Lösemitteln, wie Dichlormethan, zur Reaktion gebracht werden, bevorzugt unter Zusatz von Basen wie Pyridin, Triethylamin, Lutidin etc. Auch andere Amidbildungsmethoden der Peptidchemie sind anwendbar, z. B. die Säureaktivierung mit 1,2-Dihydro-2-ethoxychinolin-1-carbonsäureethylester (EEDQ) in Essigester, THF o. ä., bei Temperaturen zwischen Raumtemperatur und Rückflußtemperatur. Wird längere Zeit erwärmt, so wirkt ein Zusatz von Hydrochinon ausbeutesteigernd.
   Die vorstehend beschriebenen Reaktionen sind im Formelschema 1 zusammengefaßt. 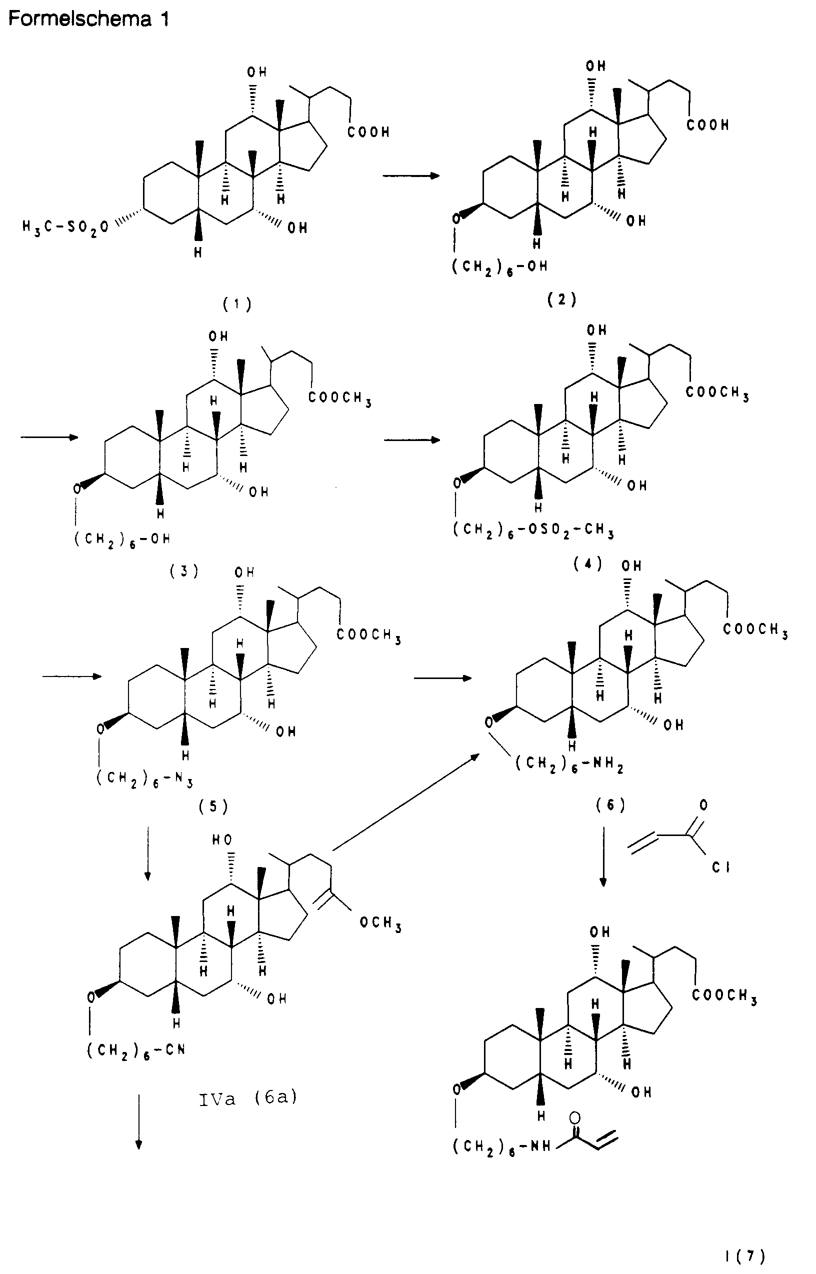
2) Verbindungen der Formel I, in denen Y = -O- und Z = Alkylen oder Aralkylen ist, wobei 1-3 Methylengruppen ersetzt sind durch Gruppen wie
   -O-,-NR'-, 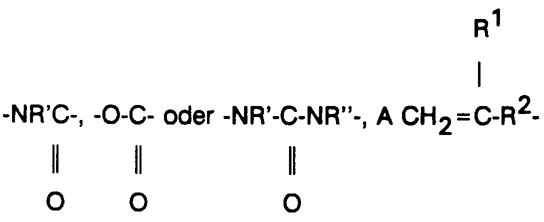 und
   eine freie Säure, ihr Salz, oder einen basisch verseifbaren Ester bedeutet, können wie folgt hergestellt werden:
   Polymerisierbare Gallensäurederivate der Formel I (10) erhält man durch Verknüpfung der Verbindung (9), die literaturbekannt ist, z. B. mit den Aminen (6) oder (8), wobei Verbindung (8) aus EP-A-0 489 423 bekannt ist, nach Aktivierung der Säurefunktion von (9), bevorzugt mit EEDQ oder mit Dicyclohexylcarbodiimid (DCC)/Hydroxybenzotriazol (HOBT), z. B. in THF bei 0 ° bis 10 °C. Die beschriebenen Reaktionen können sowohl mit den bevorzugten 3β-Gallensäurederivaten als auch mit den entsprechenden 3*α*-Analogen durchgeführt werden.
   Die beschriebene Reaktion ist in Formelschema 2 zusammengefaßt. 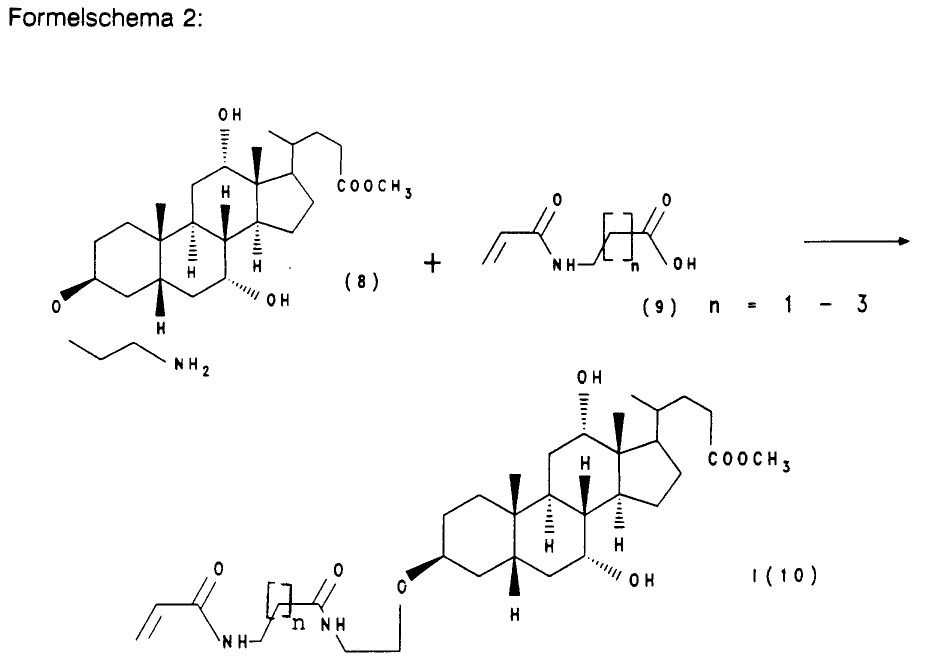
3) Verbindungen der Formel I, in denen o = null und Z = Alkylen oder Aralkylen ist, wobei 1 - 3 Methylengruppen ersetzt sind durch 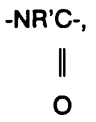 A 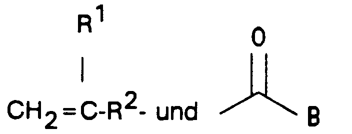 ein freie Säure, ihr Salz, oder einen basisch verseifbaren Ester, können wie folgt dargestellt werden:
   Amine der Formel (15), die als Ausgangsverbindungen für polymerisierbare Monomere I (16) dienen, erhält man aus den Ketonen (11), die literaturbekannt sind, durch eine Wittig-Horner-Reaktion mit Cyanomethyl-phosphonaten der Formel (12) unter Zusatz starker Basen. Als Lösemittel können aprotische oder protische, nicht wäßrige Lösemittel verwendet werden. Die Reaktionstemperatur liegt zwischen Raumtemperatur und 70 °C, vorzugsweise zwischen 30 und 40 °C. Als Basen können Alkalihydroxide, bevorzugt Alkalihydride oder Alkalialkoholate verwendet werden. Durch eine Verwendung von NaOCH₃ in Methanol wird eine als Nebenreaktion auftretende Umesterung der Gallensäuremethylester (11) zurückgedrängt.
   Die so gewonnenen Nitrile IVa (13) können ein- oder zweistufig (über IVa (14)) zu den Aminen IVa (15) reduziert werden. Je nach den angewandten Reaktionsbedingungen erhält man 3-*α*/β-Stereoisomerengemische, in denen ein Isomeres unterschiedlich stark überwiegen kann. Eine hohe Selektivität von 3 *α* : 3 β > 90 : 10 läßt sich erzielen, wenn man zunächst die C-C Doppelbindung mit Pd/C als Katalysator bei mäßigen Überdrücken von 1 - 5 bar hydriert und dann die Nitrilgruppe bei 30 - 50 bar über Rh auf Al₂O₃ reduziert. Eine chromatographische Trennung der Diasteromeren kann man beispielsweise auf der Aminstufe erreichen. Die Amine IVa (15) werden wiederum mit (peptidchemischen) Amidbildungsmethoden zu den monomeren Gallensäurederivaten I (16) umgesetzt (z. B. EEDQ-Methode oder DCC/HOBT-Methode, beide siehe oben). Sowohl die Verwendung der Verbindung 9 als auch die Einführung von *α*,ω-Diolen mittlerer Kettenlänge gemäß Verbindung 2 ermöglichen es, Verbindungen des Typs I (16) herzustellen.
   Die vorstehend beschriebenen Reaktionen sind im Formelschema 3 zusammengefaßt. 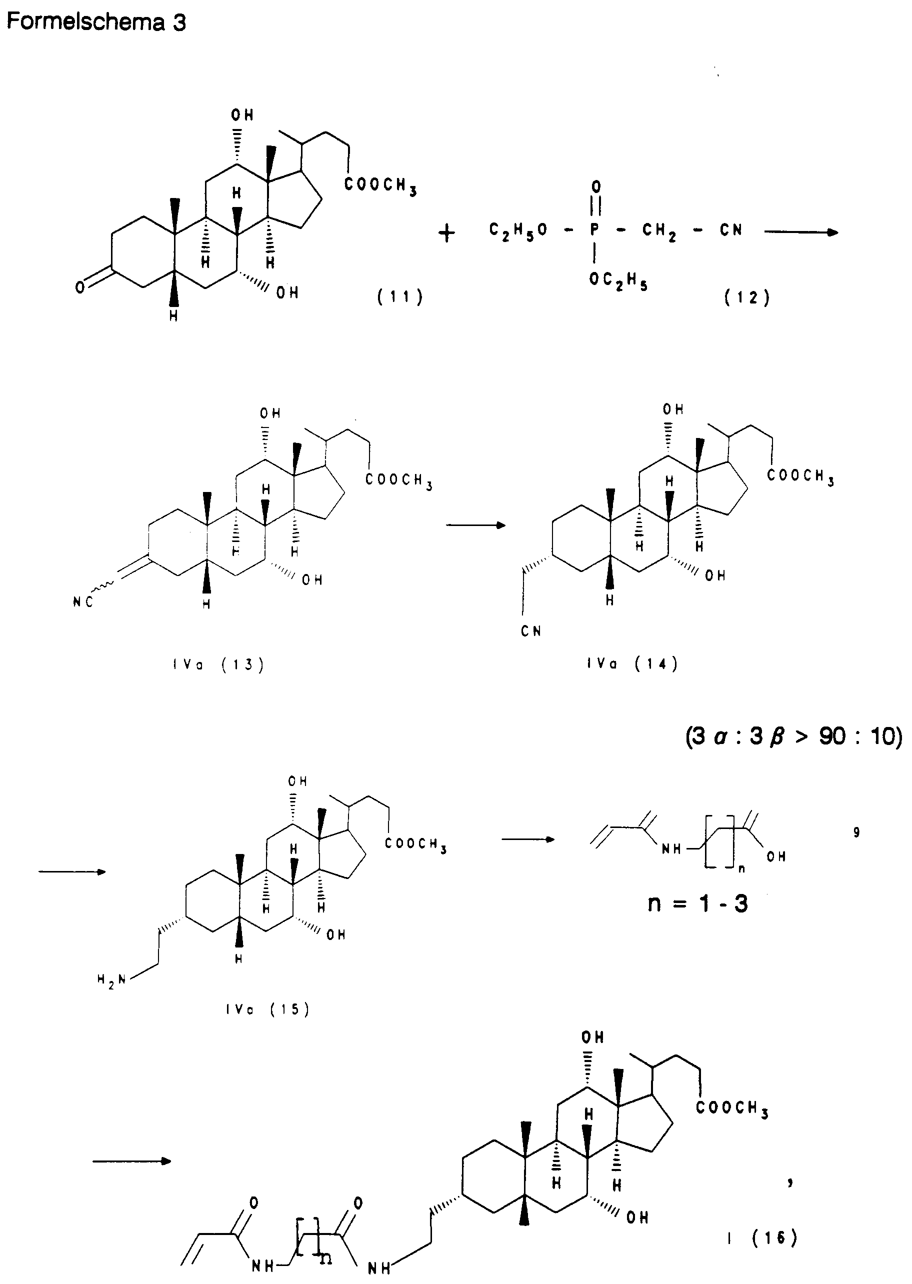
4) Das Gallensäurederivat G kann gemäß Formel III als Rest B sowohl als freie Säure (B = OH), als Alkalisalz (B = O-Alkali) oder als Ester (B = O-C₁-C₁₂-Alkyl, O-Allyl oder O-Benzyl) vorliegen. Im folgenden wird die Herstellung von Estern und ihre Verseifung beschrieben. Dabei ist es u. U. vorteilhaft, OH-Gruppen durch Einführung von Schutzgruppen zu schützen.
   Die Herstellung der basisch verseifbaren Methylester ist bereits unter 2) beschrieben (Verbindung (3) aus Verbindung (2)). In gleicher Weise können auch die höheren Alkylester dargestellt werden. Z. B. wird Verbindung (2) mit Ethanol, n-Propanol, i-Propanol oder n-Butanol zu den entsprechenden Estern umgesetzt. Im folgenden wird die Herstellung von bevorzugt sauer verseifbaren Ester (z. B. tert. Butylester) beschrieben:
   Literaturbekannte Gallensäurederivate der Formel (17), deren OH-Gruppen formylgeschützt vorliegen, werden mit Thionylchlorid oder vorzugsweise Oxalylchlorid in inerten Lösemitteln wie Toluol und Benzol bei Temperaturen zwischen 20 ° und 80 °C zu den entsprechenden Säurechloriden umgesetzt, und unter Zusatz von tert. Butylalkohol unter Basenzusatz (Pyridin oder Triethylamin) in inerten Lösemitteln, wie Dichlormethan bei -20° bis 0 °C in die tert. Butylester (18) überführt. Durch kurze Behandlung mit verdünnten Alkalilaugen in Lösemitteln wie Dioxan oder THF, vorzugsweise bei 60 - 80 °C, werden die Formylschutzgruppen wieder abgespalten, ohne daß eine Verseifung der tert. Butylesterfunktion erfolgt (Verbindung IVa (19). Das Verfahren ist ebenso anzuwenden zur Herstellung der 3-Oxoderivate der Formeln (21) und (22) aus der bekannten Verbindung (20). Die beschriebene Reaktion ist im Formelschema 4a zusammengefaßt 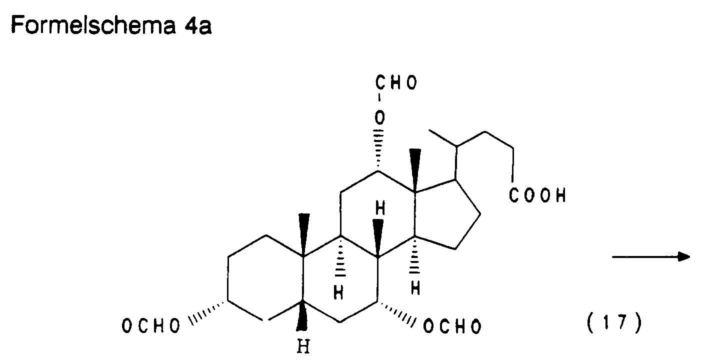 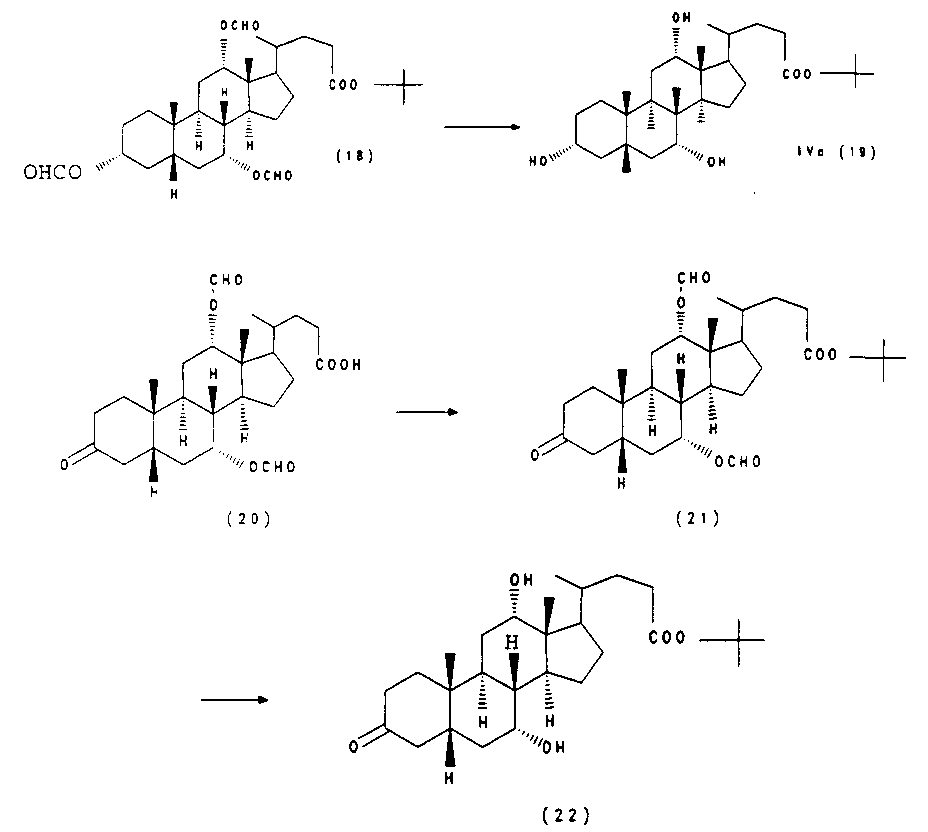 Das vorstehend beschriebene Verfahren 4a ist auch anzuwenden zur Veresterung von in 3-Position kettenverlängerten primären Alkoholen z. B. der Formel (2). Die Einführung der Formylschutzgruppen erfolgt nach üblichen Methoden (HCOOH, HClO₄). Man erhält z. B. Verbindung IVa (23), die zu den Verbindungen IVa (24) und IVa (25) umgesetzt wird (vergl. Formelschema 4b). 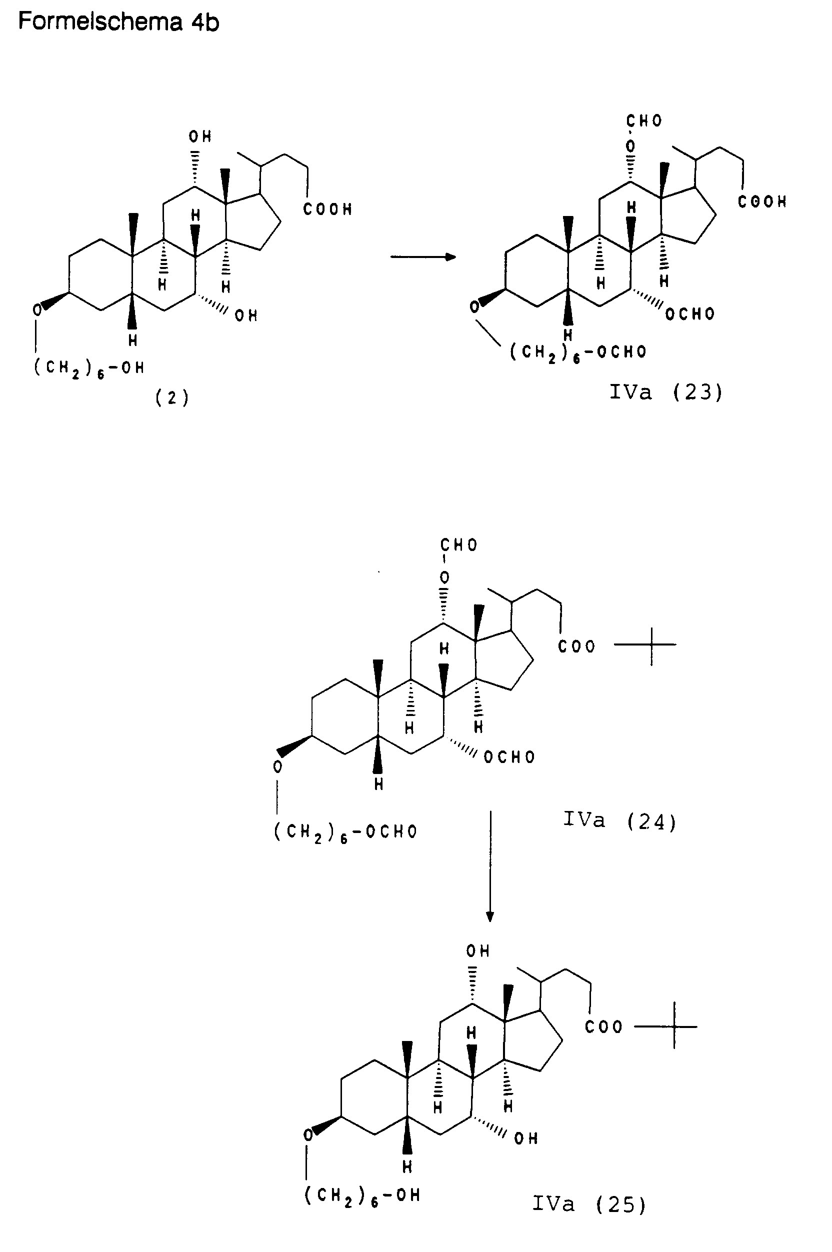 Die Butylester IVa (24) und (25) sind wie die Methylester (3) und (11) zur Herstellung der erfindungsgemäßen Gallensäurederivate der Formel I verwendbar.
5) Verbindungen der Formel I, in denen Y = -O- und Z = Alkylen
   oder Aralkylen ist, A 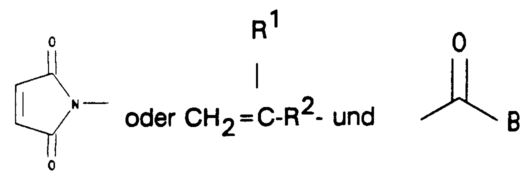 eine freie Säure, ihr Salz oder einen basisch verseifbaren Ester darstellt, können wie folgt dargestellt werden:
   Beispielsweise kann aus Verbindung (8) mit einem großen Überschuß von Allylalkohol und ohne weitere Lösemittel in Gegenwart eines Katalysators, wie z. B. Tetraethoxytitan der Allylester IVa (26) hergestellt werden. Die Umesterung wird vorzugsweise bei 60 - 100 ⁰C durchgeführt.
   Auf dem gleichen Weg kann aus dem Methylester (8) mit Benzylalkohol der Benzylester IVa (27) hergestellt werden. Die Reaktion verläuft gut mit primären und sekundären, aber nur schlecht mit tertiären Alkoholen. Die so zugänglichen Ester besitzen unterschiedliche Eigenschaften, z. B. verschiedene Stabilität gegenüber Basen bei der Verseifung; einige können im neutralen Bereich, andere unter hydrogenolytischen Bedingungen gespalten werden. Die beschriebene Reaktion ist in Formelschema 5a zusammengefaßt: 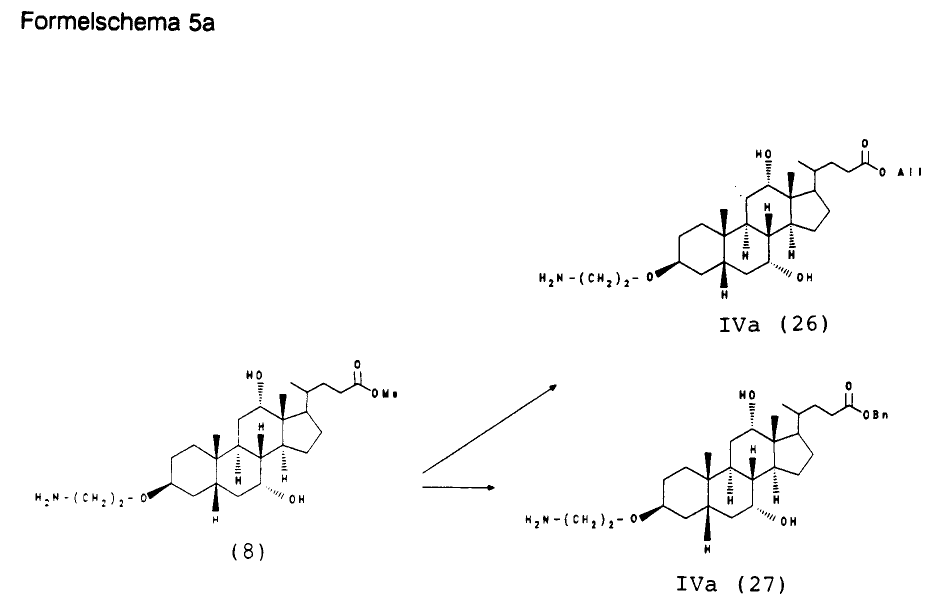 Über die Aminofunktion lassen sich polymerisierbare Gruppen in modifizierte Gallensäurederivate einführen. Beispielsweise können aus den Aminogruppen der Verbindungen (8) oder IVa (27) mit Maleinsäureanhydrid unter sauren Bedingungen, z. B. in Gegenwart von Essigsäure und bei erhöhten Temperaturen die cyclischen Maleimide I (28) oder I (29) hergestellt werden.
   Eine weitere Möglichkeit zur Einführung polymerisierbarer Gruppen besteht in der Umsetzung der Aminofunktionen zu Acrylamiden. Hierzu wird ein aktiviertes Acrylsäurederivat (Säurechlorid, gemischtes Anhydrid oder Aktivester) mit einer Aminofunktion in Gegenwart einer Base (z.B. Pyridin oder Triethylamin) umgesetzt. Für die Reaktion eignen sich Lösungsmittel wie Dichlormethan, DMF oder THF und Temperaturen von -20 bis + 50 °C. Verbindung I (30) kann nach diesem Verfahren aus (8) und Acrylsäurechlorid hergestellt werden. Die basische Verseifung des Methylesters führt zur freien Säure I (31).
   Die beschriebene Reaktion ist in Formelschema 5b zusammengefaßt: 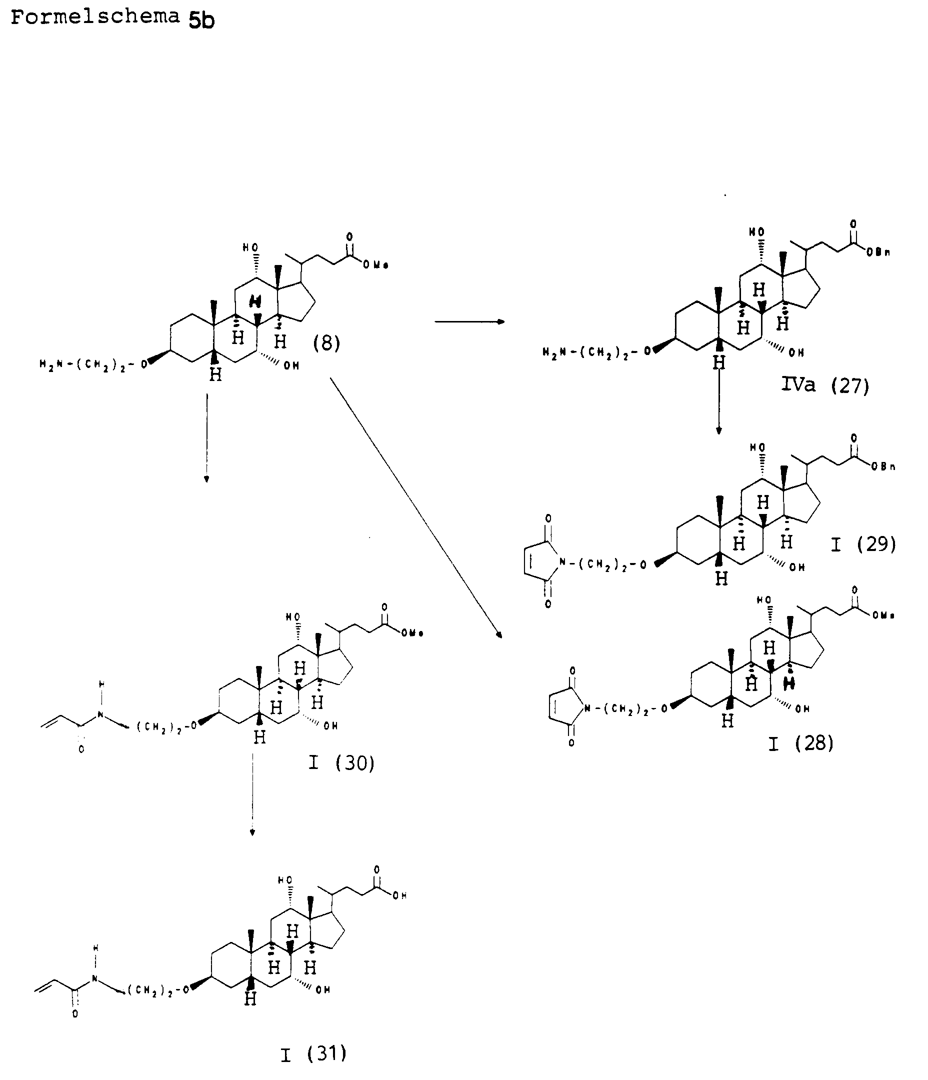
6) Verbindungen der Formel I, in denen 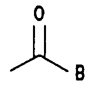 ein unter sauren Bedingungen spaltbarer Ester darstellt, werden wie folgt hergestellt:
   Z. B. kann Verbindung (32), in der Y = -O- und Z = Alkylen ist, zu Verbindung
   I (41) umgesetzt werden, in der A 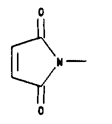 ist:
   Verbindung (32) wird selektiv an der primären Hydroxylgruppe silyliert, z. B. mit t-Butyldimethylsilylchlorid (= TBDMS) oder t-Butyldiphenylsilylchlorid in Dichlormethan, Trichlormethan, Tetrahydrofuran oder Dimethylformamid in Gegenwart einer Base (z. B. Imidazol oder Triethylamin), bei Temperaturen von -20 - 40 °C. Die Verseifung von (33) unter basischen Bedingungen führt zur freien Säure IVa (34). Hieraus kann nach Aktivierung der Carboxylgruppe, z. B. als Aktivester, gemischtes Anhydrid oder auch Säurechlorid und Umsetzung mit t-Butanol der t-Butylester IVa (35) gewonnen werden.
   Die Silylschutzgruppe der Verbindung IVa (35) läßt sich selektiv mit Tetrabutylammoniumfluorid in einem geeigneten Lösungsmittel (z. B. Tetrahydrofuran, Ether oder Dichlormethan) freisetzen. Die so erhaltene primäre Alkoholfunktion des Derivats IVa (36) kann, wie beschrieben (Mesylierung zu IVa (37), anschließender Azidaustausch zu IVa (38) und Reduktion zu IVa (39)), in die Aminofunktion der Verbindung IVa (39) überführt werden. Aus dem Amin IVa (39) kann, wie aus (8) und IVa (27), ein Maleimid dargestellt werden.
   Durch Verseifung des t-Butylesters I (40) unter sauren Bedingungen, z. B. in Trifluoressigsäure oder in Trifluoressigsäure/Wasser-Gemischen gelangt man zur Verbindung I (41).
   Die vorstehend beschriebenen Reaktionen sind in Formelschema 6a zusammengefaßt. 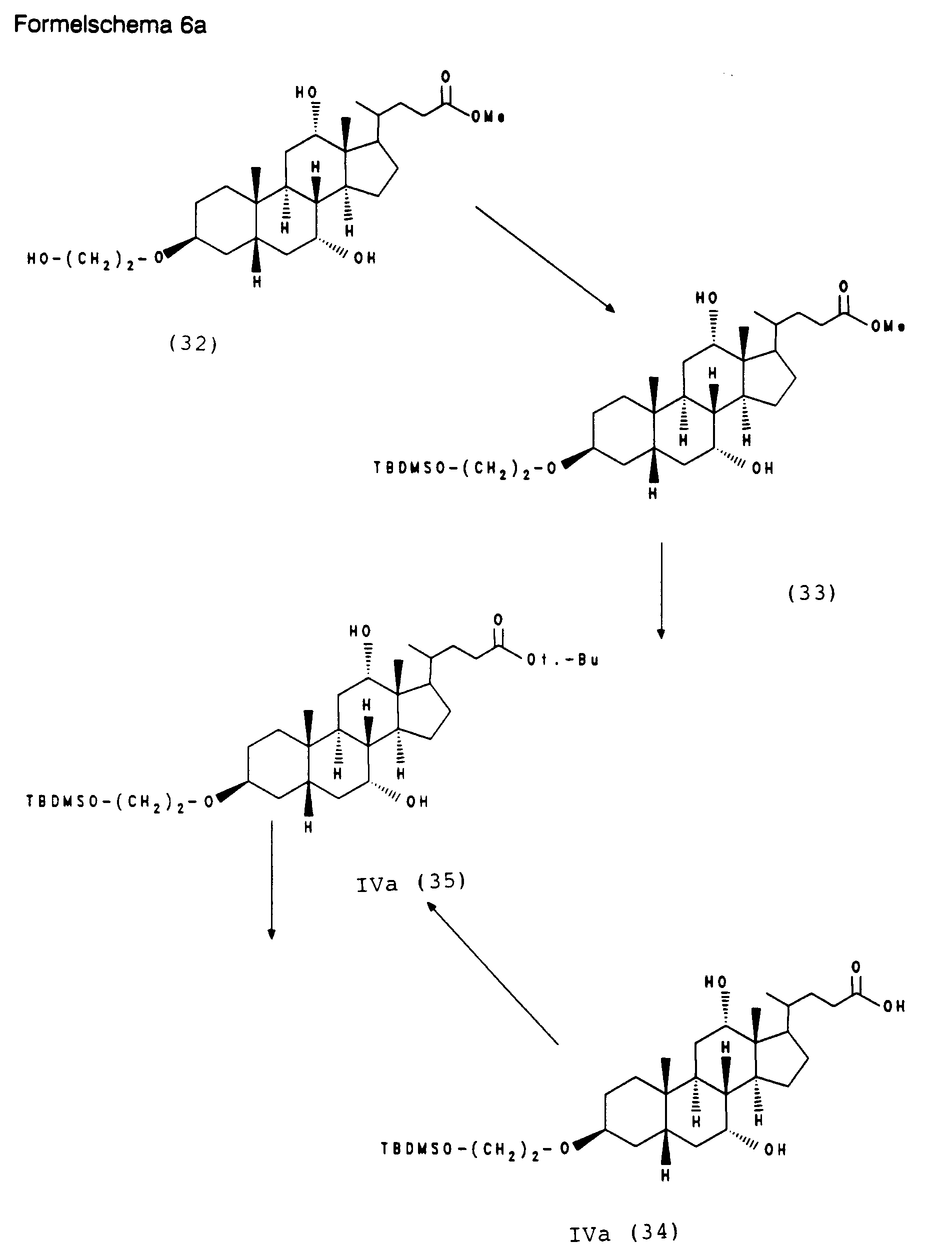 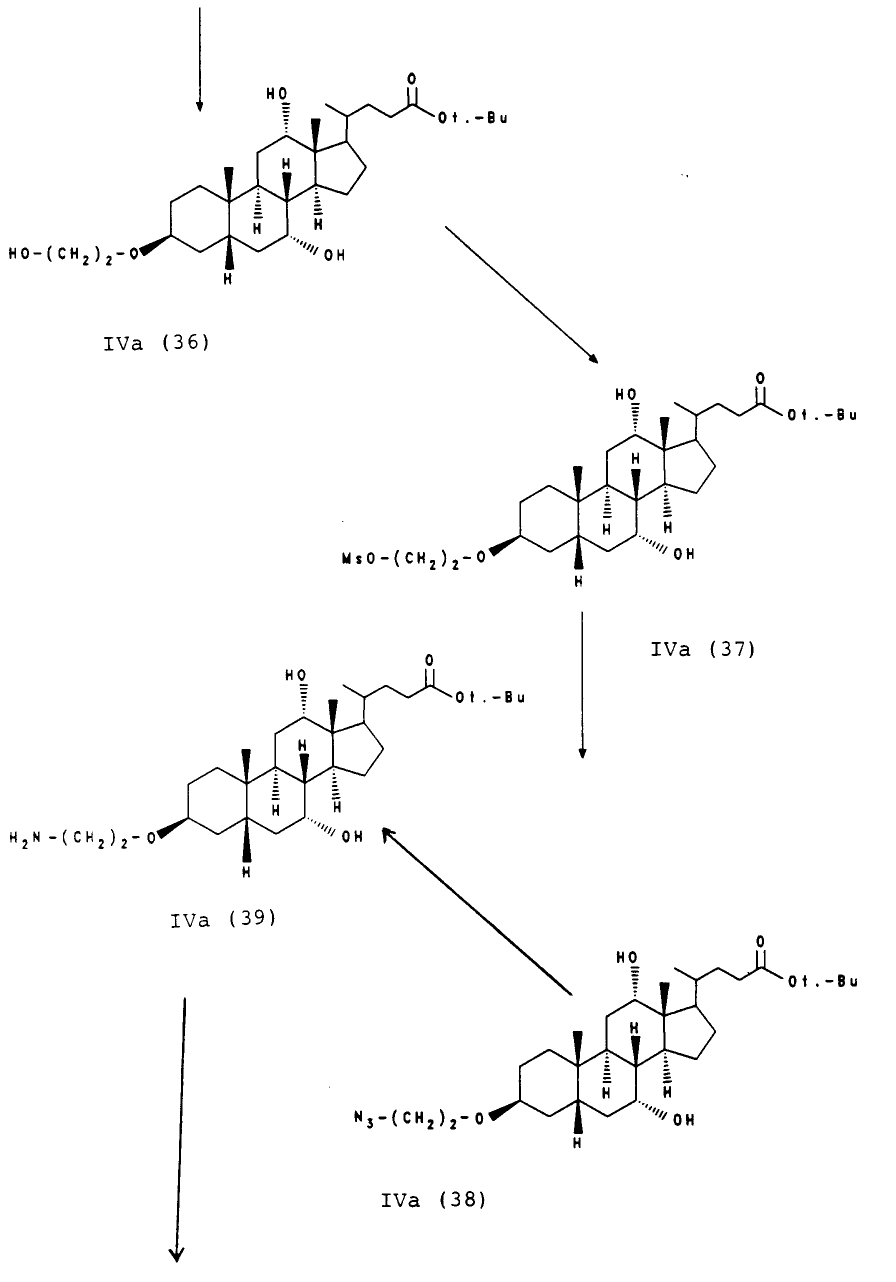 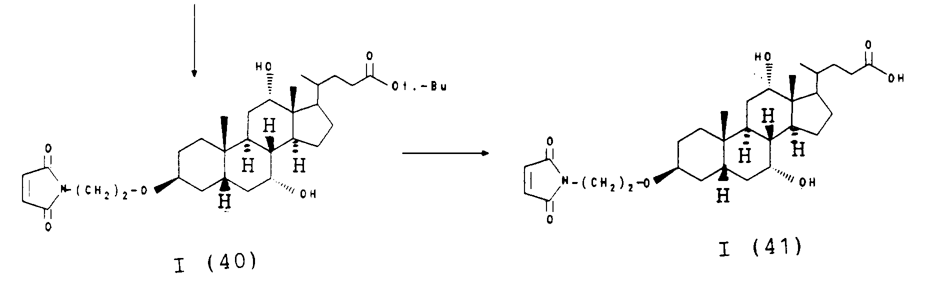 Ebenso kann Verbindung (42), in der Y = -O- und Z = Alkylen oder Aralkylen ist, zu
   Verbindung I (48) umgesetzt werden, in der A 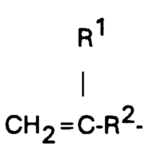 darstellt:
   Verbindung (42) kann selektiv mit Benzylbromid in Gegenwart einer geeigneten Base (Triethylamin oder Hünigbase) ohne weitere Lösungsmittel bei erhöhter Temperatur, von 70 bis 130 °C benzyliert werden. Die so erhaltene Verbindung IVa (43) kann analog der Reaktionssequenz (33) bis IVa (35) in den t-Butylester IVa (45) überführt werden. Die Benzyletherfunktion von Verbindungen des Typs IVa (45) wird mit Wasserstoff unter einem Druck von 1 - 5 bar in Gegenwart eines Katalysators wie z. B. Palladium auf Kohle, Palladium oder Platin in einem geeigneten Lösungsmittel wie beispielsweise Methanol, Essigsäureester oder Tetrahydrofuran zu Alkoholen wie die Verbindung IVa (46) gespalten. Alkohole des Typs IVa (46) können nach den bereits beschriebenen Verfahren (Verbindungen IVa (36) bis IVa (39), bzw. (3) bis (6)) in Aminoderivate wie z. B. Verbindung IVa (47) überführt werden. Durch Reaktion mit geeigneten aktivierten Acrylsäurederivaten erhält man Acrylamide des Typs I (48).
   Die Reaktionsfolge ist in Formelschema 6b zusammengefaßt. 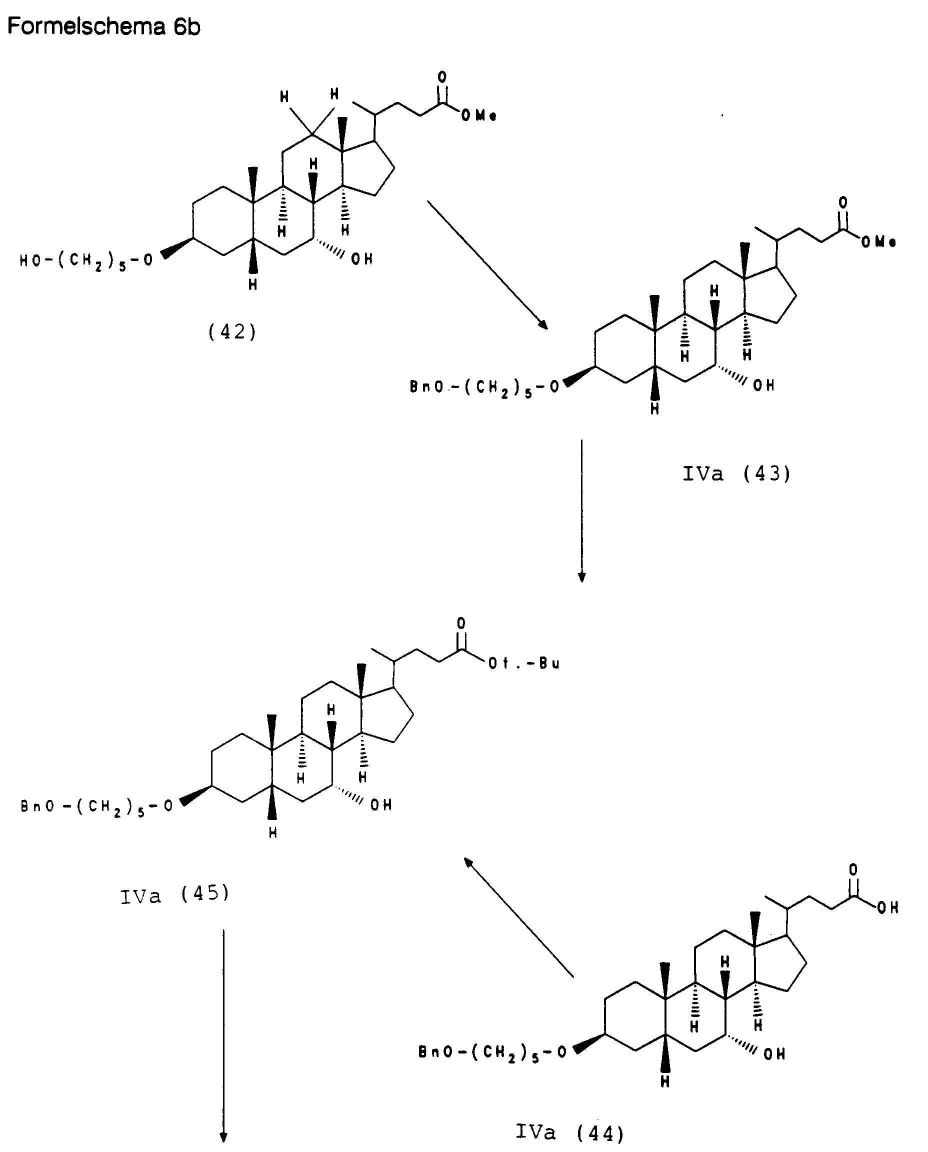 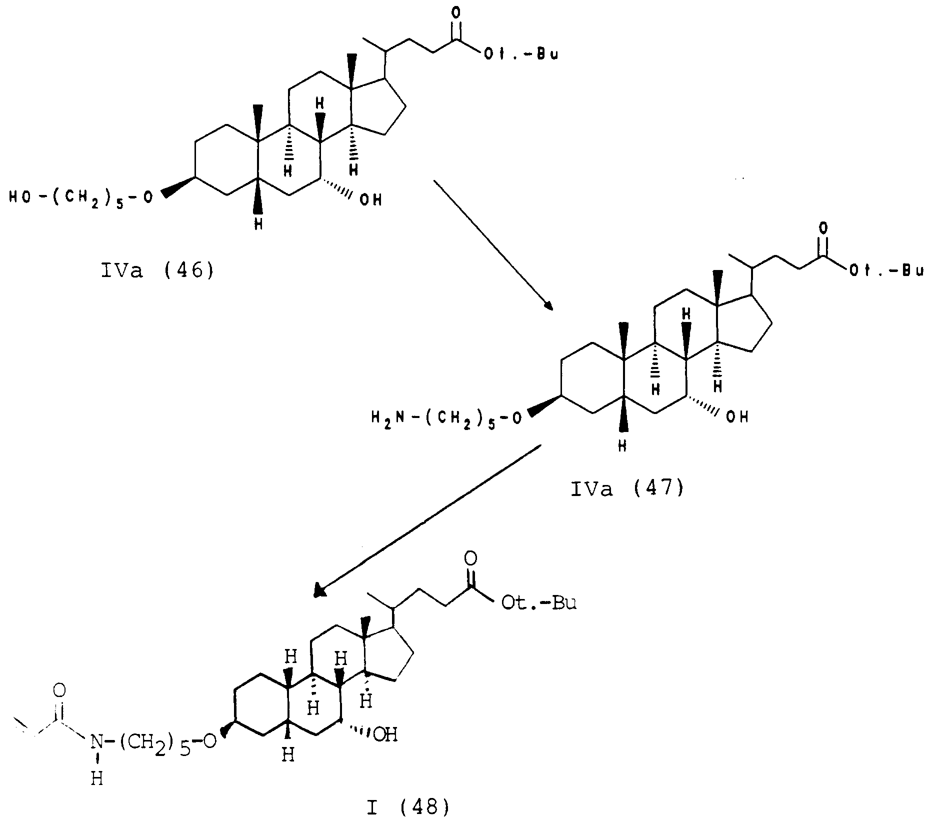
7) Verbindungen der Formel I, in denen Y = 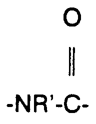 und A 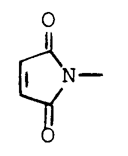 oder 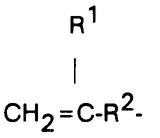 bedeuten, können wie folgt dargestellt werden:
   Für eine direkte Anknüpfung der Acrylsäure kann das Amin (49) mit einer aktivierten Acrylsäure nach den bereits beschriebenen Verfahren zur Reaktion gebracht werden, um Produkte des Typs I (50) zu erhalten. Zur Einführung des Restes X können aktivierte Formen von ω-Halogencarbonsäuren, wie z. B. 6-Bromhexansäurechlorid, mit dem Amin (49) unter den gleichen, oben genannten Bedingungen, zu Verbindungen des Typs IVa (51) umgesetzt werden. Durch nucleophile Substitution wird das Halogenatom mit Alkaliaziden, wie Natriumazid, in Dimethylformamid oder Dimethylsulfoxid bei Temperaturen von 70 - 130 °C ersetzt.
   Die resultierenden Azidoverbindungen vom Typ IVa (52) werden zu Aminen reduziert, entweder durch Hydrierung mit Wasserstoff in Gegenwart eines Katalysators (Palladium auf Kohle, Platin oder Raney-Nickel) oder durch Umsetzung mit Tributylphosphin oder Triphenylphosphin bei Zimmertemperatur in einem geeigneten Lösungsmittel wie Tetrahydrofuran. Das Acrylamid I (54) kann aus dem Amin IVa (53) nach den für I (50) beschriebenen Verfahren hergestellt werden.
   Die beschriebenen Reaktionen sind in Formelschema 7 zusammengefaßt. 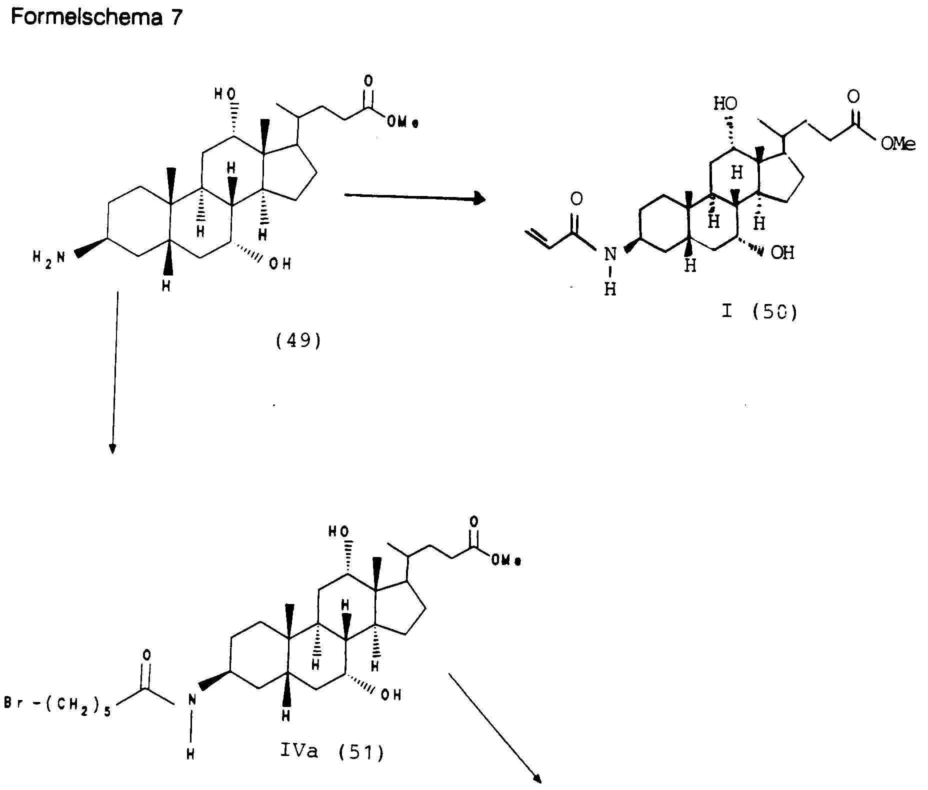 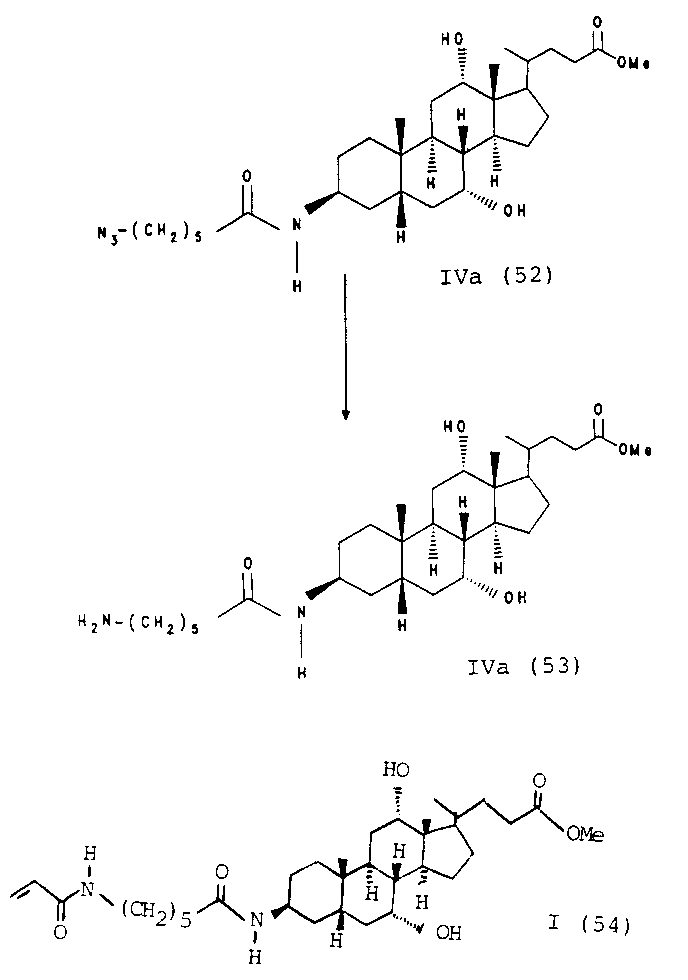
8) Verbindungen der Formel I, in denen o = Null, Z = Alkylen oder Aralkylen ist, A 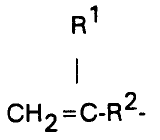 bedeutet, worin R² eine Einfachbindung bedeutet und 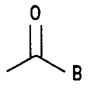 eine freie Säure, ihr Alkalisalz oder einen basisch verseifbaren Ester bedeutet, können wie folgt hergestellt werden:
   Das 3-Ketogallensäurederivat (55) wird mit Grignardverbindungen aus geschützten ω-Hydroxyhaloalkanen zur Reaktion gebracht. Beispielsweise wird Trimethylsilyloxypropylmagnesiumbromid in Ether oder Tetrahydrofuran bei Temperaturen von 20 bis 70 °C an die Verbindung (55) addiert. Daraus resultiert das Diastereomerengemisch IVa (56) im Verhältnis von ca. 3 : 1, das chromatographisch getrennt werden kann. Die Silylschutzgruppe kann mit Tetrabutylammoniumfluorid in Tetrahydrofuran oder auch Ether bei 0 - 40 °C unter Bildung des Alkohols IVa (57) entschützt werden.
   Es können auch Gruppen X eingeführt werden, die bereits eine polymerisierbare Doppelbindung enthalten. Das Keton (55) kann mit ω-Vinylalkylmagnesiumbromid oder daraus herstellbaren Vinylalkylmanganiodidverbindungen zur Reaktion gebracht werden. So erhält man z. B. unter den oben für Grignardreaktionen beschriebenen Bedingungen aus Vinylmagnesiumbromid und dem Keton (55) ein chromatographisch trennbares Diastereomerengemisch der Vinylverbindungen IVa (58) in Verhältnis von ca. 5 : 1. Unter den gleichen Reaktionsbedingungen erhält man auch durch Umsetzung von Butenylmanganiodid mit dem Keton (55) die Verbindungen IVa (59) im Verhältnis größer 10 : 1.
   Diese polymerisierbaren Verbindungen können auch zu weiteren wichtigen Synthesebausteinen umgesetzt werden. Beispielsweise kann die Doppelbindung der Verbindung IVa (59) durch Hydroborierung mit Boran oder 9-Borabicyclononan in einem Lösungsmittel wie z. B. Tetrahydrofuran bei -10 - 40 °C in die Hydroxyverbindung IVa (60) überführt werden, die wiederum einer Verbindung des Typs IVa (57) entspricht.
   Die beschriebenen Reaktionen sind in Formelschema 8 zusammengefaßt. 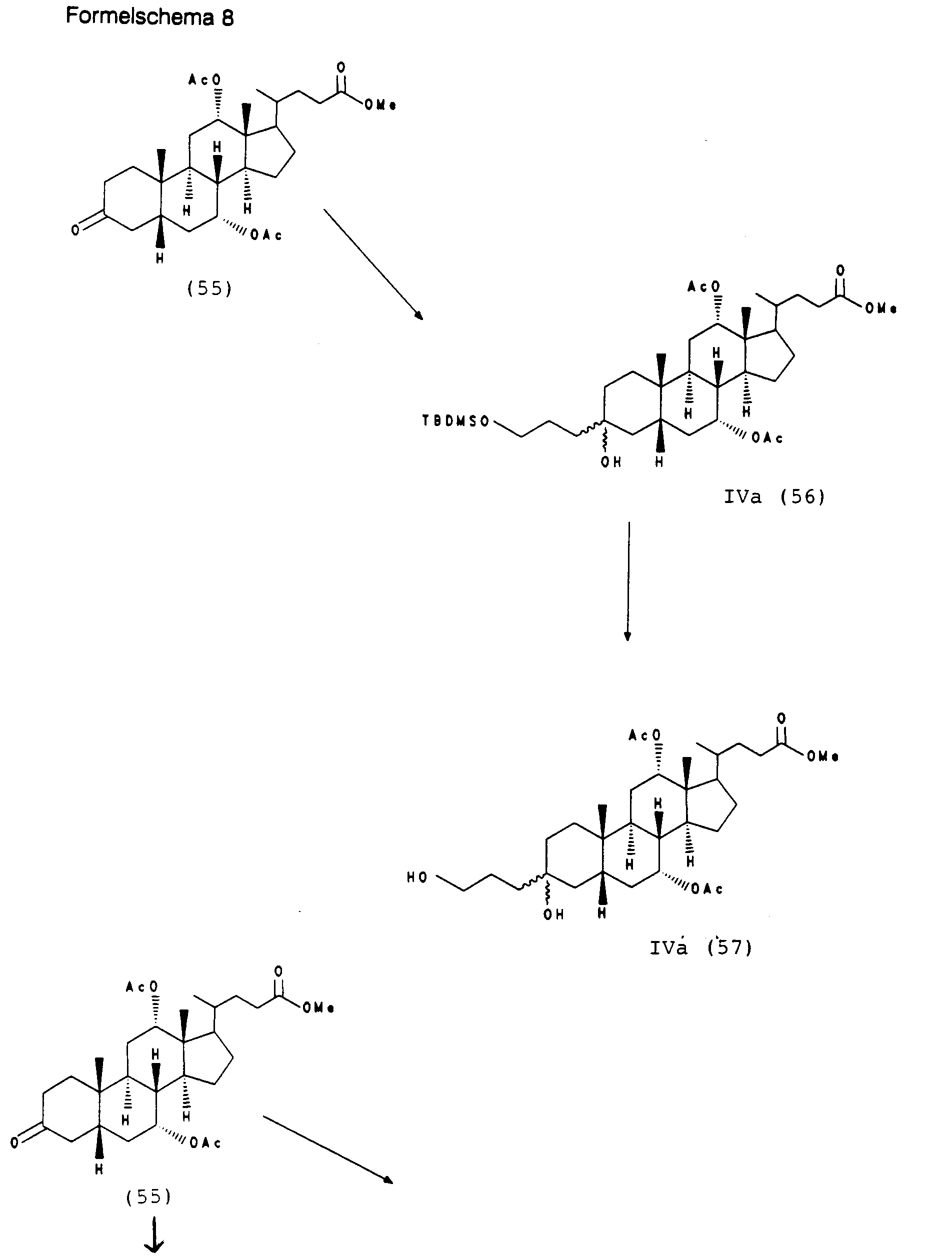 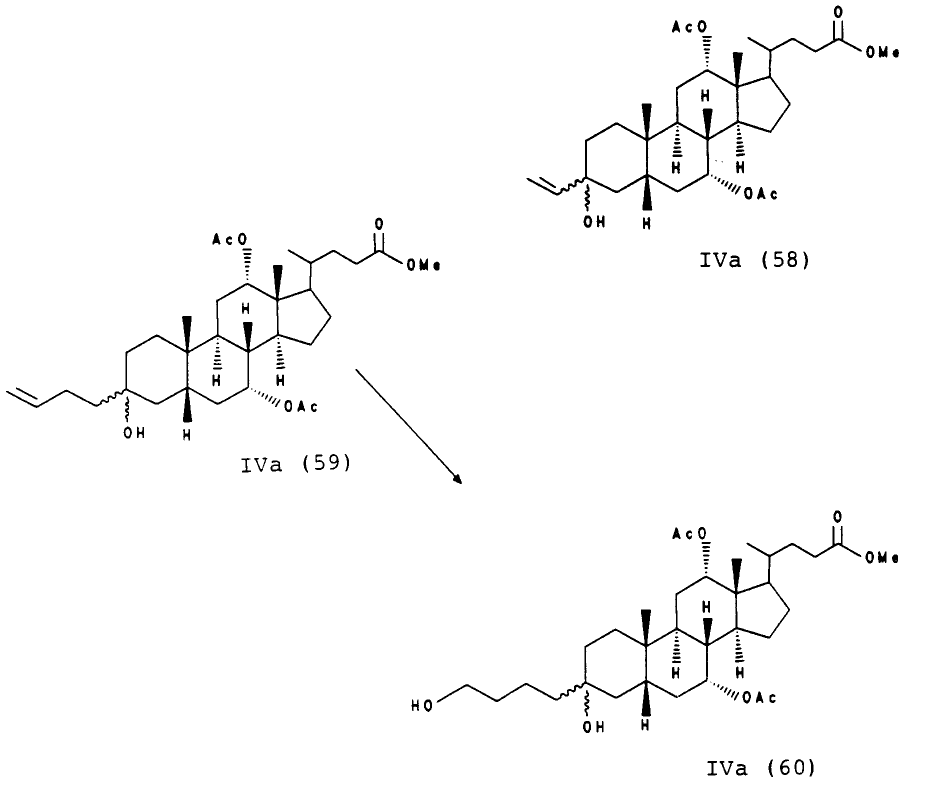
9) Verbindungen der Formel I, in denen Z Alkylen bedeuten, wobei mindestens eine Methylengruppe durch 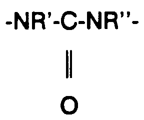 ersetzt ist, wie Aminoverbindungen vom Typ (8), lassen sich auch mit Isocyanaten, die eine polymerisierbare Gruppe enthalten, zu den gewünschten Gallensäurederivaten umsetzen. Wird z.B. das Isocyanat (61) mit dem Amin (8) in einem geeigneten Lösungsmittel wie z.B. Dichlormethan, Trichlormethan oder Tetrahydrofuran bei Temperaturen von -10 bis 30°C zur Reaktion gebracht, erhält man die Verbindung I (62). Durch basische Verseifung der Methylesterfunktion mit Natriumhydroxid in Ethanol/Wasser wird die freie Carbonsäure I (63) erhalten.
   Nach dem gleichen Verfahren gelangt man durch Reaktion des Amins 64 mit dem Isocyanat (61) zur Verbindung I (65), bei der die funktionelle Gruppe über die 7-Position der Gallensäure gebunden ist.
   Die beschriebene Reaktion ist in Formelschema 9 zusammengefaßt

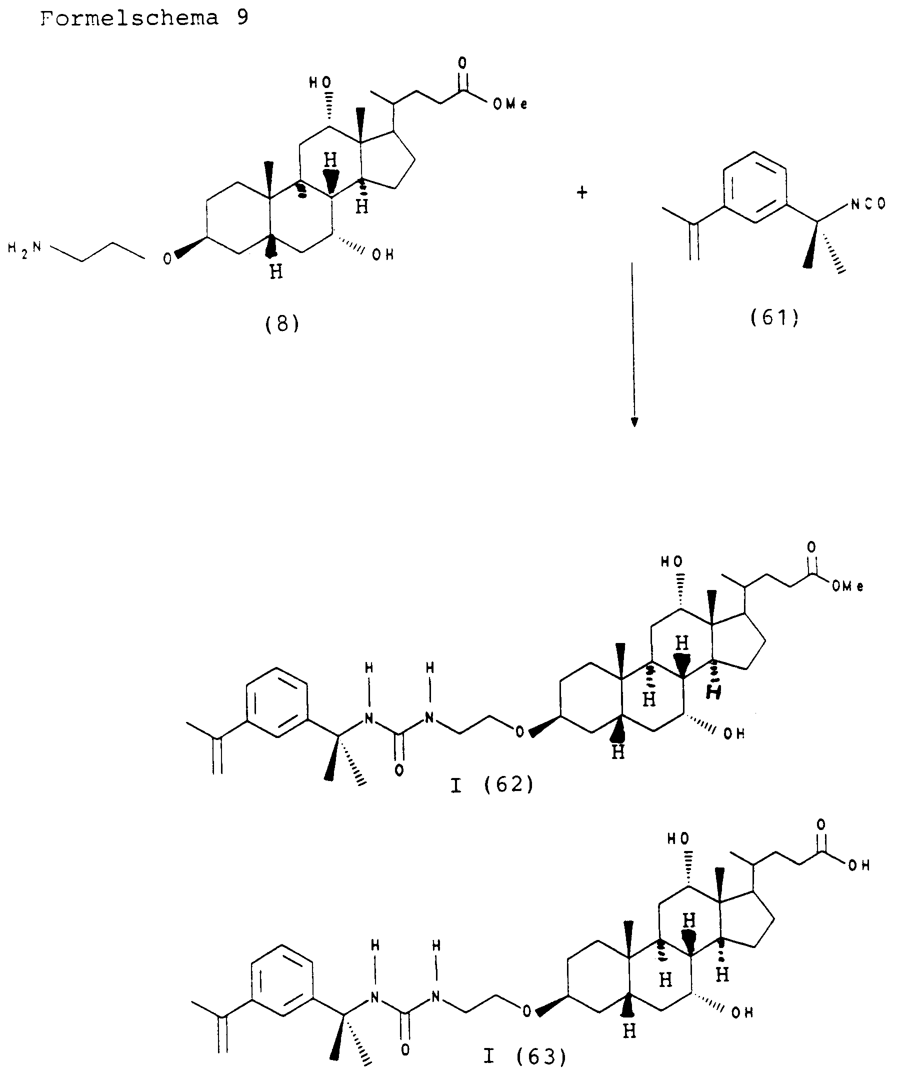

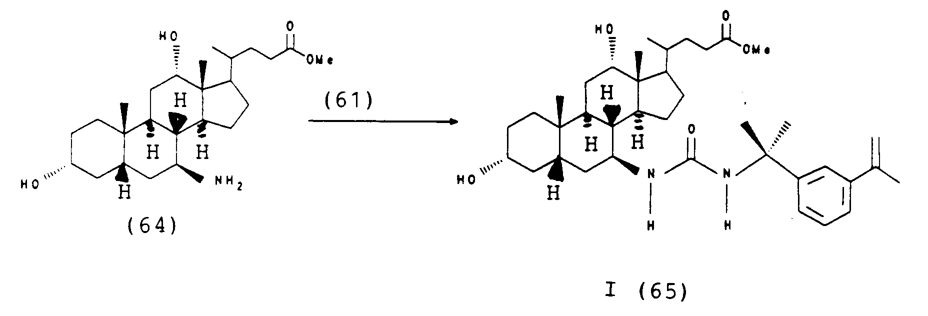

### Beispiel 1

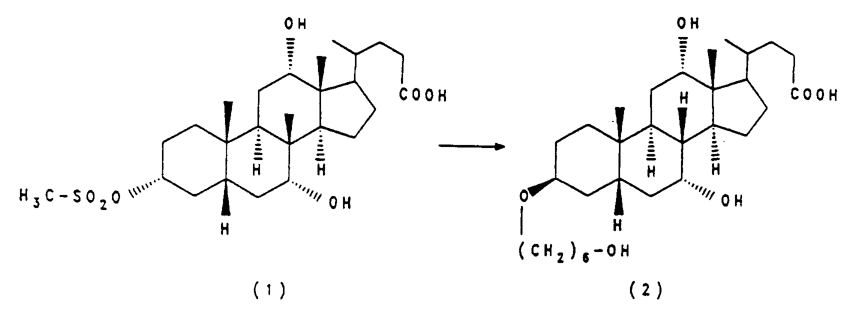

Zu einer Mischung von 570 g 1,6-Hexandiol und 120 ml Pyridin gibt man 100 g (0,205 mol) (1) und erwärmt innerhalb von 45 min auf 100°C. Man rührt 3 h bei 100°C, kühlt ab, versetzt mit 120 ml konz. H₂SO₄ in 2,5 l Wasser und rührt 1 h bei Raumtemperatur. Man extrahiert mehrfach mit Essigester, trocknet die organische Phase und engt ein. Der Rückstand wird mit Wasser/Ether am Extraktor behandelt a) unter Zusatz von 2 H NaOH bis zur deutlich alkalischen Reaktion dann b) nach Ansäuern auf pH 2-3 mit halbkonz. HCl. Die bei b) erhaltene organische Phase wird eingeengt und durch Chromatographie gereinigt (SiO₂, Essigester).
Ausbeute 42 g (40 %)(2).
MS (FAB, 3-NBA, LiCl): 515 M+Li⁺).

### Beispiel 2

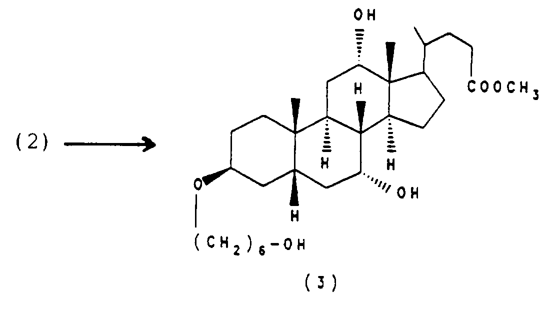

400 ml trockenes Methanol werden unter Kühlung tropfenweise mit 40 ml Acetylchlorid versetzt. Nach 1 h bei Raumtemperatur gibt man 40 g (78,6 mmol) (2), gelöst in 70 ml Methanol zu, rührt 1 h bei Raumtemperatur und läßt über Nacht im Kühlschrank stehen. Man gießt auf 2 l Eiswasser, neutralisiert mit ges. Hydrogencarbonat lösung und extrahiert mehrfach mit Ether. Die organische Phase wird getrocknet, eingeengt und der Rückstand wird chromatographisch gereinigt (SiO2, Essigester/Heptan = 4/1). Man erhält 31,5 g (77 %) (3).
MS (FAB, 3-NBA, LiCl): 529 (M+Li⁺).

### Beispiel 3a

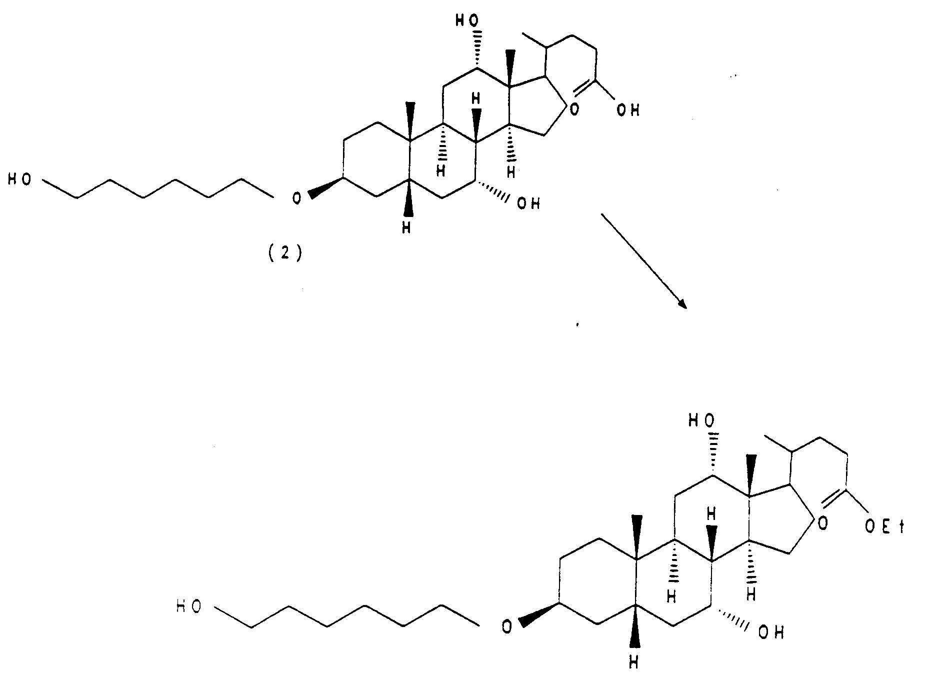

Unter Eiskühlung werden 2 ml Acetylchlorid zu 20 ml wasserfreiem Ethanol zugetropft. Nach 5 Minuten werden 2,0 g (3,93 mmol) (2) zugegeben. Es wird über Nacht bei Zimmertemperatur gerührt. Das Reaktionsgemisch wird auf 50 ml Wasser gegeben und 3 x mit Ether extrahiert. Die vereinigten organischen Phasen werden mit gesättigter NaHCO₃-Lösung gewaschen, über MgSO₄ getrocknet und eingeengt. Chromatographie auf Kieselgel (Essigester, dazu Essigester/Methanol = 9 : 1) ergibt 1,9 g (3,54 mmol, 90%) Ethylester.
C₃₂H₅₆O₆ (536), MS (FAB, 3-NBA, LiCl): 543 (M+Li⁺).

### Beispiel 3b

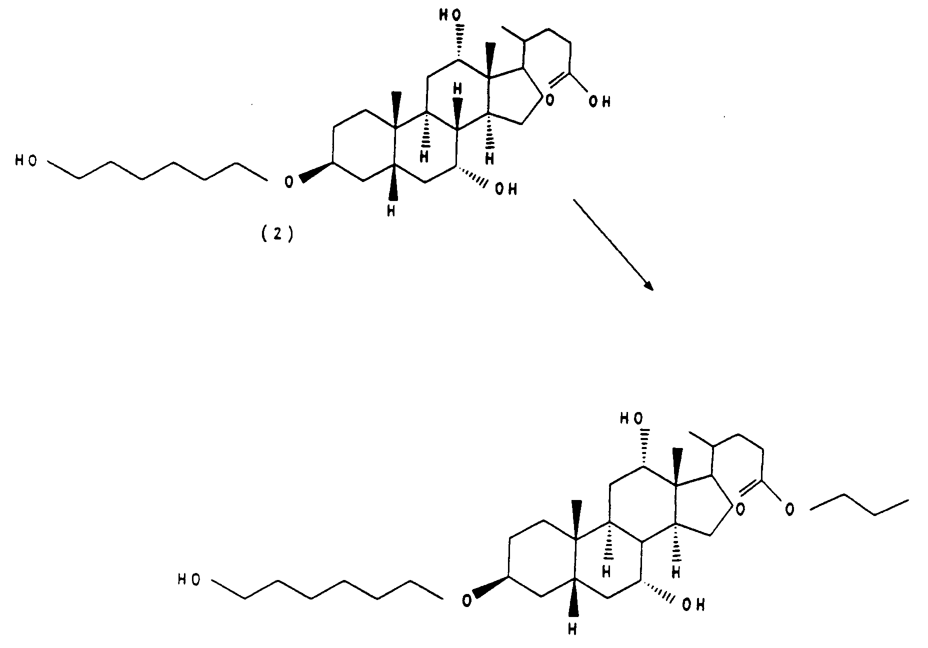

Nach dem für Beispiel 3 a beschriebenen Verfahren werden aus 2,0 g (3,93 mmol) (2), 20 ml n-Propanol und 2 ml Acetylchlorid 1,8 g (3,27 mmol, 83 %) n-Propylester hergestellt.
C₃₃H₅₈O₆ (550), MS (FAB, 3-NBA, LiCl): 557 (M+Li⁺).

### Beispiel 3 c

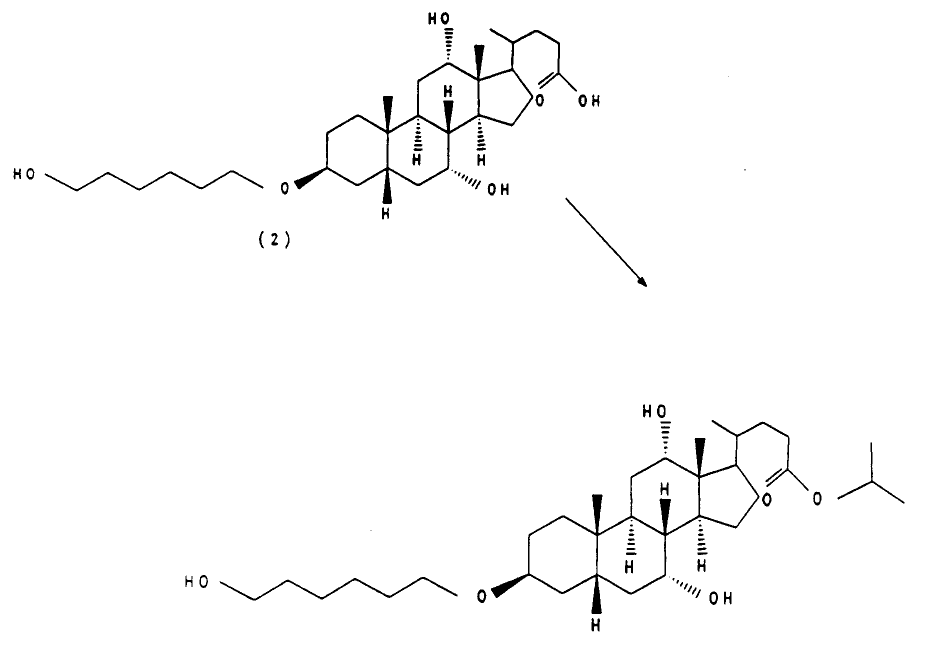

Nach dem für Beispiel 3 a beschriebenen Verfahren werden aus 2,0 g (3,93 mmol) (2), 30 ml i-Propanol und 2 ml Acetylchlorid 1,73 g (3,14 mmol, 80 %) i-Propylester hergestellt.
C₃₃H₅₈O₆ (550), MS (FAB, 3-NBA, LiCl): 557 (M+Li⁺).

### Beispiel 3 d

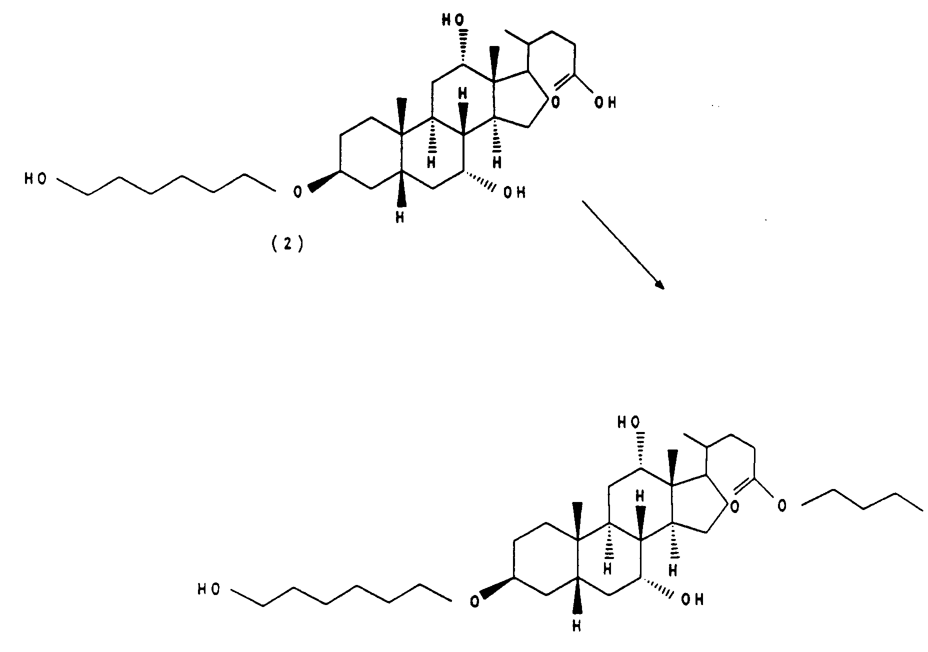

Nach dem für Beispiel 3 a beschriebenen Verfahren werden aus 2,0 g (3,93 mmol) (2), 30 ml n-Butanol und 3 ml Acetylchlorid 2,1 g (3,72 mmol, 95 %) n-Butylester hergestellt.
C₃₄H₆₀O₆ (564), MS (FAB, 3-NBA, LiCl): 571 (M+Li⁺).

### Beispiel 4

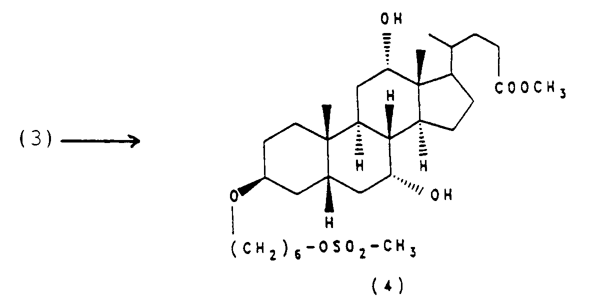

30 g (57,4 mmol) (3) werden in 150 ml trockenem Pyridin auf 0 °C gekühlt und unter Kontrolle durch Dünnschichtchromatographie portionsweise innerhalb von 2 - 3 h mit 8,3 ml Methansulfonsäurechlorid versetzt. Nach beendeter Umsetzung gibt man unter Einkühlung auf 120 ml konz. H₂SO₄ in 1,5 l Wasser und schüttelt mehrfach mit Essigester aus. Die organische Phase wird getrocknet, eingeengt und der Rückstand chromatographisch gereinigt (SiO₂, Essigester). Ausbeute: 31,4 g (91 %) (4). MS (FAB, 3-NBA, LiCl): 607 (M+Li⁺).

### Beispiel 5

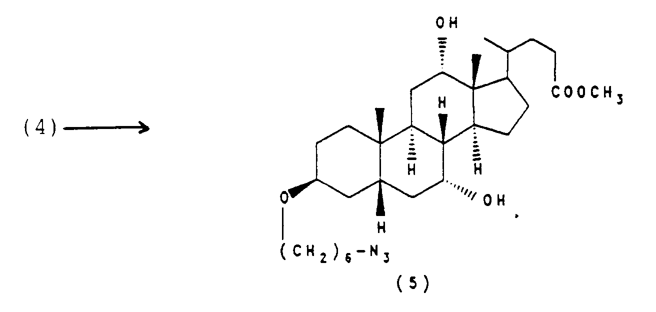

32,6 g (54,2 mmol) (4) werden in 800 ml Dimethylformamid unter Argon mit 4,6 g Natriumazid versetzt und 2 h auf 80 °C erwärmt. Man läßt über Nacht bei Raumtemperatur stehen, gießt auf 1 l Wasser und extrahiert mehrfach mit Essigester. Die organische Phase wird getrocknet und eingeengt. Der Rückstand wird mit Wasser versetzt und mit Ether mehrfach extrahiert. Nach Trocknung und Abtrennung von Lösemittel erhält man 26 g (5), das als Rohprodukt weiter eingesetzt werden kann. MS (FAB, 3-NBA, LiCl): 554 (M+Li⁺).

### Beispiel 6a

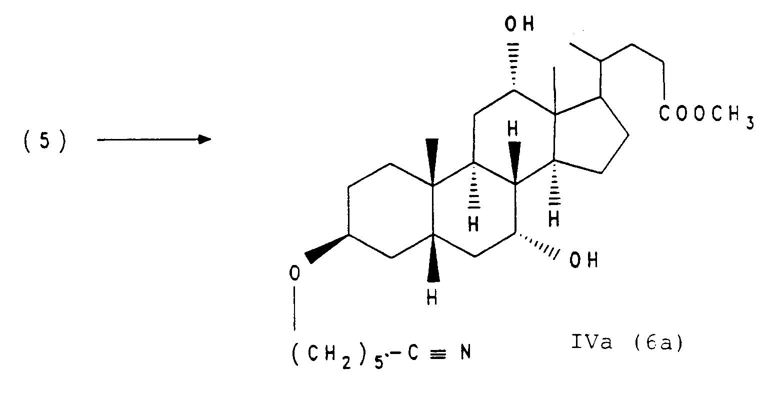

25,9 g Rohprodukt (5) werden in 500 ml Essigester gelöst und mit 5 g Palladium/Kohle (10 %) in der Schüttelende hydriert. Nach beendeter Reaktion saugt man ab, engt ein und reinigt den Rückstand durch Säulenchromatographie (SiO₂, Essigester). Man erhält 17,1 g IVa (6 a) (61% bezogen auf (4)).
MS (FAB, 3-NBA, LiCl): 524 (M+Li⁺).

### Beispiel 6 b

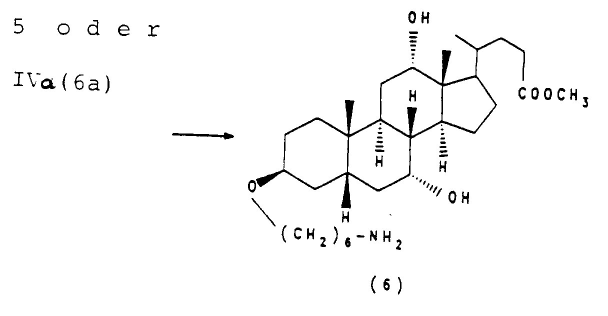

17 g (32,8 mmol) IVa (6a) werden in 500 ml Methanol + ca. 50 ml konz. Ammoniakwasser gelöst, mit 4 g 5 %igem Rhodium auf Al₂O₃ versetzt und 24 h bei 20 bar H₂ und Raumtemperatur hydriert. Nach beendeter Reaktion wird abgesaugt. eingeengt und der Rückstand durch Chromatographie gereinigt (SiO₂, CH₂Cl₂/MeOH/NH₃ konz. = 100/10/5). Man erhält 12,7g (74%) (6). MS (FAB, 3-NBA, LiCl): 528 (M+Li⁺), 522 (M+H⁺).

(6) kann ebenfalls aus dem Rohprodukt (5) direkt durch Hydrierung über Rhodium auf Al₂O₃ unter o. a. Bedingungen hergestellt werden. Aus 20 g Rohprodukt (5) erhält man 9,6 g (6) (42 % bezogen auf (4)).

### Beispiel 7

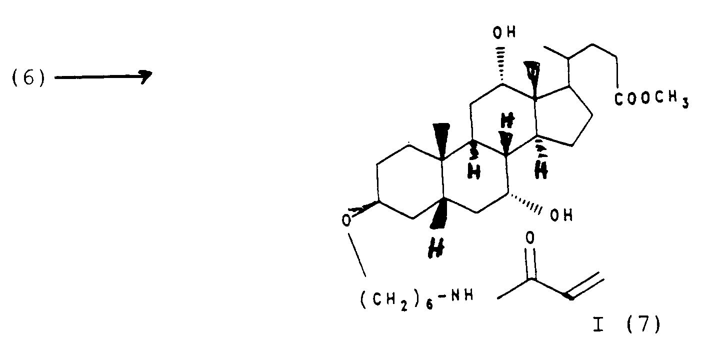

4,5 g (8,6 mmol) (6) und 1,4 ml Triethylamin werden in 200 ml trockenem CH₂Cl₂ bei - 8 °C bis - 4 °C tropfenweise mit 1 ml Acrylsäurechlorid in 10 ml CH₂Cl₂ versetzt.

Nach 1 h bei 0 °C und 1 h bei Raumtemperatur wird auf Wasser gegossen, mit CH₂Cl₂ extrahiert, die organische Phase gewaschen, getrocknet und eingeengt. Nach Chromatographie (SiO₂, Essigester) erhält man 3,5 g (70,5 %) I (7).
Schmelzpunkt: 125 °C. MS (FAB, 3-NBA, LiCl): 562 (M+Li⁺).

### Beispiel 8

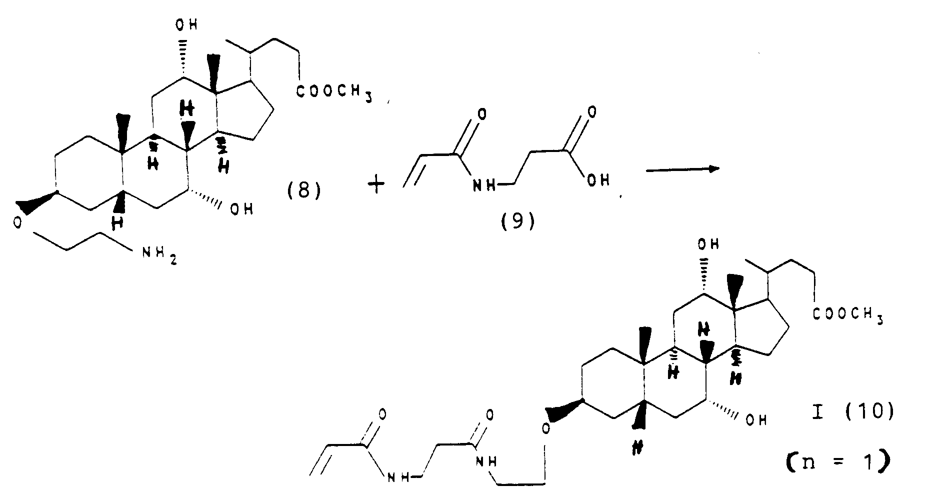

1,91 g (4,1 mmol) (8) werden in 200 ml gereinigtem Essigester mit 0,61 ml Triethylamin und 30 mg Hydrochinon gelöst. Dazu gibt man 1,1 g 1,2-Dihydro-2-ethoxychinolin-1-carbonsäureethylester (EEDQ) und 572 mg (9) und erhitzt mehrere Stunden unter Kontrolle durch Dünnschichtchromatographie zum Rückfluß.

Nach beendeter Reaktion wird mit 200 ml Essigester verdünnt, mit KHSO₄-Lösung und mit Wasser gewaschen. Die organische Phase wird getrocknet und eingeengt. Chromatographie des Rückstands (SiO2, Essigester/MeOH = 10/1) ergibt 1,2 g (50 %) I (10).
MS (FAB, 3-NBA, LiCl): 597 (M+Li⁺).

### Beispiel 9

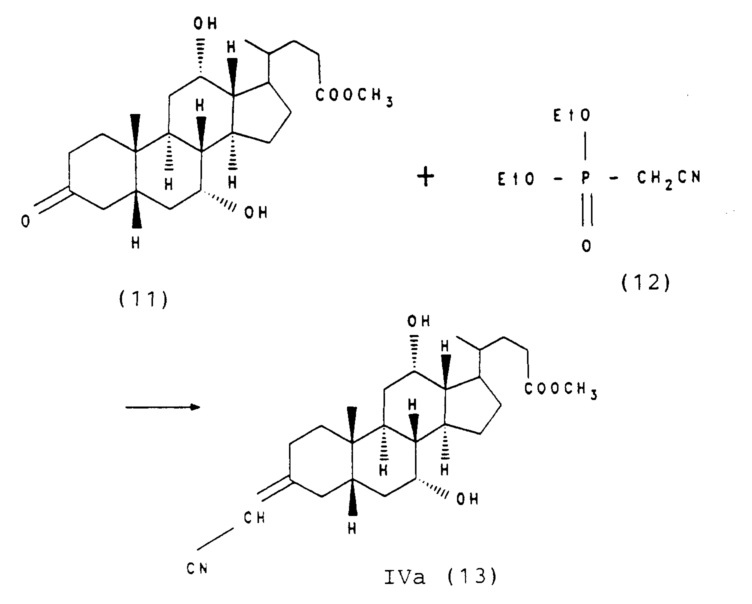

4,4 g 60 % Natriumhydrid-Suspension werden unter Kühlung und Argon in 250 ml trockenes Methanol eingetragen. Dazu gibt man tropfenweise 18 ml (0,11 mol) Cyanomethylphosphonsäurediethylester (12) und rührt 1 h bei Raumtemperatur. Dann gibt man tropfenweise 42 g (0,1 mol) (11) in 450 ml Methanol hinzu und rührt 2 h bei Raumtemperatur. Die Reaktionsmischung wird in der Kälte eingeengt und der Rückstand zwischen CH₂Cl₂ und Wasser verteilt. Die abgetrennte wäßrige Phase wird mehrfach mit CH₂Cl₂ extrahiert. Die organischen Phasen werden getrocknet und eingeengt und der verbleibende Rückstand chromatographisch gereinigt (SiO₂, Essigester/Cyclohexan = 1/1). Man erhält 34,5 g (78 %) IVa (13).
MS (FAB, 3-NBA, LiCl): 450 (M+Li⁺).

### Beispiel 10

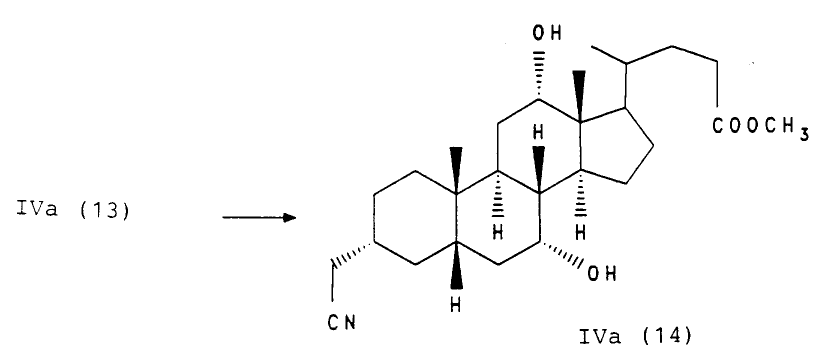

35 g (78.8 mol) IVa (13) werden in 1,2 l Methanol gelöst und mit 5 g 10-proz. Palladium/Kohle in der Schüttelente hydriert. Nach Filtration, Einengen und Chromatographie (SiO₂, Essigester/Cyclohexan = 4/1) erhält man 33,5 g (95 %) IVa (14).
MS (FAB, 3-NBA, LiCl): 452 (M + Li⁺).
Dabei überwiegt das 3*α*-Isomer entsprechend der Analyse von IVa (15) [hergestellt nach a) und b)] im Verhältnis ≧ 95 : 5.

### Beispiel 11

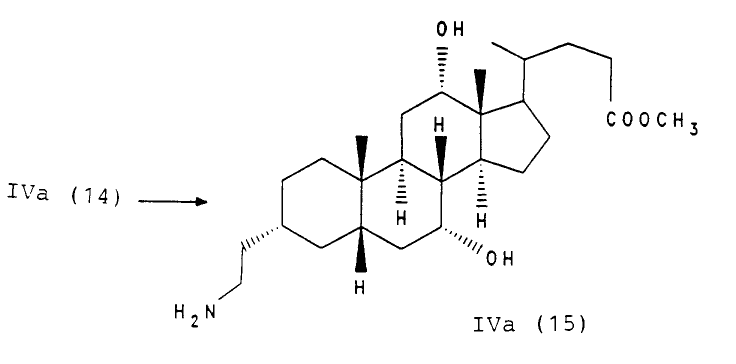

a) 15 g (33,8 mmol) IVa (14) werden in 800 ml Methanol und 10 ml konz. wäßrigem Ammoniak mit 4 g 5-proz. Rhodium auf Al₂O₃ 24 Stunden bei Raumtemperatur und 20 - 25 bar H₂ hydriert. Nach Filtrieren, Einengen und Chromatographie des Rückstands erhält man 12,5 g (81 %) IVa (15) als 3*α*/3β-Isomerengemisch ca. 1 : 1.
   Die Stereoisomere werden durch Säulenchromatographie (SiO₂, CH₂Cl₂/MeOH/ konz. wäßr. NH₃ = 100 : 15 : 5) getrennt.
b) 33 g (74 mmol) IVa (14) werden in 1,6 ml Methanol und 20 ml konz. wäßr. Ammoniak mit 8 g 5-proz. Rhodium auf Al₂O₃ bei 20 bar Wasserstoff und Raumtemperatur 24 Stunden hydriert. Nach Filtrieren, Einengen und Chromatographie (SiO₂/CH₂Cl₂/MeOH/konz. wäßr. NH₃ = 100 : 15 : 5) erhält man 0,9 g (2,7 %) weniger polares 3β-IVa (15) und 28,8 g (86,5 %) polares 3*α*-Isomer IVa (15) [3*α*/3β = 97 : 3].

### Beispiel 12

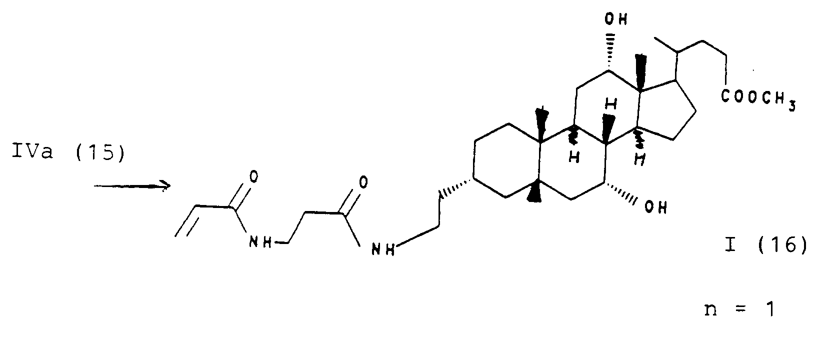

1.4 g (3,1 mmol) IVa (15) werden in 150 ml THF mit 0,45 ml Triethylamin, 20 g Hydrochinon und 850 mg EEDQ 4 h zum Rückfluß erwärmt.
Man entfernt den größten Teil des Lösungsmittels i. Vak., verdünnt mit Essigester und wäscht mit KHSO₄-Lösung und Wasser. Die organische Phase wird getrocknet, eingeengt und durch Chromatographie gereinigt (SiO₂, Essigester/MeOH = 10 : 1). Man erhält 1,2 g (67 %) I (16).
MS (FAB, 3-NBA, LiCl): 581 (M+Li⁺).

### Beispiel 13

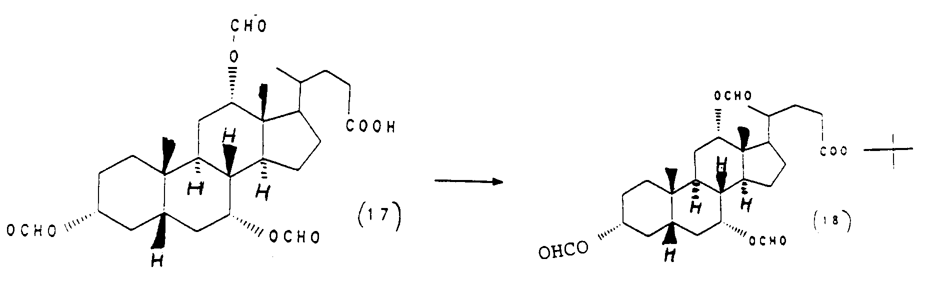

40 ml frisch über Chinolin und Leinöl destilliertes Thionylchlorid werden zu 20 g (40.5 mmol) (17) in 400 ml trockenem Toluol gegeben und unter Argon 4 Stunden auf 80 °C erwärmt. Das überschüssige SOCl₂ wird durch mehrfache Destillation mit Toluol weitestgehend entfernt, der Rückstand mit 100 ml trockenem CH₂Cl₂ aufgenommen und die Lösung bei 0°C zu einer Mischung aus 100 ml tert.-Butanol, 100 ml CH₂Cl₂ und 4 ml Pyridin gegeben. Man rührt 2 Stunden bei Raumtemperatur, gießt dann in Wasser, extrahiert nach Abtrennung der organischen Phase mit CH₂Cl₂ und reinigt den nach Trocknung und Einengen verbleibenden Rückstand durch Chromatographie (SiO₂; CH₂Cl₂/Aceton = 4 : 1). Man erhält 18,1 g (81 %) (18).
MS (FAB, 3-NBA, LiCl): 555 (M+Li⁺).

### Beispiel 14

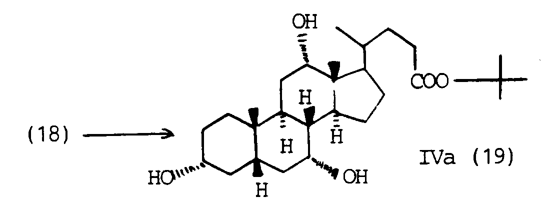

18 g (32.8 mmol) (18) werden in 250 ml Dioxan und 100 ml 2N NaOH 10 min auf dem Dampfbad erhitzt. Danach wird die Mischung i. Vak. auf ca. die Hälfte eingeengt und mit 400 ml CH₂Cl₂ und Wasser bis zur Phasentrennung versetzt. Nach Abtrennung der organischen Phase, Extrahieren mit CH₂Cl₂, Trocknung und Einengen wird der Rückstand durch Säulenfiltration gereinigt. Man erhält 14,8 g (97 %) IVa (19).
MS (FAB, 3-NBA, LiCl): 471 (M+Li⁺).

### Beispiel 15

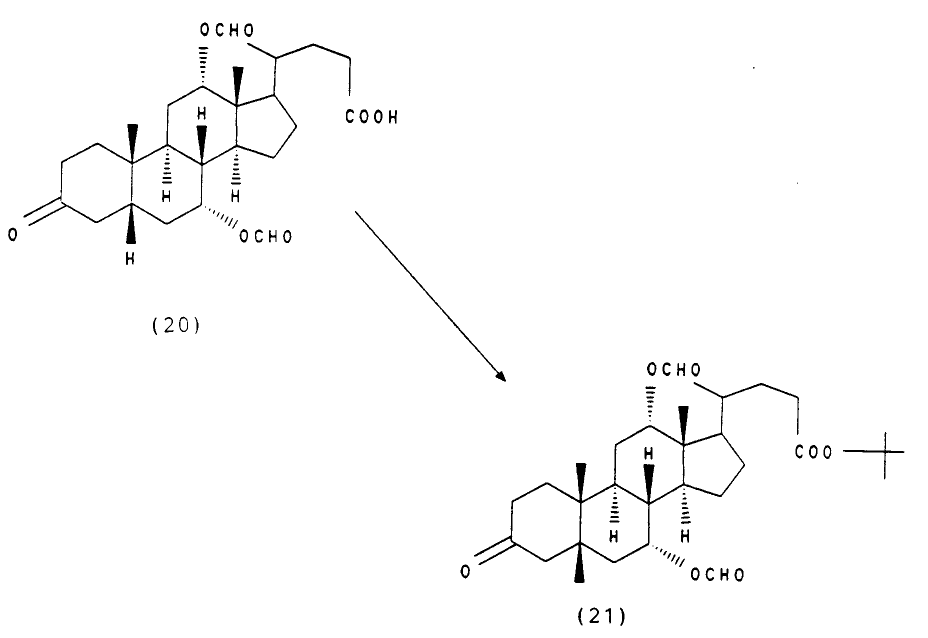

2 g (4,3 mmol) (20) in 20 ml trockenem Toluol werden mit 4 ml Oxalylchlorid versetzt und 4 Stunden bei 80°C gerührt. Danach wird das überschüssige Oxalylchlorid durch Destillation mit Toluol (zweimalige Zugabe) entfernt und der Rückstand in 20 ml trockenem CH₂Cl₂ aufgenommen. Die Lösung gibt man bei 0 °C zu einer Mischung aus 10 ml CH₂Cl₂, 10 ml tert.-Butanol und 0,4 ml Pyridin. Nach 2 Stunden bei Raumtemperatur wird mit Wasser versetzt, abgetrennt, extrahiert, getrocknet, eingeengt und der Rückstand durch Chromatographie gereinigt (SiO₂, CH₂Cl₂/Aceton = 10 : 1).
Ausbeute: 1,43 g (64 %) I (21).
MS (FAB, 3-NBA, LiCl): 525 (M+Li⁺).

### Beispiel 16

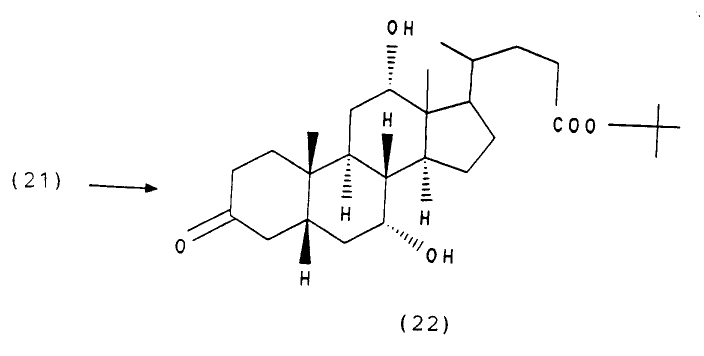

15 g (32.3 mmol) I (21) und 20 g Aluminium-tert.-butylat werden unter Argon in einer Mischung aus 450 ml trockenem Toluol und 190 ml trockenem Aceton 28 Stunden zum Rückfluß erhitzt. Nach Abkühlung wird unter Kühlung auf 300 ml 2N H₂SO₄ gegossen und dreimal mit Ether extrahiert. Die organische Phase wird zweimal mit 2N H₂SO₄, zweimal mit Wasser, zweimal mit ges. NaHCO₃-Lösung und nochmals mit Wasser gewaschen, getrocknet und eingeengt. Säulenchromatographie (SiO₂, CH₂Cl₂/Aceton = 4 : 1) ergibt 9.7 g (65 %) (22).
MS (FAB, 3-NBA, LiCl): 469 (M+Li⁺).

### Beispiel 17

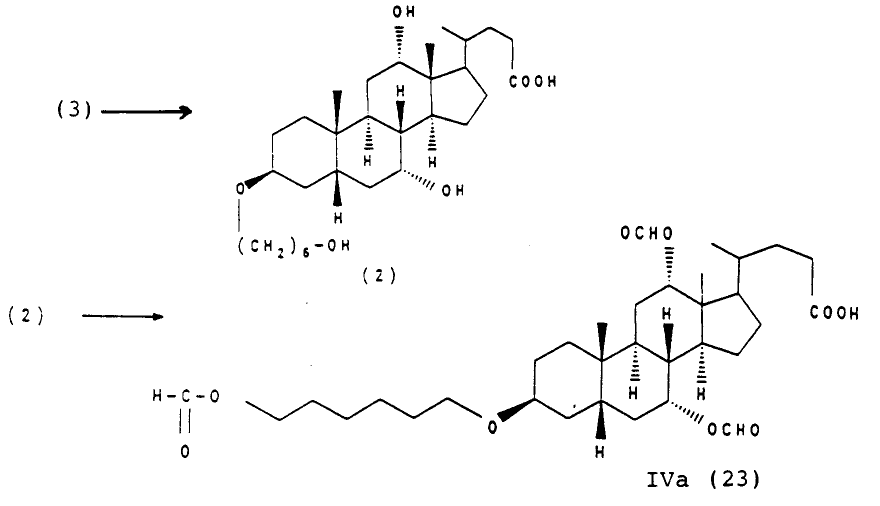

Aus (3) wird nach dem in Beispiel 26 beschriebenen Verfahren Verbindung (2) hergestellt.

5,1 g (0.01 mol) (2) werden in 25 ml 90 proz. Ameisensäure und 8 Tropfen 60 proz. Perchlorsäure 1,5 Stunden auf 55 - 60 °C erwärmt. Nach Abkühlung auf 40 °C gibt man tropfenweise 15 ml Acetanhydrid hinzu, bis eine deutliche Gasentwicklung eintritt (Temp.-Anstieg 40 bis 50 °C). Man kühlt auf Raumtemperatur, gießt die Lösung unter kräftigem Rühren in 200 ml Wasser und extrahiert das Produkt mit Dichlormethan.
Nach Trocknung, Eindampfen und Umkristallisieren des Rückstands aus CH₃OH/H₂O erhält man 5,44 g (92 %) IVa (23)
MS (FAB, 3-NBA, LiCl): 599 (M+Li⁺).

### Beispiel 18

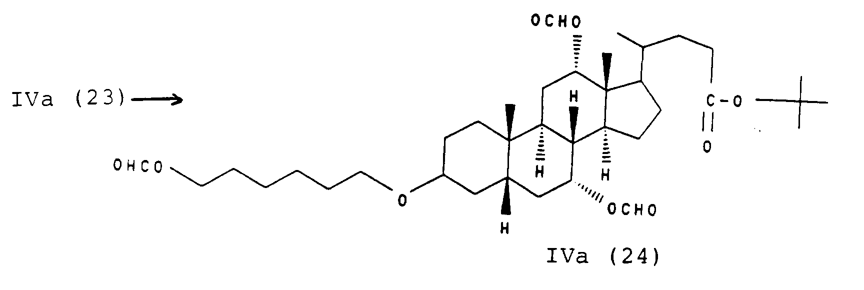

8 ml frisch über Chinolin und Leinöl destilliertes Thionylchlorid wird zu 4 g (6.75 mmol) IVa (23) in 80 ml trockenem Toluol gegeben und unter Argon 4 Stunden auf 80 °C erwärmt. Danach wird das überschüssige SOCl₂ mit Toluol abdestilliert und durch zweimalige Zugabe von Toluol und erneute Destillation weitestgehend entfernt. Der Rückstand wird mit 50 ml trockenem CH₂Cl₂ aufgenommen und bei 0°C zu einer Lösung von 20 ml tert.-Butanol in 40 ml CH₂Cl₂ und 0,8 ml Pyridin gegeben. Nach 1 Stunde Rühren bei Raumtemperatur und Stehen über Nacht versetzt man mit Wasser, extrahiert nach Abtrennung der organischen Phase mehrfach mit CH₂Cl₂, trocknet und engt ein. Das Rohprodukt wird durch Chromatographie gereinigt (SiO2, n-Heptan/Essigester = 3/1).
Ausbeute: 3.8 g (87 %) IVa (24).
MS (FAB, 3-NBA, LiCl): 655 (M+Li⁺).

### Beispiel 19

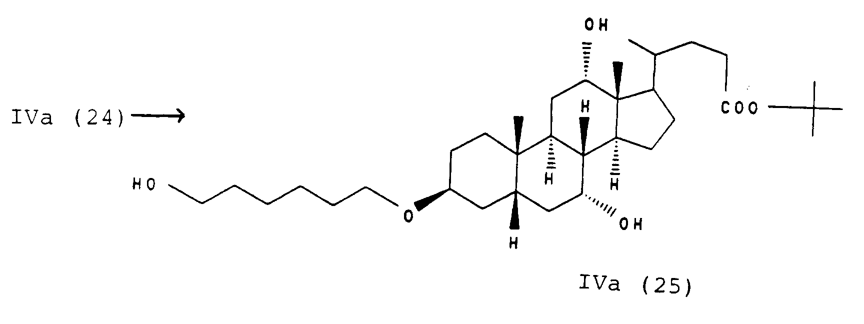

3,5 g (5.4 mmol) IVa (24) werden in 50 ml Dioxan und 20 ml 2N NaOH gelöst und 10 min. auf dem Dampfbad erhitzt. Man verdünnt mit 100 ml CH₂Cl₂, trocknet grob mit MgSO₄, filtriert, engt ein und reinigt den Rückstand durch Säulenfiltration. Man erhält 3,05 g (93 %) IVa (25).
MS (FAB, 3-NBA, LiCl): 571 (M+Li⁺).

### Beispiel 20

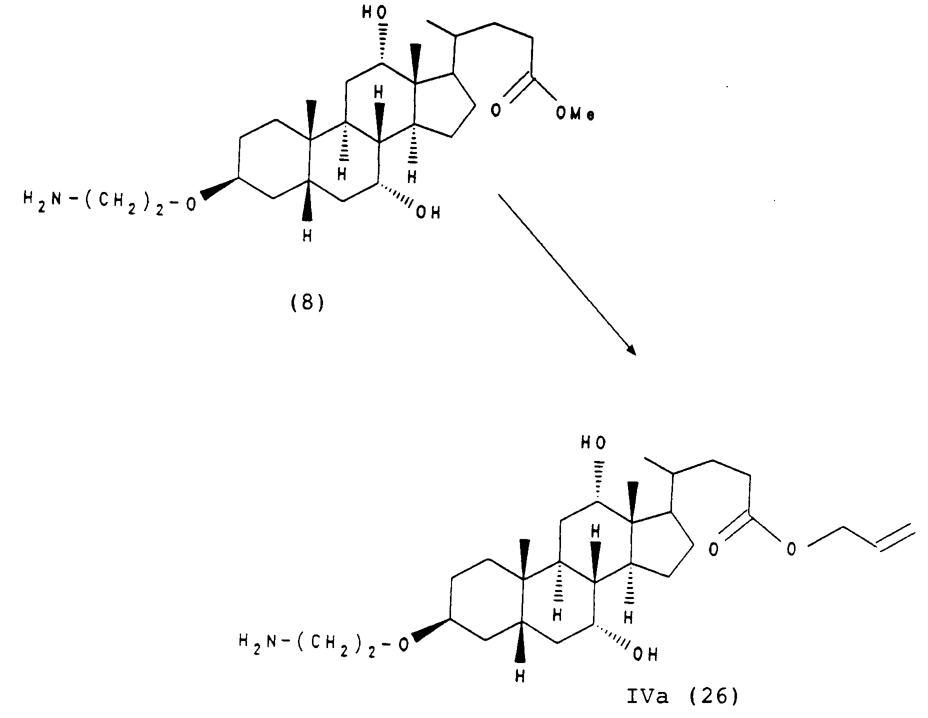

3,0 g (6.44 mmol) Methylester (8) (EP-A-0 417 725) und 0,43 g (1.88 mmol) Tetraethoxytitan werden in 50 ml trockenem Allylalkohol 20 Stunden bei 100 °C gerührt. Das Reaktionsgemisch wird im Vakuum eingeengt. Nach Chromatographie des Rückstandes auf Kieselgel (Essigester/Methanol/Triethylamin 5 : 1 : 1) werden 1,8 g (3.66 mmol, 57 %) Allylester IVa (26) erhalten.
C₂₉H₄₉NO₅ (491), MS (FAB, 3-NBA, LiCl): 498 (M+Li⁺).

### Beispiel 21

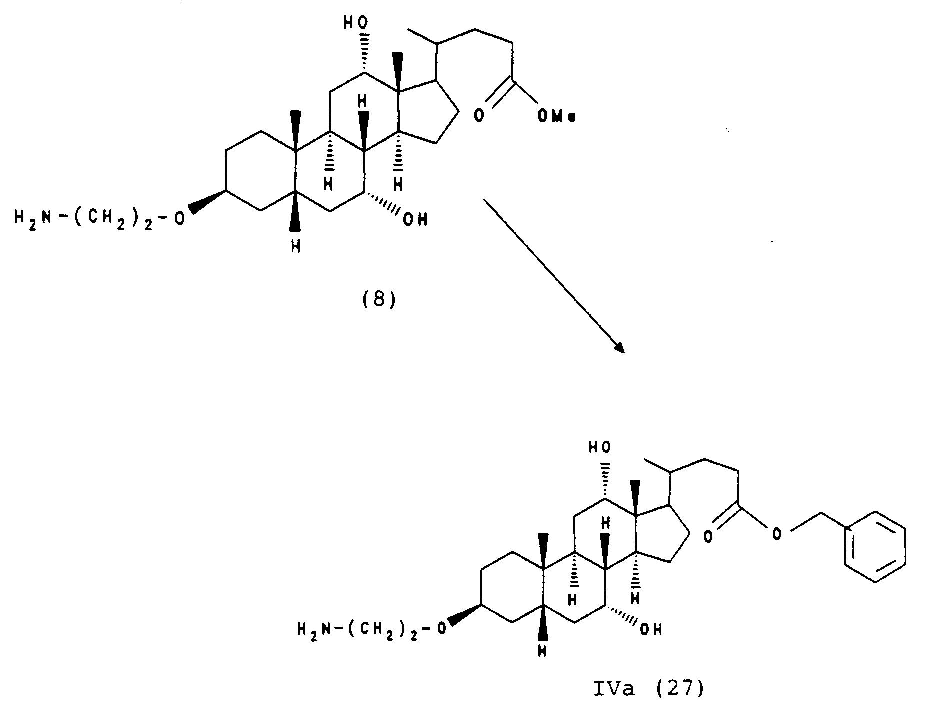

15,0 g (32.21 mmol) Methylester (8) und 2,28 g (10 mmol) Tetraethoxytitan werden in 300 ml trockenem Benzylalkohol 8 Stunden bei 100 °C gerührt. Das Reaktionsgemisch wird im Vakuum eingeengt. Nach Chromatographie des Rückstands auf Kieselgel (Essigester/Methanol/Triethylamin 5 : 1 : 1) werden 10,0 g (18,46 mmol, 57 %) Benzylester IVa (27) erhalten.
C₃₃H₅₁NO₅ (541), MS (FAB, 3-NBA, LiCl): 548 (M+Li⁺).

### Beispiel 22

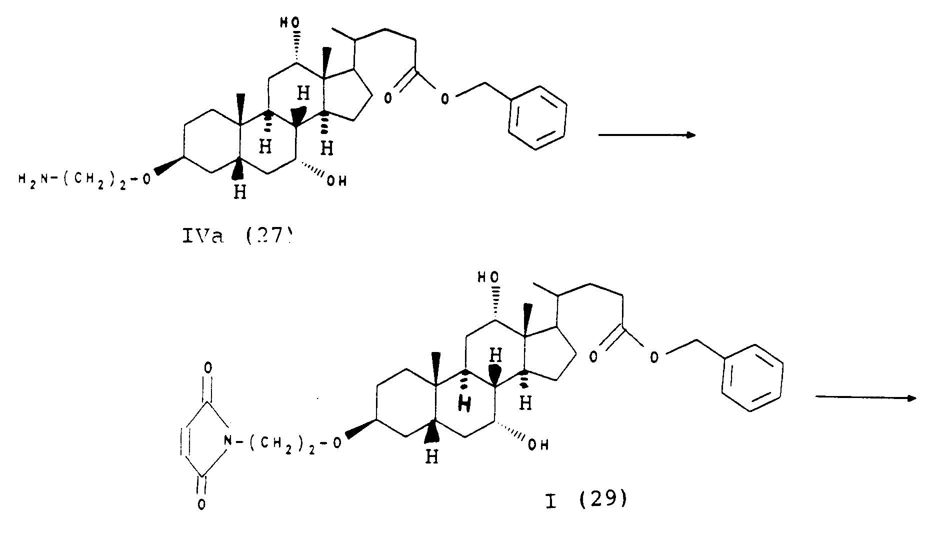

540 mg (1.00 mmol) IVa (27) und 147 mg (1.50 mmol) Maleinsäureanhydrid werden in 10 ml Essigsäure gelöst und 4 Stunden unter Rückfluß erhitzt. Nach dem Abkühlen auf Zimmertemperatur wird das Lösungsmittel im Vakuum abgezogen. Chromatographie des Rohprodukts auf Kieselgel (Essigester/Methanol/Triethylamin 15 : 4 : 1) ergibt 490 mg (0.79 mmol, 79 %) I (29).
C₃₇H₅₁NO₇ (621), MS (FAB, 3-NBA, LiCl): 628 (M+Li⁺).

### Beispiel 23

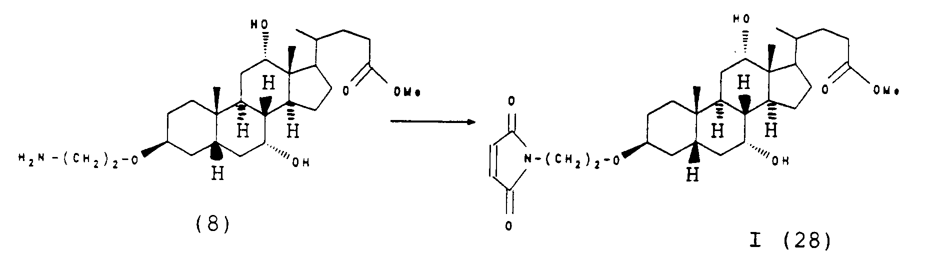

460 mg (1.00 mmol) (8) und 100 mg (1.00 mmol) Maleinsäureanhydrid werden in 10 ml Essigsäure gelöst und 4 Stunden unter Rückfluß erhitzt. Nach dem Abkühlen auf Zimmertemperatur wird das Lösungsmittel im Vakuum abgezogen. Chromatographie auf Kieselgel ergibt 330 mg (0.60 mmol, 61 %) I (28).
C₃₁H₄₇NO₇ (545), MS (FAB, 3-NBA, LiCl): 552 (M+Li⁺).

### Beispiel 24

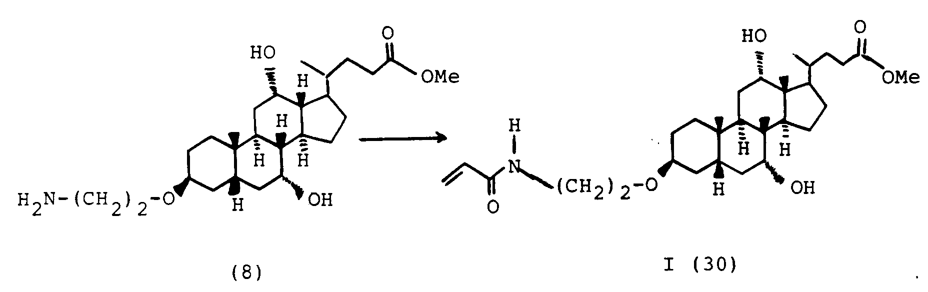

Zu einer Lösung von 5,0 g (10.74 mmol) (8) und 1,8 ml (12.9 mmol) Triethylamin in 80 ml Tetrahydrofuran wird bei -30 °C langsam eine Lösung von 0,94 ml (11.57 mmol) Acrylsäurechlorid in 20 ml THF zugetropft. Nach 30 min. bei -30 °C wird auf Wasser gegossen und 3 x mit Essigester extrahiert. Die vereinigten organischen Phasen werden über Natriumsulfat getrocknet und im Vakuum eingeengt. Der Rückstand wird über Kieselgel chromatographiert (Essigester) und man erhält 3,2 g (6.16 mmol, 57 %) I (30).
C₃₀H₄₉NO₆ (519), MS (FAB, 3-NBA, LiCl): 526 (M+Li⁺).

### Beispiel 25

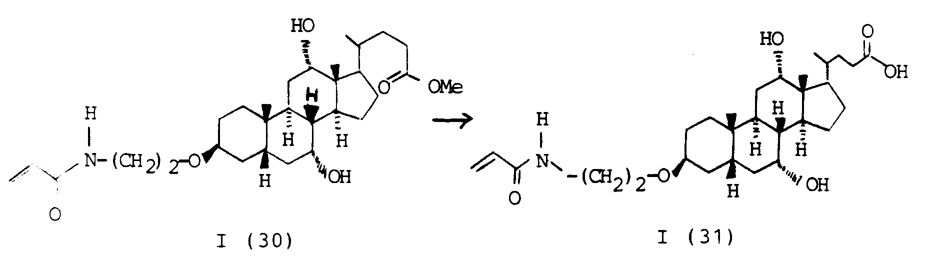

500 mg (0.96 mmol) I (30) werden in 50 ml Ethanol gelöst, mit 5 ml 1N NaOH versetzt und 3 Stunden bei Zimmertemperatur gerührt. Es werden 50 ml Wasser zugesetzt, der Alkohol im Vakuum abgedampft, mit HCl angesäuert und 3x mit Essigester extrahiert. Die vereinigten organischen Phasen werden über Natriumsulfat getrocknet und eingedampft. Chromatographie über Kieselgel (Chloroform/Methanol 9:1) ergibt 280 mg (0.55 mmol, 58 %) I (31).
C₂₉H₄₇NO₆ (505), MS (FAB, 3-NBA, LiCl): 512 (M+Li⁺).

### Beispiel 26

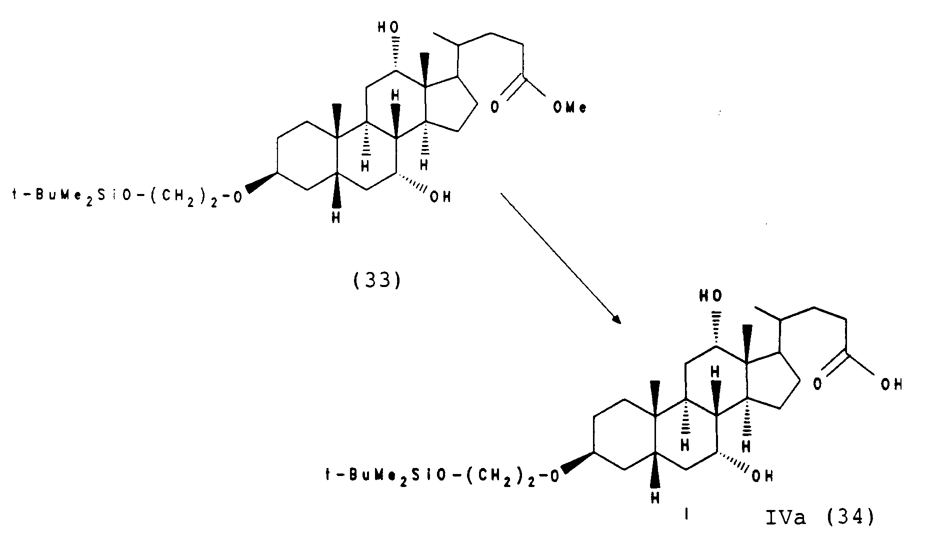

21,2 g (36.5 mmol) (33) (EP-A-0 417 725) werden in Methanol (500 ml) gelöst und unter Rückfluß erhitzt. Innerhalb von 24 Stunden werden 43,7 ml 1N NaOH zugetropft und anschließend weitere 6 Stunden unter Rückfluß erhitzt. Danach wird das Lösungsmittel weitgehend abgezogen, der Rückstand in 400 ml Waser aufgenommen, mit 44,0 ml 1N HCl versetzt und 3 x mit Ether extrahiert. Die vereinigten organischen Phasen werden getrocknet (MgSO₄) und eingeengt. Chromatographie auf Kieselgel ergibt 19,9 g (28 mmol, 77 %) IVa (34).
C₃₂H₅₈O₆Si (566), MS (FAB, 3-NBA, LiCl): 573 (M+Li⁺).
Schmelzpunkt: 188 - 190 °C.

### Beispiel 27

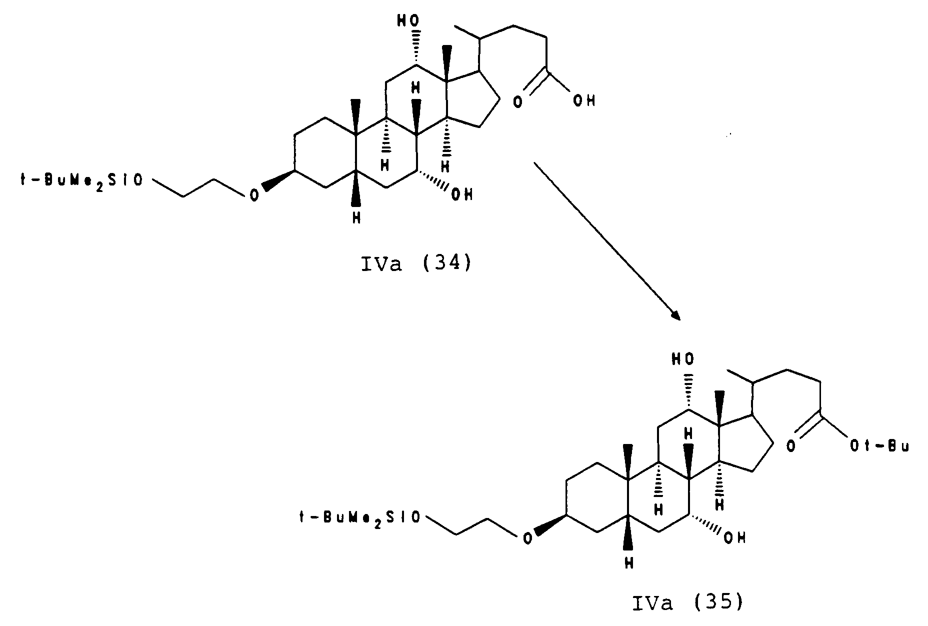

15,9 g (28.0 mmol) IVa (34) und 3,68 g (36.5 mmol) Triethylamin werden in 300 ml Tetrahydrofuran gelöst und auf 0 °C gekühlt. Nach Zugabe von 7.03 g (33.6 mmol) 2,6-Dichlorbenzoylchlorid wird das Eisbad entfernt und 4 Stunden unter Rückfluß erhitzt. Anschließend werden bei Raumtemperatur 31,2 g (0.42 mol) t-Butanol und 3,42 g (28 mmol) Dimethylaminopyridin zugegeben. Es wird weitere 4 Stunden unter Rückfluß erhitzt. Das Lösungsmittel wird weitgehend abdestilliert, der Rückstand in 300 ml Essigester aufgenommen, 3 x mit Wasser gewaschen, über Magnesiumsulfat getrocknet. Nach Abziehen des Lösungsmittels wird über Kieselgel chromatographiert (Cyclohexan/Essigester 7 : 3) und man erhält 8,9 g (14.3 mmol, 51 %) IVa (35).
C₃₆H₆₆O₆Si (622), MS (FAB, 3-NBA, LiCl): 629 (M+Li⁺).

### Beispiel 28

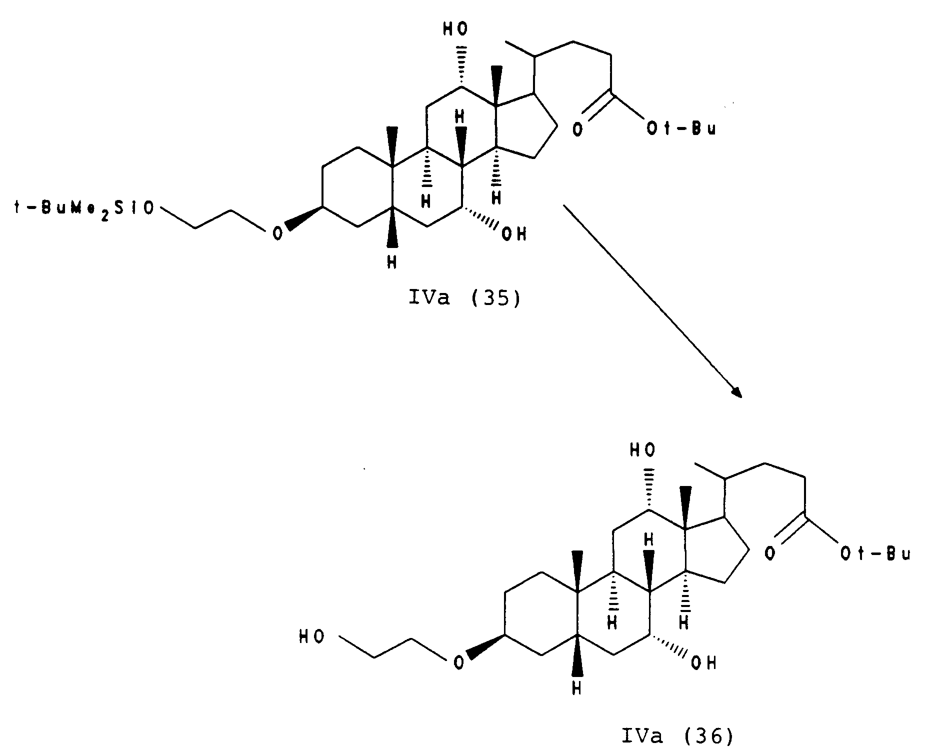

8,35 g (13.4 mmol) Verbindung IVa (33) werden in 150 ml Tetrahydrofuran gelöst, mit 3,21 g (53 mmol) Essigsäure und 12,94 g (40 mmol) Tetrabutylammoniumfluorid Trihydrat versetzt und 20 Stunden bei Raumtemperatur gerührt. Das Lösungsmittel wird abdestilliert, der Rückstand in 200 ml Essigester aufgenommen, 4 x mit Wasser gewaschen, über Magnesiumsulfat getrocknet. Das nach dem Eindampfen erhaltene Rohprodukt wird über Kieselgel chromatographiert (Essigester/Cyclohexan 9 : 1). Man erhält 5,5 g (10.8 mmol, 81 %) IVa (36).
C₃₀H₅₂O₆ (508), MS (FAB, 3-NBA, LiCl): 515 (M+Li⁺).
Schmelzpunkt 127 - 129 °C.

### Beispiel 29

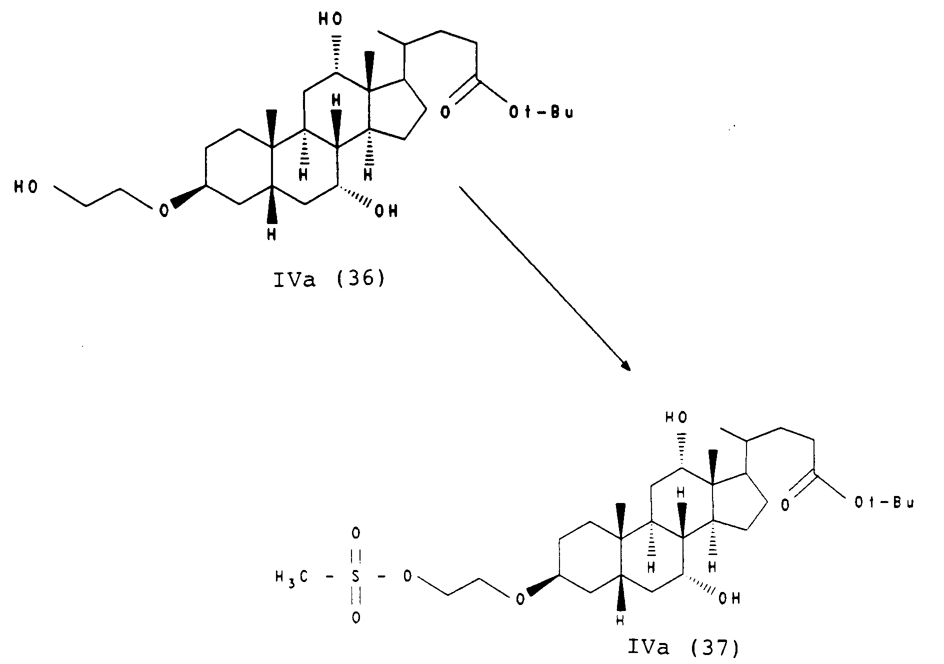

Zu 8,3 g (16.3 mmol) IVa (36) in 50 ml Pyridin werden bei 0 °C 2,1 g (18.3 mmol) Methansulfonsäurechlorid getropft. Man läßt 15 Minuten bei 0 °C und 1 Stunde bei Raumtemperatur rühren. Das Reaktionsgemisch wird auf 100 ml Wasser gegossen und 3 x mit Essigester extrahiert. Trocknen der vereinigten organischen Phasen (MgSO₄), Abziehen des Lösungsmittels und Chromatographie über Kieselgel (Essigester/Cyclohexan 3 : 1) ergibt 8,6 g (14.7 mmol, 90 %) IVa (37).
C₃₁H₅₄O₈S (586), MS (FAB, 3-NBA, LiJ): 593 (M+Li⁺).

### Beispiel 30

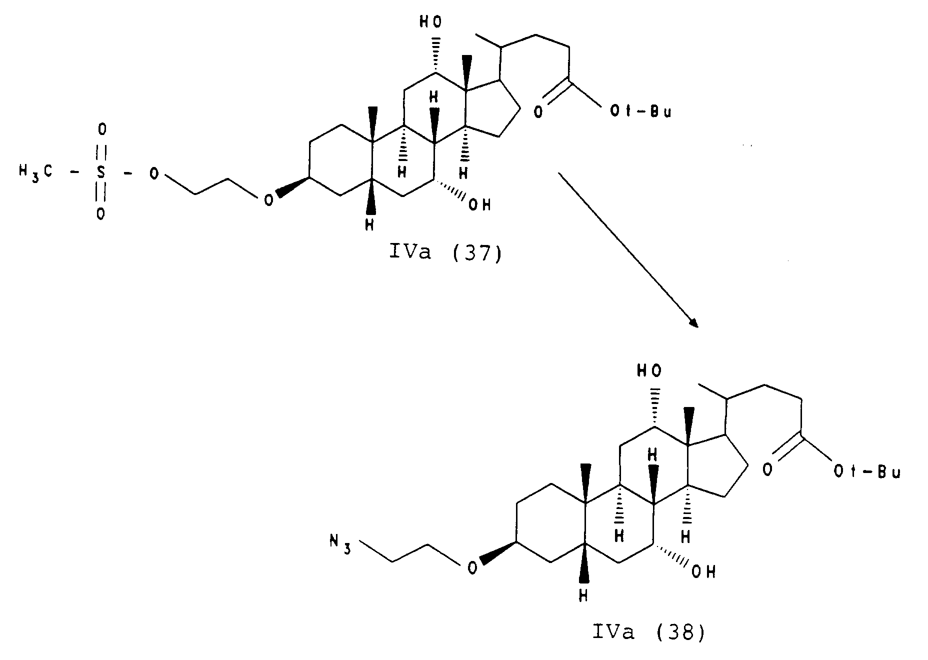

8,4 g (14.3 mmol) IVa (37) werden mit 1,0 g (15.4 mmol) Natriumazid in 100 ml trockenem DMSO 2 Stunden bei 70 °C gerührt. Das Reaktionsgemisch wird auf Wasser gegossen und 3 x mit Essigester extrahiert. Die vereinigten organischen Phasen werden getrocknet (MgSO₄) und eingedampft. Der Rückstand wird in Toluol aufgenommen und wieder eingedampft (2 x). Ausbeute 7,6 g (quant.) IVa (38). Das Azid wird ohne weitere Reinigung zur nächsten Stufe eingesetzt.

### Beispiel 31

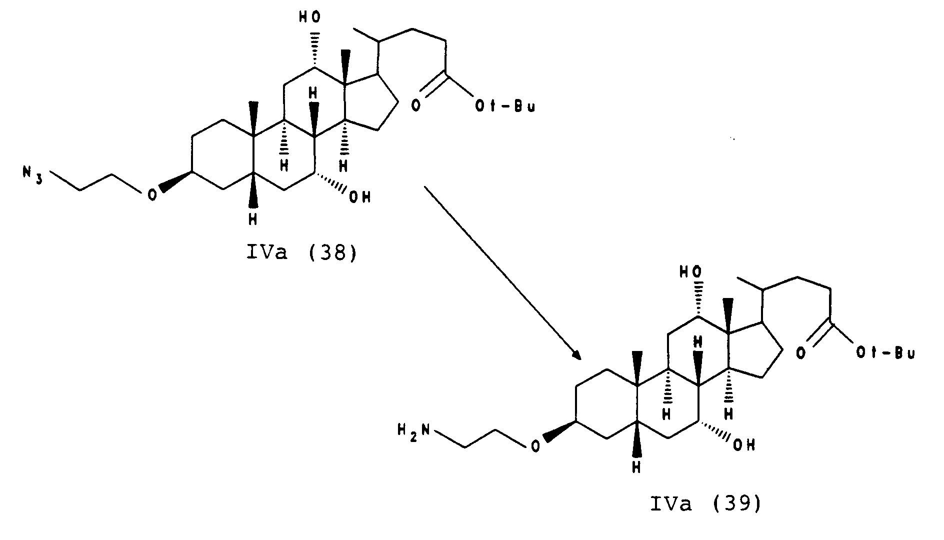

7,6 g (14.2 mmol) IVa (38) werden in 200 ml Essigester in Gegenwart von 5 g Pd/C (10 %) bei Raumtemperatur unter Normaldruck hydriert. Der Katalysator wird abfiltriert und das Filtrat eingedampft. Chromatographie auf Kieselgel (Essigester/Methanol/Triethylamin 5 : 1 : 1) ergibt 5,0 g (9.85 mmol, 69 %) IVa (39).
C₃₀H₅₃NO₃ (507), MS (FAB, 3-NBA, LiJ): 514 (M+Li⁺)..

### Beispiel 32

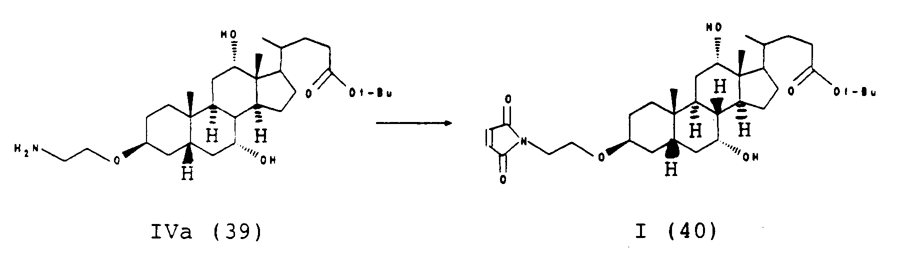

2,0 g (3.94 mmol) IVa (39) und 570 mg (5.9 mmol) Maleinsäureanhydrid werden in 10 ml Essigsäure gelöst und 2 Stunden unter Rückfluß erhitzt. Nach Abziehen des Lösungsmittels wird über Kieselgel chromatographiert (Essigester/Methanol/ Triethylamin 10 : 2 : 1). Man erhält 1,0 g (1.7 mmol, 43 %) I (40).

### Beispiel 33

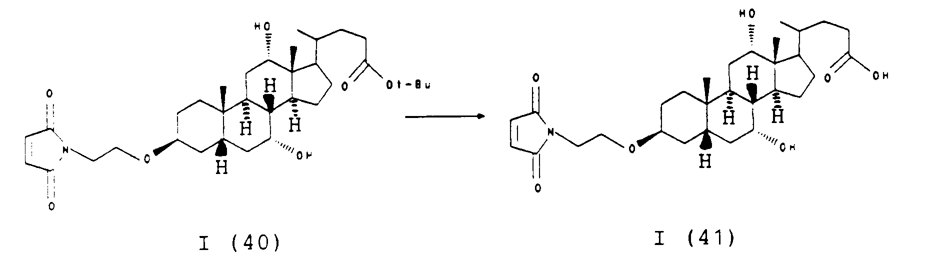

850 mg (1.45 mmol) I (40) werden in 20 ml Dichlormethan gelöst. Bei 0 °C werden langsam 20 ml Trifluoressigsäure zugetropft. Nach 1,5 Stunden bei 0 °C wird noch 1 Stunde bei Zimmertemperatur gerührt. Anschließend wird im Vakuum eingeengt, 20 ml Toluol zugegeben und nochmals eingeengt. Chromatographie über Kieselgel (Chloroform/Methanol 92:8) ergibt 380 mg (0.72 mmol, 49 %) I (41)
C₃₀H₄₅NO₇ (531), MS (FAB, 3-NBA, LiCl): 538 (M+Li⁺).

### Beispiel 34

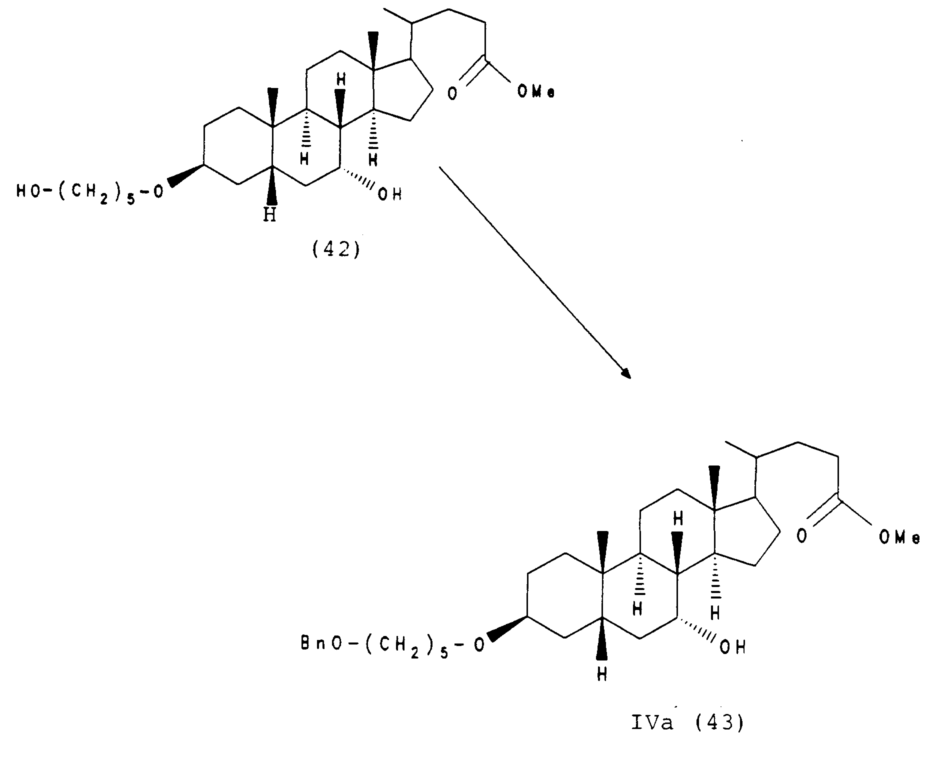

50 g (101.5 mmol) (42) (EP-A-0 417 725) und 60 g (350 mmol) Benzylbromid werden in 300 ml N-Ethyldiisopropylamin 4 Stunden bei 100°C gerührt. Nach dem Abkühlen wird das Reaktionsgemisch auf 21 2M Schwefelsäure gegossen und 3 x mit Essigester extrahiert. Die vereinigten organischen Phasen werden mit wäßriger NaHCO₃-Lösung ausgeschüttelt, über MgSO₄ getrocknet und im Vakuum eingedampft. Chromatographie über Kieselgel (Cyclohexan/Essigester 6 : 4) ergibt 18,3 g (31.5 mmol, 31 %) IVa (43).
C₃₇H₅₈O₅ (582), MS (FAB, 3-NBA, LiCl): 589 (M+Li⁺).

### Beispiel 35

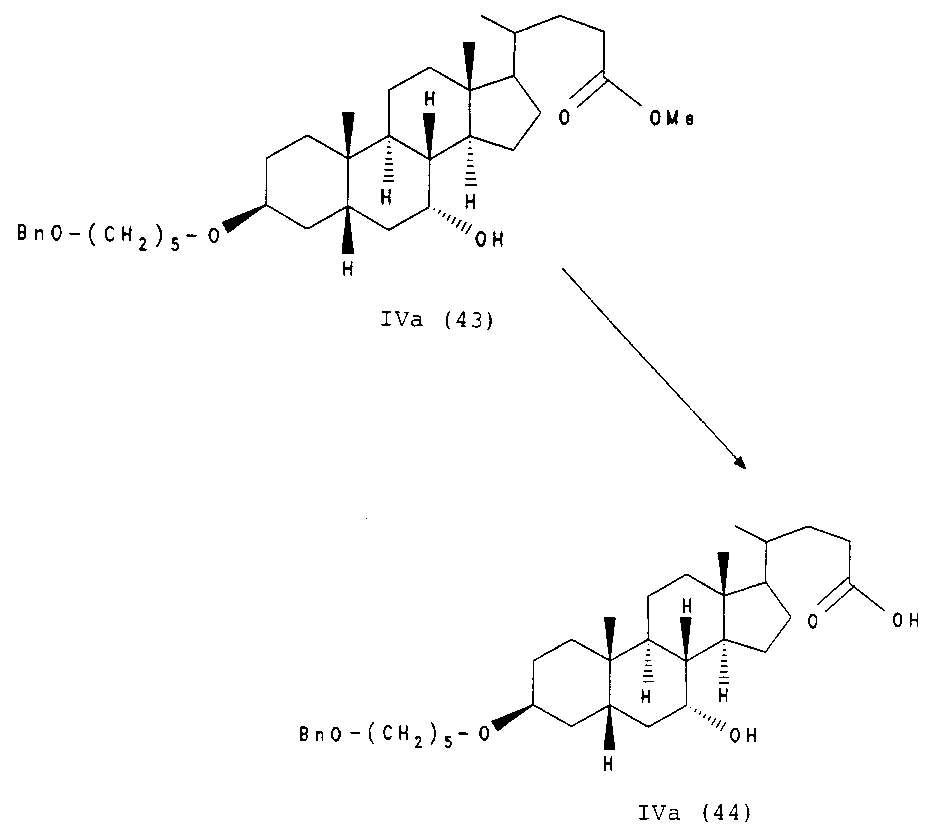

Analog zu Beispiel 26 werden aus 18,0 g (30.9 mmol) Verbindung IVa (43) 16,0 g (28.1 mmol, 91 %) IVa (44) hergestellt.
C₃₆H₅₆O₅ (568), MS (FAB, 3-NBA, LiCl): 575 (M+Li⁺).

### Beispiel 36

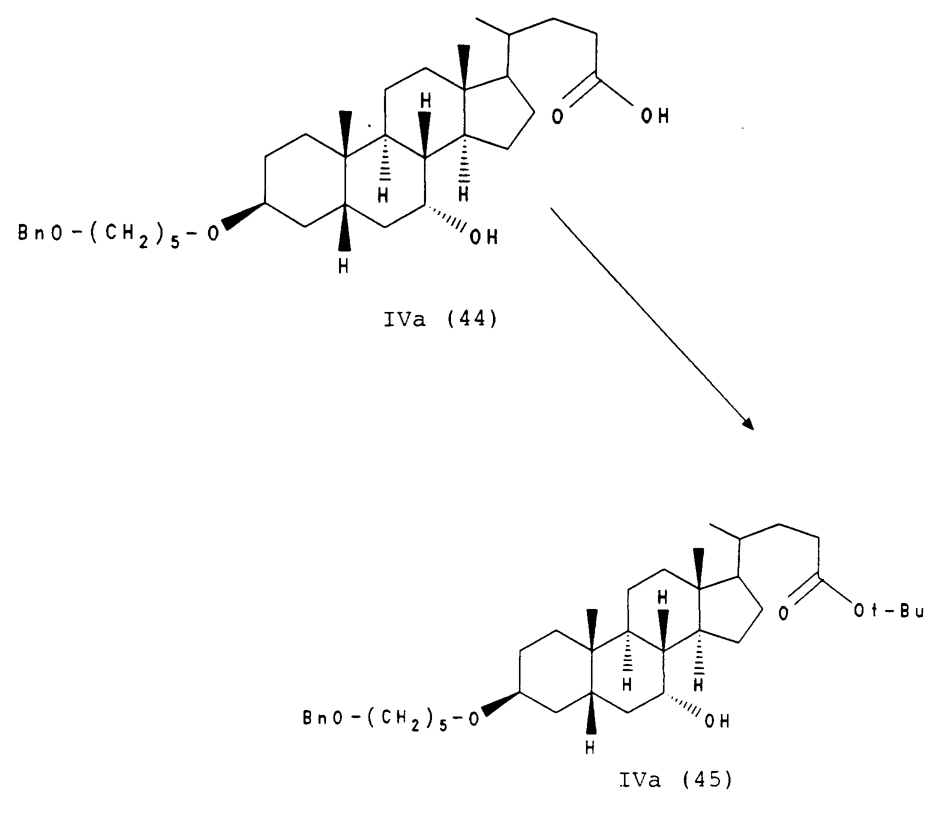

Analog zu Beispiel 27 werden aus 16,0 g (28.1 mmol) IVa (44) 8,5 g (13.6 mmol, 48 %) IVa (45) hergestellt.
C₄₀H₆₄O₅ (624), MS (FAB, 3-NBA, LiCl): 631 (M+Li⁺).

### Beispiel 37

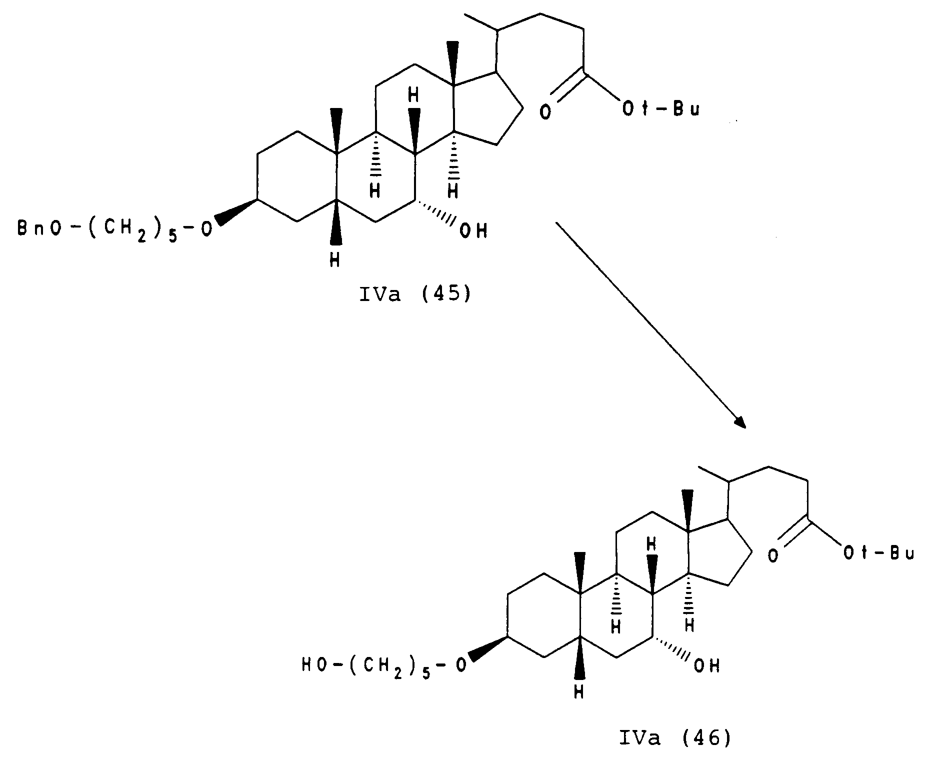

8,1 g (12.96 mmol) IVa (45) werden in Gegenwart von 1 g Pd/C (10 %) in 250 ml Essigester bei Zimmertemperatur unter Normaldruck hydriert. Nach Beendigung der Reaktion wird vom Katalysator abfiltriert und eingeengt. Man erhält 6,8 g (12.7 mmol, 98 %) IVa (46).
C₃₃H₅₈O₅ (534), MS (FAB, 3-NBA, LiCl): 541 (M+Li⁺).

### Beispiel 38

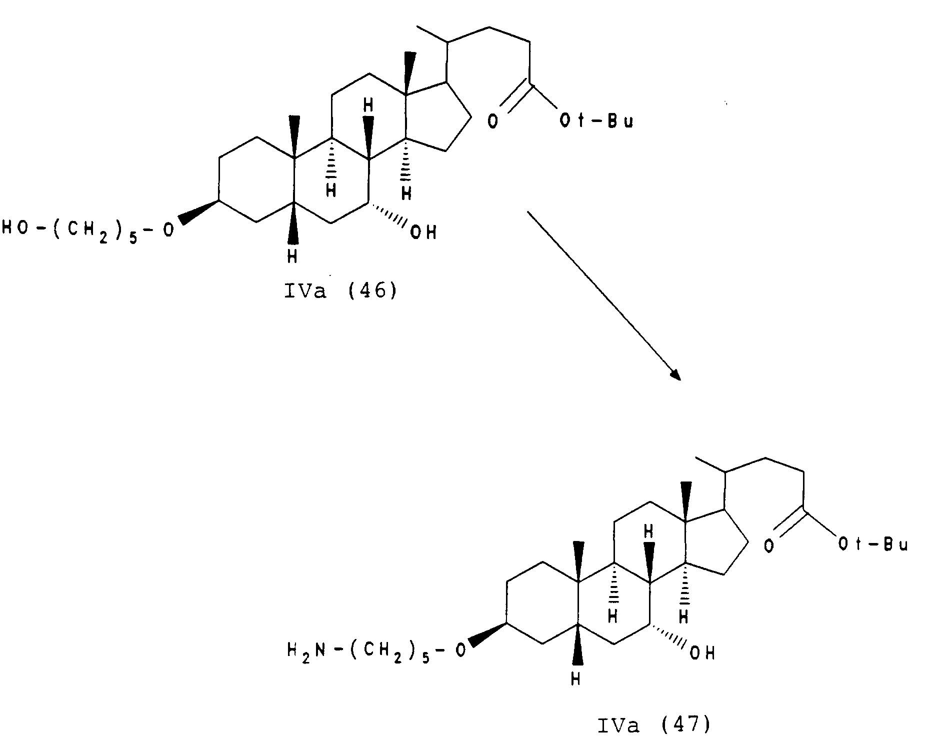

In Analogie zu den Beispielen 29 - 31 wird aus Verbindung IVa (46) die Verbindung IVa (47) hergestellt.
C₃₃H₅₉NO₄ (533), MS (FAB, 3-NBA, LiCl): 540 (M+Li⁺).

### Beispiel 39

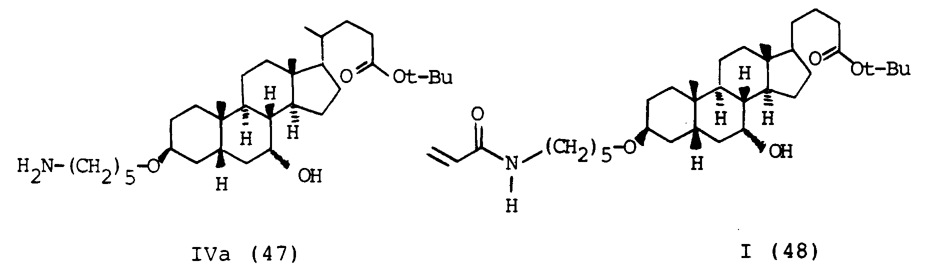

Analog Beispiel 24 werden aus 1,8 g (3.37 mmol) IVa (47) 1,2 g (2.04 mmol, 61 %) I (48) erhalten.
C₃₆H₆₁NO₅ (587), MS (FAB, 3-NBA, LiCl): 694 (M+Li⁺).

### Beispiel 40

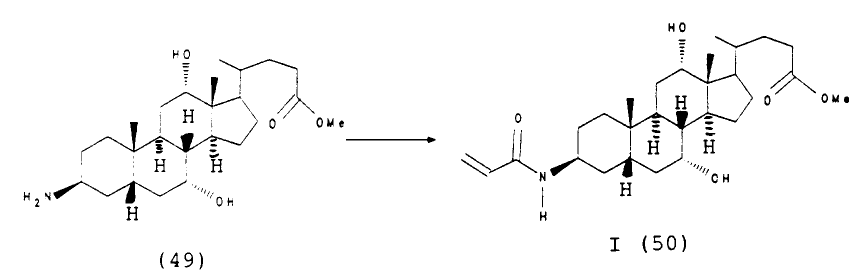

Analog zu Beispiel 24 werden aus 3,5 g (8.30 mmol) (49) (EP-A-0 417 725) 2,4 g (5.05 mmol, 61 %) I (50) hergestellt.
C₂₈H₄₅NO₅ (475), MS, (FAB, 3-NBA, LiCl): 482 (M+Li⁺).

### Beispiel 41

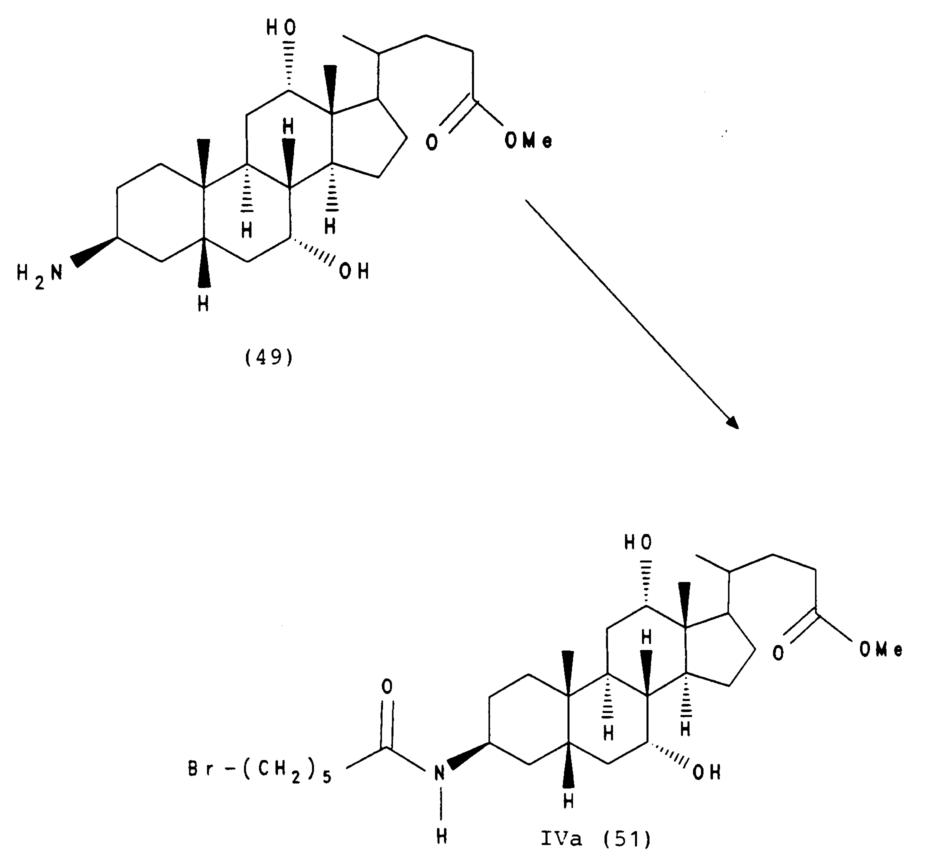

Zu einer Lösung von 2,0 g (4.74 mmol) Verbindung (49) und 0,8 ml (5.74 mmol) Triethylamin in 50 ml Dichlormethan werden bei 0 °C 0,8 ml (5.26 mmol) Bromhexansäurechlorid zugetropft. Nach 10 min. bei 0 °C wird noch 1 Stunde bei Zimmertemperatur gerührt. Zur Aufarbeitung wird auf Wasser gegossen, die organische Phase nochmals mit Wasser gewaschen, dann über Natriumsulfat getrocknet und im Vakuum eingeengt. Nach Chromatographie (Essigester/Cyclohexan 4 : 1) erhält man 1,36 g (2.27 mmol, 48 %) IVa (51).
C₃₁H₅₂BrNO₅ (597, 599), MS (FAB, 3-NBA, LiCl): 604, 606 (M+Li⁺).

### Beispiel 42

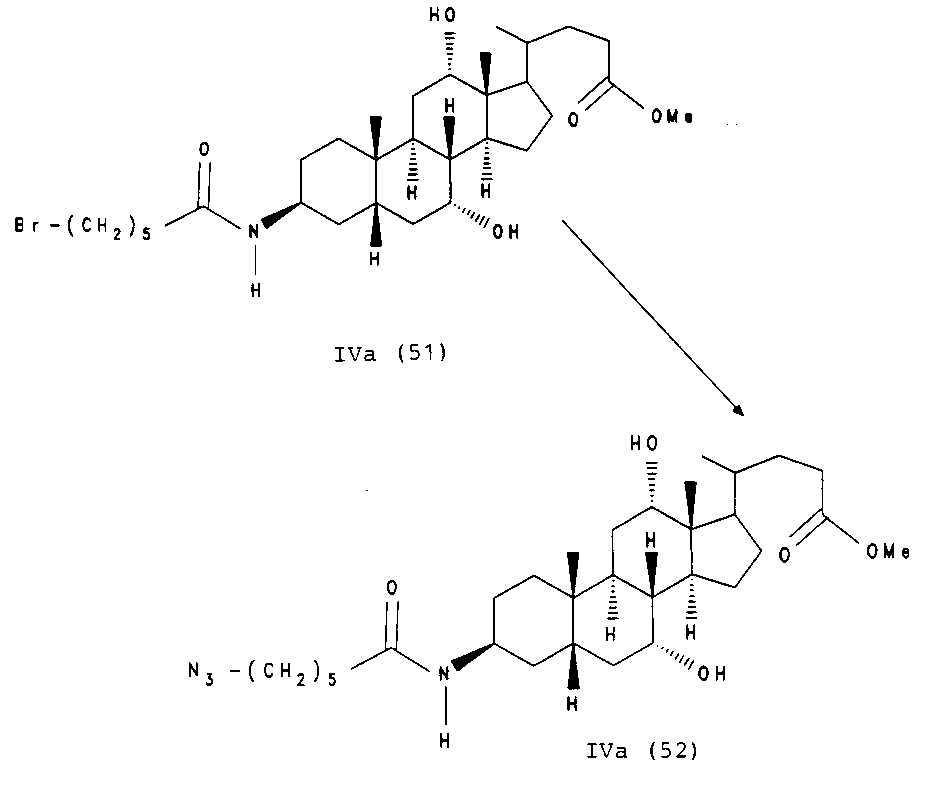

5,7g (9.52 mmol) IVa (51) und 1,0 g (15.4 mmol) NaN₃ werden in 100 ml Dimethylformamid 4 Stunden bei 70 °C gerührt. Nach dem Abkühlen wird auf Wasser gegossen und 3 x mit Ether extrahiert. Die organischen Phasen werden über Natriumsulfat getrocknet und eingeengt. Nach Chromatographie (Essigester) erhält man 4,6 g (8.20 mmol, 86 %) IVa (52).
C₃₀H₅₂N₄O₅ (560), MS (FAB, 3-NBA, LiCl): 567 (M+Li⁺).

### Beispiel 43

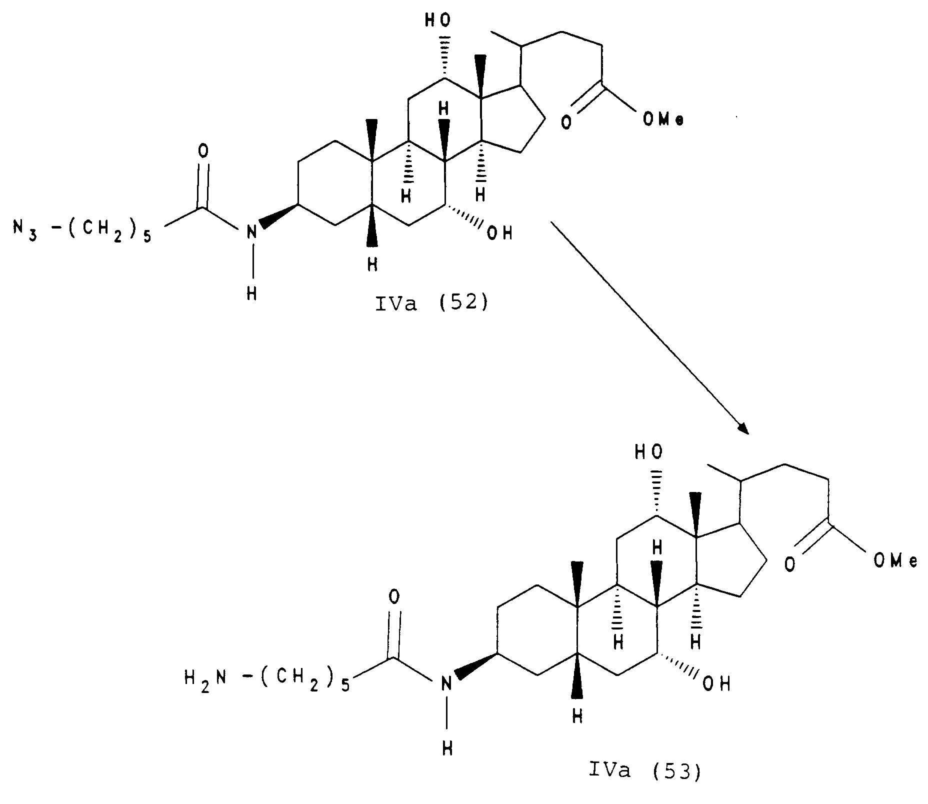

4,55 g (8.11 mmol) Verbindung IVa (52) werden in 200 ml Essigester gelöst und in Gegenwart von 500 mg Pd/C (10 %) bei Zimmertemperatur unter Normaldruck hydriert. Nach Beendigung der Reaktion wird vom Katalysator abfiltriert und eingeengt. Nach Chromatographie (Chloroform/Methanol 8 : 2) werden 2,7 g (5.05 mmol, 62 %) IVa (53) erhalten.
C₃₁H₅₄N₂O₅ (534), MS (FAB, 3-NBA, LiCl): 541 (M+Li⁺).

### Beispiel 44

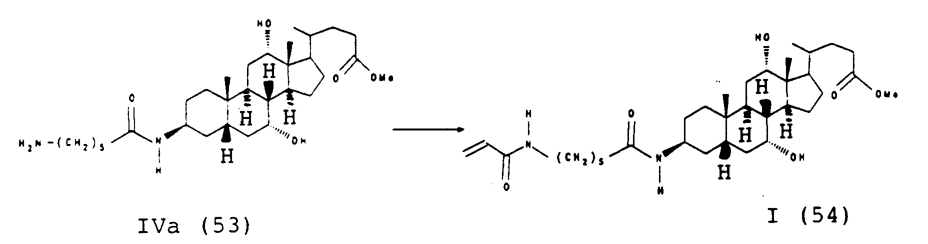

Analog zu Beispiel 24 werden aus 2,6 g (4.86 mmol) IVa (53) 1,4 g (2.38 mmol, 49 %) I (54) hergestellt.
C₃₄H₅₆N₂O₆ (588), MS (FAB, 3-NBA, LiCl): 595 (M+Li⁺).

### Beispiel 45

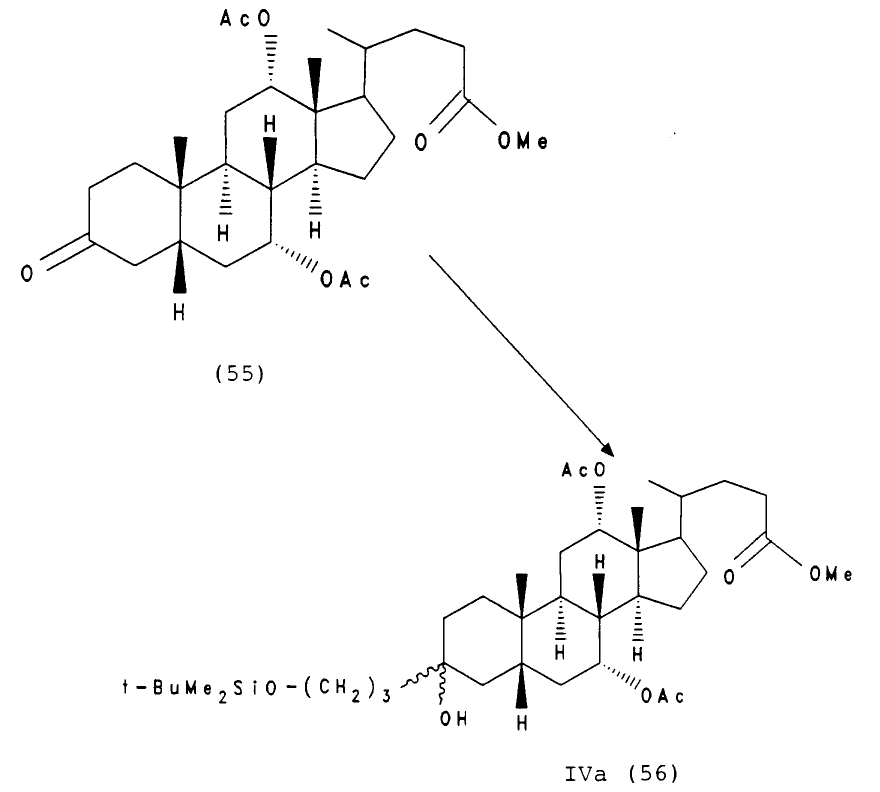

Zu einer Lösung von 8,0 g (15.85 mmol) Verbindung (55) (Helv. chim. Acta 28, 344, 1945) in 100 ml THF tropft man bei Zimmertemperatur eine aus 9,4 g (37.1 mmol) 1-Brom-3-t-butyldimethylsilyloxypropan und 1,0 g (42 mmol) Magnesium hergestellte Grignardlösung. Anschließend wird 2 Stunden unter Rückfluß erhitzt. Zur Aufarbeitung wird auf wäßrige NH₄Cl-Lösung (10 %) gegossen und 3 x mit Essigester extrahiert. Die vereinigten organischen Phasen werden über MgSO₄ getrocknet und im Vakuum eingedampft. Durch Chromatographie des Rohprodukts auf Kieselgel (Cyclohexan/Essigester 4 : 1, dann 2 : 1) erhält man als erste Fraktion 3,6 g (5.30 mmol, 33 %) IVa (56) als Hauptprodukt und als zweite Fraktion

1,2 g (1.77 mmol, 11 %) Nebenprodukt.

Hauptprodukt: C₃₈H₆₆O₈Si (678), MS (FAB, 3-NBA, LiCl): 685 (M+Li⁺). Nebenprodukt: C₃₈H₆₆O₈Si (678), MS (FAB, 3-NBA, LiCl): 685 (M+Li⁺).

### Beispiel 46

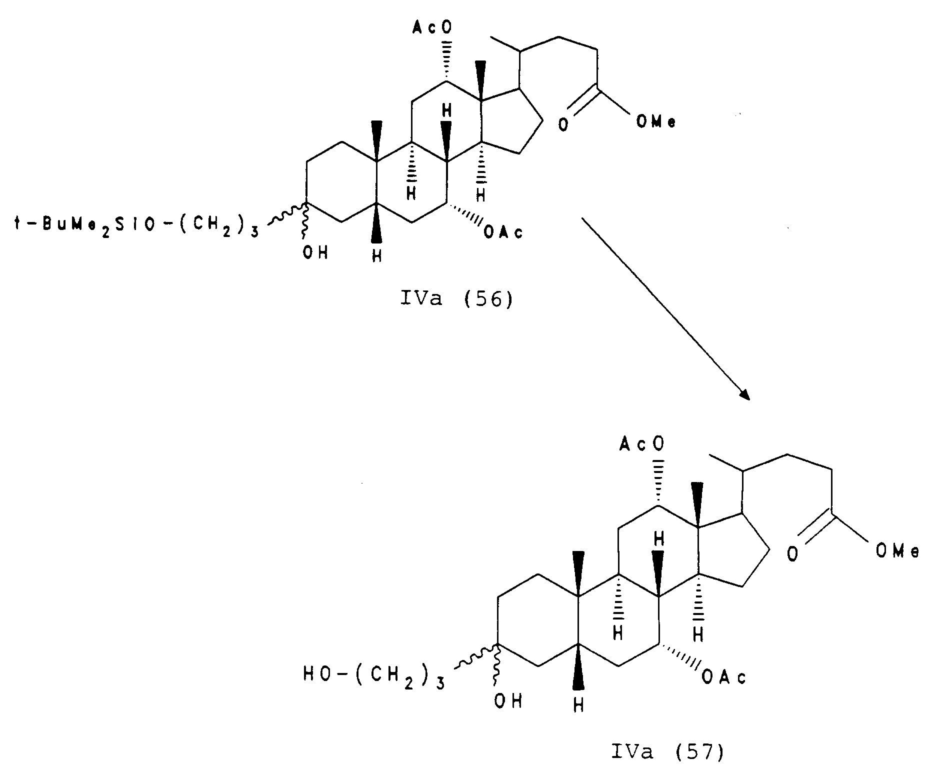

3,4 g (5.01 mmol) Verbindung IVa (56) werden mit 1,75 g (5.55 mmol) Tetrabutylammoniumfluorid-Trihydrat in 100 ml Tetrahydrofuran 1 Stunde bei Zimmertemperatur gerührt. Zur Aufarbeitung wird auf Wasser gegossen und 3 x mit Essigester extrahiert. Nach dem Trocknen der organischen Phasen über MgSO₄ wird im Vakuum eingedampft. Chromatographie des Rückstandes über Kieselgel (Cyclohexan/Essigester 1 : 1) ergibt 2,1 g (3.72 mmol, 74 %) IVa (57)
C₃₂H₅₂O₈ (564), MS (FAB, 3-NBA, LiCl): 571 (M+Li⁺).

### Beispiel 47

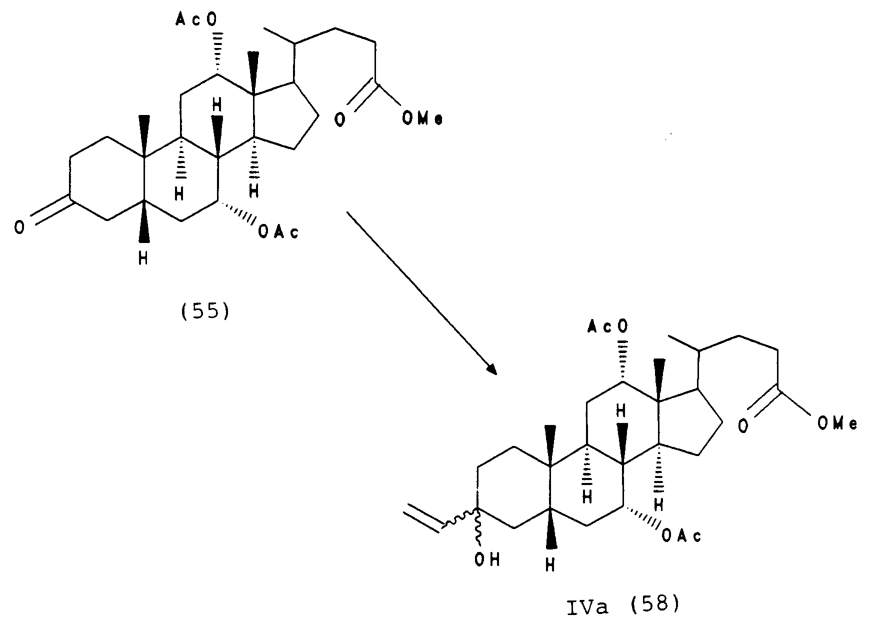

Zu einer Lösung von 10,0 g (19.81 mmol) Verbindung (55) in 300 ml Tetrahydrofuran tropft man bei -70 °C 40 ml einer 1M Lösung von Vinylmagnesiumbromid in Tetrahydrofuran. Es wird noch 1 Stunde bei -70 °C gerührt. Nach Beendigung der Reaktion werden 50 ml NH₄Cl-Lösung (10 %) zugegeben und auf Zimmertemperatur erwärmt. Es wurde noch Wasser zugegeben und 3 x mit Essigester extrahiert. Nach Trocknung der vereinigten organischen Phasen über MgSO₄ wird im Vakuum eingedampft. Das erhaltene Diastereomerengemisch wird über Kieselgel (Cyclohexan/Essigester 2 : 1) chromatographiert. Man erhält das Hauptprodukt IVa (58) als erste Fraktion, 5,7 g (10.70 mmol, 54 %) und das Nebenprodukt als zweite Fraktion, 6,3 g (2.44 mmol, 12 %).
Hauptprodukt: C₃₁H₄₈O₇ (532), MS (FAB, 3-NBA, LiCl):539 (M+Li⁺)
Nebenprodukt: C₃₁H₄₈O₇ (532), MS (FAB, 3-NBA, LiCl): 539 (M+Li⁺).

### Beispiel 48

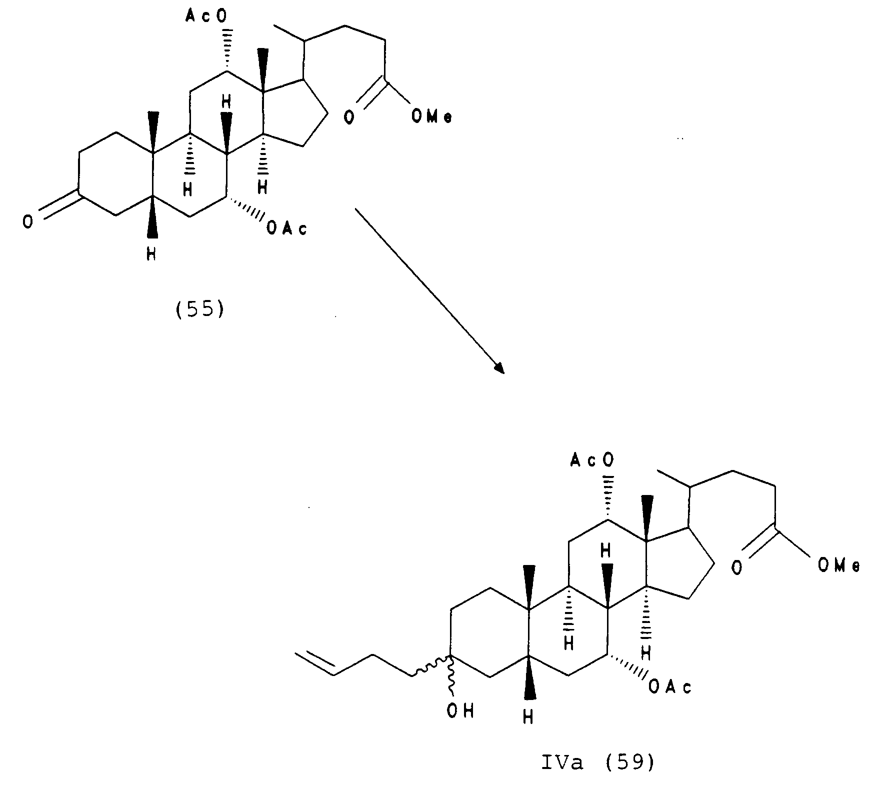

Aus 10,2 ml (110 mmol) 4-Brom-1-buten und 2,4 g (100 mmol) Magnesium wird in 300 ml THF eine Grignardlösung hergestellt. Bei Zimmertemperatur werden 20 g (39.6 mmol) Verbindung (55) in 100 ml THF zugetropft. Nach 3-stündigem Rühren bei Zimmertemperatur werden 250 ml NH₄Cl-Lösung zugegeben und 3 x mit Essigester extrahiert. Die vereinigten organischen Phasen werden über Na₂SO₄ getrocknet und eingeengt. Chromatographie über Kieselgel (Cyclohexan/Essigester 4 : 1) ergibt 13,5 g (24.1 mmol, 61 %) IVa (59) als Hauptprodukt.
C₃₃H₅₂O₇ (560) MS (FAB, 3-NBA, LiCl): 567 (M+Li⁺).

### Beispiel 49

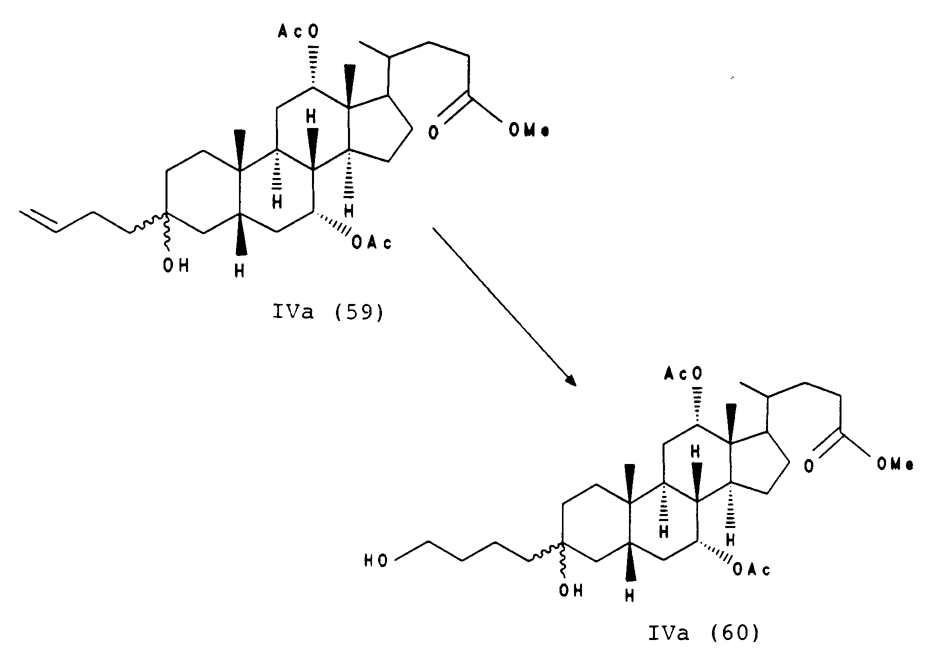

Zu 1,0 g (1.78 mmol) IVa (59) in 30 ml Tetrahydrofuran werden bei - 30 °C 4,0 ml einer 1M Lösung von Boran in Tetrahydrofuran zugetropft, Nach 2 Stunden bei - 30 °C und 8 Stunden bei Zimmertemperatur wird auf 0 °C gekühlt, 2,0 ml 2M Natriumhydroxidlösung und dann 0,68 ml 36 %ige H₂O₂ zugegeben und noch 30 min bei Zimmertemperatur gerührt. Zur Aufarbeitung wird mit gesättigter Kochsalzlösung versetzt und 3 x mit Essigester extrahiert. Die vereinigten organischen Phasen werden über MgSO₄ getrocknet und eingeengt. Chromatographie über Kieselgel (Essigester) ergibt 0,5 g (0.86 mmol, 48 %) IVa (60).
C₃₃H₅₄O₈ (578), MS (FAB, 3-NBA, LiCl): 585 (M+Li⁺).

### Beispiel 50

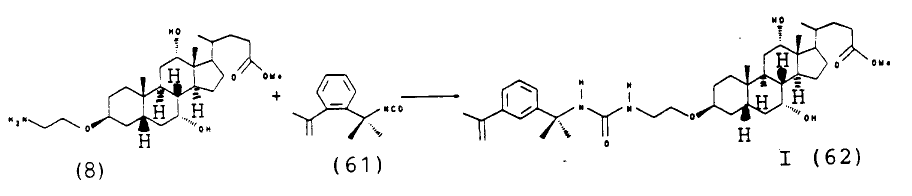

Zu einer Lösung von 1,57 g (3.37 mmol) (8) in 50 ml Chloroform werden bei 0 °C langsam 700 mg (3.48 mmol) (61) (1-(1-Isocyanato-1-methylethyl)-4-(1-methylethenyl)-benzol) in 5 ml Chloroform zugetropft. Es wird 1 Stunde bei 0 °C und 15 min bei Zimmertemperatur gerührt. Zur Aufarbeitung wird auf Wasser gegossen und 3 x mit Chloroform extrahiert. Die vereinigten organischen Phasen werden über MgSO₄ getrocknet und eingeengt. Chromatographie über Kieselgel (Chloroform/Methanol 92,5 : 7,5) ergibt 1,92 g (2.88 mmol, 85 %) I (62)
C₄₀H₆₂N₂O₆ (666), MS (FAB, 3-NBA, LiCl): 673 (M+Li⁺).

### Beispiel 51

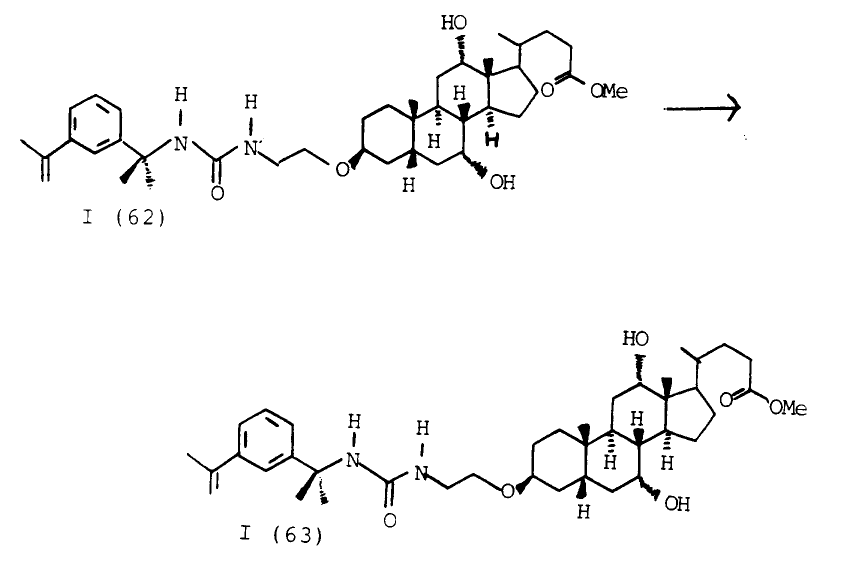

380 mg (0.57 mmol) I (62) werden in 20 ml Ethanol gelöst und mit 6 ml 1M wäßriger NaOH versetzt. Nach 4-stündigem Rühren bei Zimmertemperatur werden 100 ml Wasser zugegeben. Das Ethanol wird am Rotationsverdampfer abgezogen. Mit 2N HCl wird auf pH 1 gebracht und 3 x mit Chloroform extrahiert. Die vereinigten organischen Phasen werden über MgSO₄ getrocknet und im Vakuum eingedampft. Chromatographie über Kieselgel ergibt 300 mg (0.46 mmol, 81 %) I (63).
C₃₉H₆₀N₂O₆ (652), MS (FAB, 3-NBA, LiCl): 659 (M+Li⁺)

### Beispiel 52

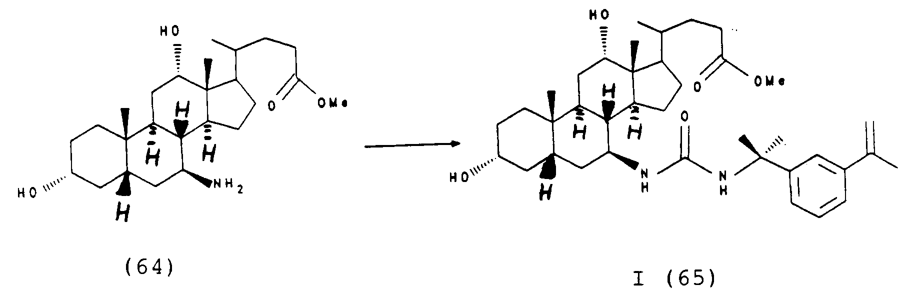

Aus 1,5 g (3.56 mmol) (64) (Bull. Chim. Soc. France 877, 1949; J. Chem. Soc. 2164, 1949) und 750 mg (3.73 mmol) (61) werden nach dem für Beispiel 50 beschriebenen Verfahren 1,39 g (2.23 mmol, 63 %) I (65) hergestellt.
C₃₈H₅₈N₂O₅ (622), MS (FAB, 3-NBA, LiCl): 629 (M+Li⁺).

## Patentansprüche

1. Ethylenisch ungesättigtes Gallensäurederivat der Formel I
G-X-A (I)
in der
G ein Gallensäurederivat,
X eine Brückengruppe und
A eine polymerisierbare, ethylenisch ungesättigte Gruppe
bedeutet.

2. Verbindung nach Anspruch 1, wobei
G eine freie Gallensäure bzw. ihr Alkali- oder Eralkalisalz oder eine am Ring D veresterte Gallensäure bedeutet, die über ihren Ring A oder B, vorzugsweise über Ring A, verbunden ist mit der Gruppe
X, für die die allgemeine Formel II gilt
(Y)ₒ - (Z)ₚ, (II)
worin
Y benachbart zu G steht und -O-, -NR'-, 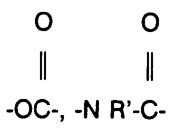 bedeutet,
Z (C₁-C₁₂)-Alkylen oder (C₇-C₁₃)-Aralkylen ist, wobei einzelne, bevorzugt 1 bis 4, Methylengruppen in der Alkylenkette des Alkylen- oder Aralkylenrestes durch Gruppen wie -O-, -NR'-, 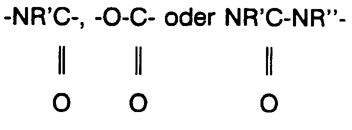 bevorzugt durch Gruppen eines Typs, ersetzt sein können und
o und p unabhängig voneinander null oder 1, wobei o und p nicht gleichzeitig null bedeuten, sind und
A 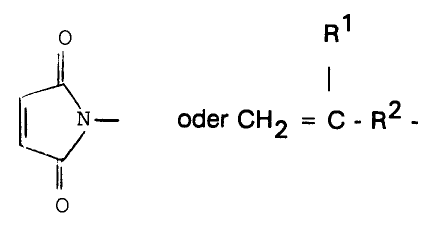 bedeutet, wobei
R¹ Wasserstoff oder CH₃ und
R² 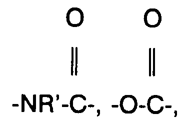 -O-, -NR'- oder eine Einfachbindung bedeuten, wobei die Carbonylgruppen benachbart zur C-C-Doppelbindung stehen, und
R' und R'' unabhängig voneinander Wasserstoff oder (C₁ -C₆)-Alkyl, bevorzugt (C₁ -C₃)-Alkyl bedeuten.

3. Verbindung nach einem der Ansprüche 1 oder 2, in der G der Formel III 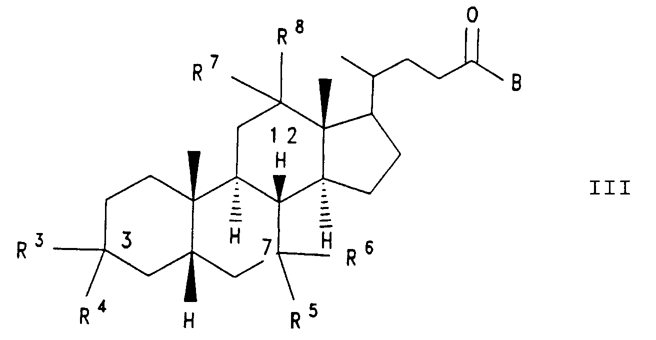 entspricht, worin
R³ bis R⁸ unabhängig voneinander Wasserstoff, OH, NH₂ oder eine mit einer OH- Schutrgruppe geschützte OH Gruppe, und einer der Reste R³, R⁴, R⁵, R⁶ eine Bindung zur Gruppe X bedeuten, wobei diese Bindung von den Positionen 3 (R³ oder R⁴), bevorzugt 3β, oder 7 (R⁵ oder R⁶) ausgeht und die jeweils andere Position 7 oder 3 eine OH Gruppe oder eine geschützte OH Gruppe trägt,
B -OH, -O-Alkali, -O-Erdalkali, -O-(C₁-C₁₂)-Alkyl, -O-Allyl oder -O-Benzyl bedeutet, bevorzugt -OH, -O-Alkali oder -O-(C₁-C₆)-Alkyl,
-O-Allyl oder -O-Benzyl, bedeutet,
wobei eine gebildete Estergruppe 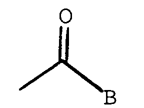 sowohl sauer wie auch basisch verseifbare Ester darstellt,
Y -O-, -NR'-, 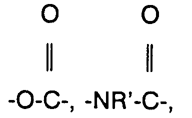
Z (C₁-C₁₂)-Alkylen, (C₇-C₁₃)-Aralkylen, wobei 1 bis 3 Methylengruppen in der Alkylenkette durch die Gruppen -O-, -NR'-, 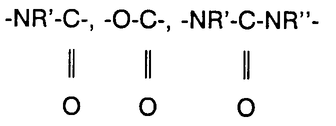 ersetzt sind und
o und p unabhängig voneinander null oder 1 bedeuten, aber nicht gleichzeitig null sind
A 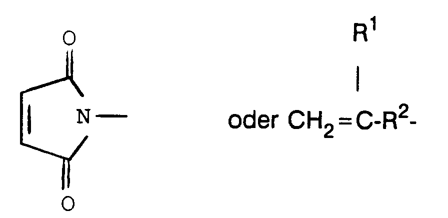 bedeuten, wobei
R¹ Wasserstoff oder CH₃ und
R² 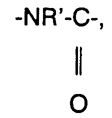 -NR'- oder eine Einfachbindung bedeutet, worin
R' und R'' unabhängig voneinander Wasserstoff oder (C₁-C₆)-Alkyl bedeuten.

4. Verbindung nach einem der Ansprüche 1 bis 3, dadurch gekennzeichnet, daß,
sofern p = null und o = 1 ist, Y bevorzugt 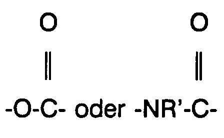 bedeutet.

5. Verbindung nach einem der Ansprüche 1 bis 3, dadurch gekennzeichnet, daß, sofern p = 1 und o = null ist, Z bevorzugt (C₁-C₁₂)-Alkylen ist, wobei 1 - 3 Methylengruppe durch 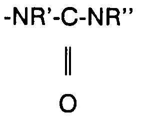 ersetzt sind.

6. Verbindung nach einem der Ansprüche 1 bis 3, dadurch gekennzeichnet, daß, sofern p = 1 und o = 1 ist, Y bevorzugt -O- und Z (C₁-C₁₂)-Alkylen oder (C₇-C₁₃)-Aralkylen bedeutet, wobei 1 oder 2 Methylengruppen durch 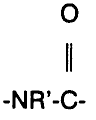 oder 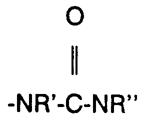 ersetzt sind.

7. Verbindung nach einem der Ansprüche 1 bis 6, dadurch gekennzeichnet, daß
A 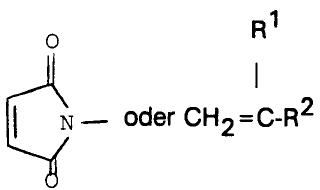 bedeutet, wobei
R¹ Wasserstoff oder CH₃ und
R² 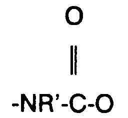 ist.

8. Verwendung der Verbindungen nach den Ansprüchen 1 bis 7 zur Herstellung von Gallensäurepolymeren oder -copolymeren.

9. Gallensäurederivat der Formel IVa
G - X' IVa
in der
G einen Gallensäurerest der Formel V 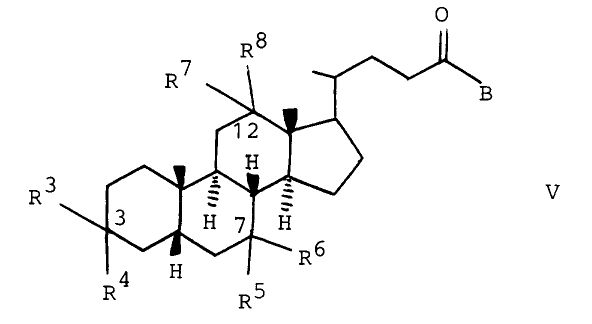
bedeutet, worin
einer der Reste R³ bis R⁶ die Bedeutung von X' hat und die übrigen Reste R³ bis R⁸ unabhängig voneinander Wasserstoff, OH oder eine mit einer OH-Schutzgruppe geschützte OH-Gruppe darstellen
B -OH, -O-Alkali, -O-Erdalkali, -O(C₁-C₁₂)-Alkyl, -O-Allyl oder O-Benzyl bedeutet,
und
X' einen Rest der Formel VI
Y' - Z' VI
bedeutet, worin
Y' benachbart zu G steht und eine einfache Bindung,
-O-(CH₂)₂₋₁₂-,
-(CH₂)₁₋₆- oder 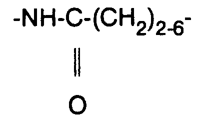 bedeutet
Z' -OH, -OSO₂-CH₃, 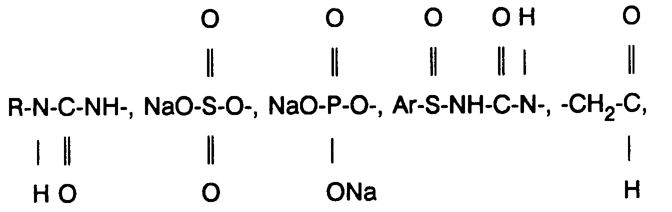 -O-Benzyl, -O-Allyl, -O-t-Butyldimethylsilyl, 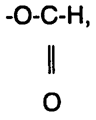 -N₃, -NH₂, -CN
oder Brom darstellt

10. Gallensäurederivat gemäß Anspruch 9, dadurch gekennzeichnet, daß R³ oder R⁴ die Bedeutung von X' hat.

11. Verwendung einer Verbindung gemäß Anspruch 9 zur Herstellung von Pharmaka.

12. Verwendung einer Verbindung gemäß Anspruch 9 zur Herstellung von Verbindungen der Formel I gemäß Anspruch 1.
